(19) Europäisches Patentamt
European Patent Office
Office européen des brevets

(11) Veröffentlichungsnummer: **0 065 485 B1**

(12) # EUROPÄISCHE PATENTSCHRIFT

(45) Veröffentlichungstag der Patentschrift: **18.04.90**

(51) Int. Cl.⁵: **C 07 D 405/06**, A 61 K 31/41, A 61 K 31/415

(21) Anmeldenummer: **82810193.1**

(22) Anmeldetag: **06.05.82**

(54) **Arylphenylether-derivate als Mikrobizide, Verfahren zu deren Herstellung und deren Verwendung.**

(30) Priorität: **12.05.81 CH 3066/81**
**21.04.82 CH 2428/82**

(43) Veröffentlichungstag der Anmeldung:
**24.11.82 Patentblatt 82/47**

(45) Bekanntmachung des Hinweises auf die Patenterteilung:
**18.04.90 Patentblatt 90/16**

(84) Benannte Vertragsstaaten:
**AT BE CH DE FR IT LI LU NL SE**

(56) Entgegenhaltungen:
EP-A-0 026 990
EP-A-0 029 355
EP-A-0 043 419
EP-A-0 049 565
EP-A-0 080 071
US-A-3 936 470
US-A-4 101 666
US-A-4 156 008
US-A-4 160 838

**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Klybeckstrasse 141**
**CH-4002 Basel (CH)**

(72) Erfinder: **Hubele, Adolf, Dr.**
**Obere Egg 9**
**CH-4465 Magden (CH)**
Erfinder: **Riebli, Peter**
**Bünten 17**
**CH-4446 Buckten (CH)**

**EP 0 065 485 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft substituierte Arylphenyletherderivate der nachstehenden Formel I sowie deren Säureadditionssalze und Metallkomplexe. Sie betrifft ferner die Herstellung dieser Verbindungen sowie agrochemische Mittel, die als Wirkstoff mindestens eine der Verbindungen der Formel I enthalten, und ihre Verwendung zur Bekämpfung oder Verhütung eines Befalls von Pflanzen durch phytopathogene Mikroorganismen.

Anmerkung

Die für einen Teilbereich dieser Erfindung unter Art. 54(3) EPÜ zu berücksichtigende Referenz EP—A—80071 erfordert eine Kennzeichnung der Erfindungsteile mit 1. Priorität vom 12.05. 81, wie sie am Schluss in Form der Patentansprüche 1 bis 6, 9, 10, 12 und 13 für den Vertragsstaat AT und in Form der Patentansprüche 1 bis 13, 19, 20, 22, 23, 26 und 27 für die übrigen Vertragsstaaten wiedergegeben wird.

Die erfindungsgemässen Arylphenylether-derivate haben die Formel I

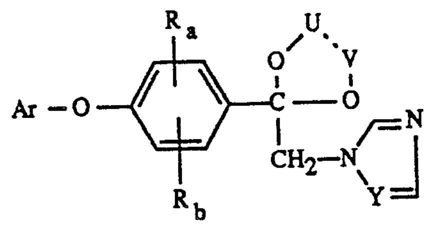

(I)

worin

Y für —CH= oder —N= steht;

$R_a$ und $R_b$ unabhängig voneinander für Wasserstoff, Halogen, $C_1$—$C_3$-Alkyl, $C_1$—$C_3$-Alkoxy oder Nitro stehen;

Ar unsubstituiertes oder durch Halogen, $C_1$—$C_7$-Alkyl, $C_1$—$C_7$-Alkoxy, Nitro und/oder $CF_3$ ein- oder mehrfach substituiertes Phenyl oder Naphthyl bedeutet;

U und V unabhängig voneinander für gegebenenfalls durch Halogen oder $C_1$—$C_6$-Alkoxy substituiertes $C_1$—$C_{12}$-Alkyl stehen oder zusammen eine der folgenden Alkylenbrücken

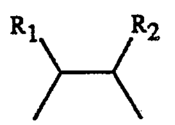

,

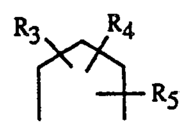

oder

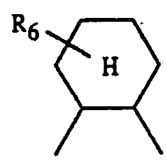

bilden, wobei

$R_1$ und $R_2$ unabhängig voneinander für Wasserstoff, $C_1$—$C_{12}$-Alkyl, ein- oder mehrfach durch Halogen substituiertes $C_1$—$C_{12}$-Alkyl, Phenyl, ein- oder mehrfach durch Halogen und/oder $C_1$—$C_3$-Alkyl substituiertes Phenyl oder für die Gruppe $—CH_2—Z—R_7$ stehen, wobei

Z Sauerstoff oder Schwefel bedeutet und

$R_7$ für Wasserstoff, $C_1$—$C_8$-Alkyl, ein durch $C_1$—$C_2$-Alkoxy substituiertes $C_1$—$C_8$-Alkyl, $C_3$—$C_4$-Alkenyl, 2-Propinyl, 3-Halogen-2-propinyl, Phenyl, ein durch Halogen, $C_1$—$C_3$-Alkyl, $C_1$—$C_3$-Alkoxy, Nitro und/oder $CF_3$ ein- oder mehrfach substituiertes Phenyl, Benzyl oder ein durch Halogen, $C_1$—$C_3$-Alkyl und/oder $C_1$—$C_3$-Alkoxy ein- oder mehrfach substituiertes Benzyl steht;

$R_3$, $R_4$ und $R_5$ unabhängig voneinander Wasserstoff oder $C_1$—$C_4$-Alkyl bedeuten, wobei die Gesamtzahl der Kohlenstoffatome in $R_3$, $R_4$ und $R_5$ die Zahl 6 nicht übersteigt und

$R_6$ für Wasserstoff oder $C_1$—$C_3$-Alkyl steht;

unter Einschluss ihrer Säureadditionssalze und Metallkomplexe.

Der Substituent Ar hat als gegebenenfalls substituiertes Phenyl oder Naphthyl beispielsweise die Formel

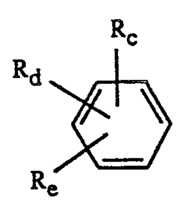

oder

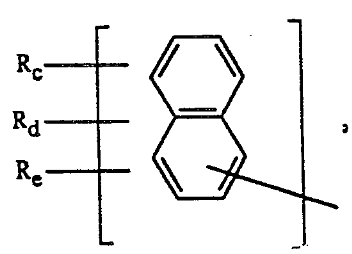

,

worin $R_c$, $R_d$ und $R_e$ unabhängig voneinander für Wasserstoff, Halogen, $C_1$—$C_7$-Alkyl, $C_1$—$C_7$-Alkoxy, Nitro oder $CF_3$ stehen.

Unter dem Begriff Alkyl selbst oder als Bestandteil eines anderen Substituenten sind je nach Zahl der

angegebenen Kohlenstoffatome beispielsweise folgende Gruppen zu verstehen: Methyl, Ethyl, Propyl, Butyl, Pentyl, Hexyl, Heptyl, Octyl, Nonyl, Decyl, Undecyl oder Dodecyl sowie ihre Isomeren wie z.B. Isopropyl, Isobutyl, tert.-Butyl, sek.-Butyl, Isopentyl usw. Alkenyl steht z.B. für Propenyl-(1), Allyl, Butenyl-(1), Butenyl-(2) oder Butenyl-(3). Halogen soll hier und im fogenden Fluor, Chlor, Brom oder Jod, vorzugsweise Chlor oder Brom, bedeuten.

Die Erfindung betrifft sowohl die freien Verbindungen der Formel I, als auch deren Additionssalze mit anorganischen und organischen Säuren, ebenso deren Komplexe mit Metallsalzen.

Erfindungsgemässe Salze sind insbesondere Additionssalze mit nach Massgabe des Einsatzzweckes physiologisch unbedenklichen anorganischen oder organischen Säuren.

Bezüglich des Einsatzes als Mikrobizide im Pflanzenschutz physiologisch unbedenkliche anorganische und organische Säuren sind beispielsweise Halogenwasserstoffsäuren, z.B. Chlor-, Brom- oder Jodwasserstoffsäure, Schwefelsäure, Phosphorsäure, phosphorige Säure, Salpetersäure, gegebenenfalls halogenierte Fettsäuren, wie Essigsäure, Trichloressigsäure und Oxalsäure, oder Sulfonsäuren, wie Benzolsulfonsäure und Methansulfonsäure.

Metallkomplexe der Formel I bestehen aus dem zugrundeliegenden organischen Molekül und einem anorganischen oder organischen Metallsalz, z.B. den Halogeniden, Nitraten, Sulfaten, Phosphaten, Tartraten usw. des Kupfers, Mangans, Eisens, Zinks und anderer Metalle. Dabei können die Metallkationen in den verschiedenen ihnen zukommenden Wertigkeiten vorliegen.

Die Verbindungen der Formel I sind bei Raumtemperatur stabile Oele, Harze oder Feststoffe, die sich durch sehr wertvolle physiologische, wie mikrobizide, beispielsweise pflanzenfungizide Eigenschaften auszeichnen. Sie lassen sich daher auf dem Agrarsektor oder verwandten Gebieten zur Bekämpfung von phytopathogenen Mikroorganismen einsetzen.

Eine für den Einsatz im Pflanzenschutz wichtige Gruppe von Mikrobiziden besteht aus Verbindungen der Formel I, worin Y für —CH= oder —N= steht; $R_a$ und $R_b$ unabhängig voneinander für Wasserstoff, Halogen, $C_1$—$C_3$ Alkyl, $C_1$—$C_3$-Alkoxy oder Nitro stehen; Ar für die Gruppe

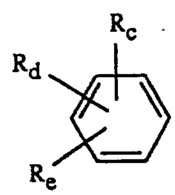

oder

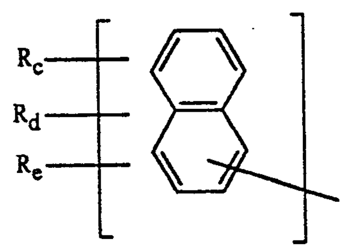

steht; wobei

$R_c$, $R_d$ und $R_e$ unabhängig voneinander für Wasserstoff, Halogen, $C_1$—$C_3$-Alkyl, $C_1$—$C_3$-Alkoxy, Nitro oder $CF_3$ stehen;

U und V unabhängig voneinander für $C_1$—$C_{12}$-Alkyl stehen oder zusammen eine der folgenden Alkylenbrücken

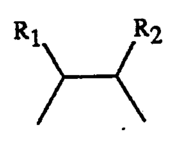

,

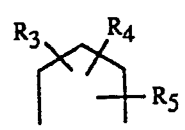

oder

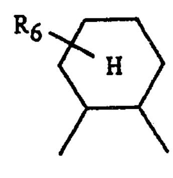

bilden, wobei

$R_1$ und $R_2$ unabhängig voneinander für Wasserstoff, $C_1$—$C_{12}$-Alkyl, ein- oder mehrfach durch Halogen substituiertes $C_1$—$C_{12}$-Alkyl, Phenyl, ein- oder mehrfach durch Halogen und/oder $C_1$—$C_3$-Alkyl substituiertes Phenyl oder für die Gruppe —$CH_2$—Z—$R_7$ stehen, wobei

Z Sauerstoff oder Schwefel bedeutet und

$R_7$ für Wasserstoff, $C_1$—$C_8$-Alkyl, ein durch $C_1$—$C_2$-Alkoxy substituiertes $C_1$—$C_8$-Alkyl, $C_3$—$C_4$-Alkenyl, 2-Propionyl, 3-Halogen-2-propinyl, Phenyl, ein durch Halogen, $C_1$—$C_3$-Alkyl, $C_1$—$C_3$-Alkoxy, Nitro und/oder $CF_3$ ein- oder mehrfach substituiertes Phenyl, Benzyl oder ein durch Halogen, $C_1$—$C_3$-Alkyl und/oder $C_1$—$C_3$-Alkoxy ein- oder mehrfach substituiertes Benzyl steht;

$R_3$, $R_4$ und $R_5$ unabhängig voneinander Wasserstoff oder $C_1$—$C_4$-Alkyl bedeuten, wobei die Gesamtzahl der Kohlenstoffatome in $R_3$, $R_4$ und $R_5$ die Zahl 6 nicht übersteigt und

$R_6$ für Wasserstoff oder $C_1$—$C_3$-Alkyl steht;

unter Einschluss ihrer Säureadditionssalze und Metallkomplexe. Diese Untergruppe soll hier und im folgenden als Gruppe Ia bezeichnet werden.

Eine bevorzugte Gruppe von landwirtschaftlich nutzbaren Mikrobiziden bilden Verbindungen der Formel I unter Einschluss ihrer Salze und Metallkomplexe, worin Y für —CH= oder —N= steht; $R_a$ und $R_b$ unabhängig voneinander für Wasserstoff, Halogen oder $C_1$—$C_3$-Alkyl stehen; Ar für die Gruppe

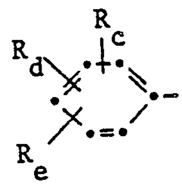

steht, worin $R_c$, $R_d$ und $R_e$ unabhängig voneinander Wasserstoff, Halogen, $CF_3$ oder $C_1$—$C_3$-Alkyl bedeuten; und U sowie V die unter Formel I angegebenen Bedeutungen haben; diese Gruppe soll Ib genannt werden.

Innerhalb der Untergruppe Ib sind diejenigen Verbindungen der Formel I als Mikrobizide bevorzugt, worin U und V unabhängig voneinander für $C_1$—$C_3$-Alkyl stehen oder zusammen eine der folgenden Alkylengruppen

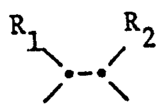 oder 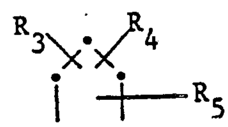

bilden, worin $R_1$, $R_2$, $R_3$, $R_4$ und $R_5$ unabhängig voneinander für Wasserstoff oder $C_1$—$C_4$-Alkyl stehen, wobei die Gesamtzahl der Kohlenstoffatome in $R_3$, $R_4$ und $R_5$ die Zahl 6 nicht übersteigt; diese Gruppe soll Ic genannt werden.

Eine weitere bevorzugte Gruppe von landwirtschaftlich vorteilhaften Mikrobiziden bilden Verbindungen der Formel I, worin Y für —CH= oder —N= steht; Ar die unter Formel I angegebenen Bedeutungen hat; $R_a$, $R_b$, $R_c$, $R_d$ und $R_e$ unabhängig voneinander für Wasserstoff, Chlor, Brom, Fluor, Methyl, Methoxy oder Nitro stehen; U und V unabhängig voneinander für $C_1$—$C_3$-Alkyl stehen oder zusammen eine der unter Formel I genannten Alkylengruppe bilden, wobei $R_1$, $R_2$, $R_3$, $R_4$, $R_5$ und $R_6$ unabhängig voneinander für Wasserstoff oder $C_1$—$C_3$-Alkyl stehen oder $R_1$ für —$CH_2$—O—$R_7$ steht, wobei $R_7$ $C_1$—$C_3$-Alkyl, durch $C_1$—$C_3$-Alkoxy substituiertes $C_2$—$C_4$-Alkyl, $C_3$—$C_4$-Alkenyl oder Phenyl bedeutet; diese Gruppe soll Id genannt werden.

Unter den Mikrobiziden der Untergruppe Id sind besonders jene bevorzugt, worin U und V zusammen eine unsubstituierte oder einfach substituierte Ethylen- oder Propylenbrücke bilden; diese Gruppe soll Ie genannt werden.

So gehören beispielsweise folgende Substanzen zu den besonders bevorzugten landwirtschaftlich nutzbaren Einzelsubstanzen:

2-[p-(Phenoxy)-phenyl]-2-[1-(1H-1,2,4-triazolyl)-methyl]-4-methyl-1,3-dioxan,
2-[p-(Phenoxy)-phenyl]-2-[1-(1H-1,2,4-triazolyl)-methyl]-4-ethyl-1,3-dioxan,
2-[p-(Phenoxy)-phenyl]-2-[1-(1H-1,2,4-triazolyl)-methyl]-4-methyl-1,3-dioxolan,
2-[p-(Phenoxy)-phenyl]-2-[1-(1H-1,2,4-triazolyl)-methyl]-4-ethyl-1,3-dioxolan.

Die Verbindungen der Formel I können folgendermassen hergestellt werden, indem man

A) eine Verbindung der Formel II

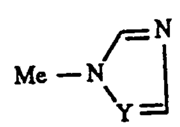 (II)

worin Me Wasserstoff oder ein Metallkation darstellt, mit einer Verbindung der Formel III

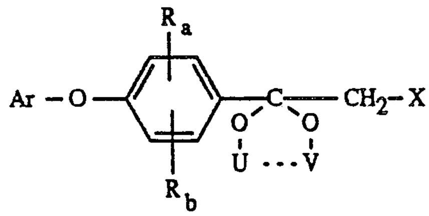 (III)

worin X eine nukleofuge Abgangsgruppe bedeutet, kondensiert oder

B) in einer Verbindung der Formel IV

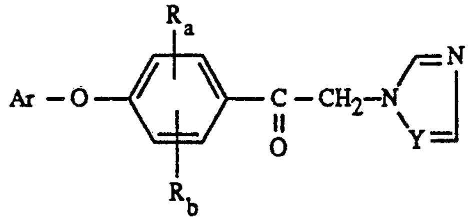

(IV)

die Carbonylgruppe in eine Gruppe der Formel V

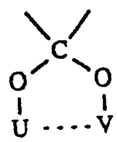

(V)

überführt, oder

C) zur Herstellung von Verbindungen der Formel I, worin U und V gemeinsam eine Gruppe der Formel $—CH_2—CH(CH_2ZR_7')—$ darstellen und $R_7'$ einen von Wasserstoff verschiedenen Rest $R_7$ bedeutet, Verbindungen der Formeln VI und VII

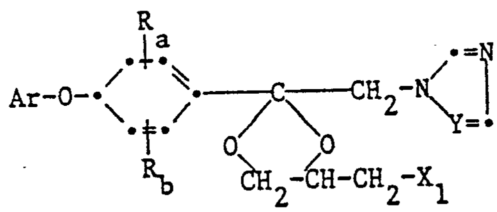

(VI) und $X_2 - R_7$ (VII)

worin einer der Reste $X_1$ und $X_2$ gegebenenfalls in Salzform vorliegendes Hydroxy oder Mercapto, z.B. der Formel —Z—Me, und der andere eine nukleophile Abgangsgruppe X bedeutet oder sowohl $X_1$ als auch $X_2$ Hydroxygruppen darstellen, miteinander kondensiert oder

D) Verbindungen der Formel VIII und IX

$Ar\text{-}X_3$ (VIII) und

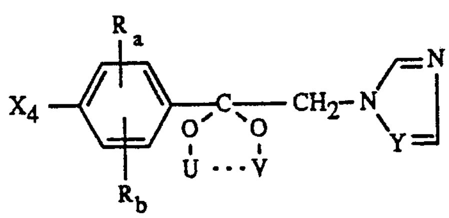

(IX)

worin einer der Reste $X_3$ und $X_4$ eine Gruppe —O—Me, in der Me Wasserstoff oder vorzugsweise ein Metallkation ist, und der andere einen gegen Aryloxy austauschbaren Rest bedeutet, miteinander kondensiert und, wenn erwünscht, eine verfahrensgemäss erhältliche Verbindung in eine andere Verbindung der Formel I umwandelt und/oder eine verfahrensgemäss erhältliche freie Verbindung in ein Säureadditionssalz, ein verfahrensgemäss erhältliches Säureadditionssalz in die freie Verbindung oder in ein anderes Säureadditionssalz, oder eine verfahrensgemäss erhältliche freie Verbindung bzw. ein verfahrensgemäss erhältliches Säureadditionssalz in einen Metallkomplex überführt.

Metallkationen Me sind dabei beispielsweise Alkalimetall-, z.B. Lithium-, Natrium- oder Kaliumkationen, oder Erdalkalimetall-, z.B. Magnesium-, Calcium-, Strontium- oder Bariumkationen.

Nukleofuge Abgangsgruppen sind beispielsweise reaktionsfähige veresterte Hydroxygruppen, wie mit einer Halogenwasserstoffsäure, z.B. mit Fluor, Chlor-, Brom- oder Jodwasserstoffsäure, oder einer Niederalkan-, gegebenenfalls substituierten Benzol- oder Halogensulfonsäure, z.B. mit Methan-, Ethan-, Benzol-, p-Toluol- oder Fluorsulfonsäure, veresterte Hydroxygruppen.

Die Umsetzung eines Azols der Formel II

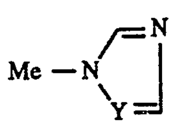

(II)

worin Y für —CH= oder —N= steht und Me bevorzugt ein Metallatom, insbesondere ein Alkalimetallatom darstellt, mit einer Verbindung der Formel III

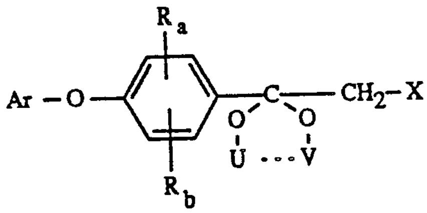

(III)

worin Ar, $R_a$, $R_b$, U und V die unter Formel I angegebenen Bedeutungen haben und X beispielsweise für Halogen, insbesondere Chlor, Brom oder Jod, oder Benzolsulfonyloxy, p-Tosyloxy, Trifluoracetyloxy oder bevorzugt Niederalkylsulfonyloxy wie z.B. Mesyloxy, bedeutet, wird bevorzugt in einem relativ polaren, jedoch reaktionsinerten organischen Lösungsmittel durchgeführt, z.B. N,N-Dimethylformamid, N,N-Dimethylacetamid, Dimethylsulfoxid, Acetonitril, Benzonitril und anderen. Derartige Lösungsmittel können in Kombination mit anderen reaktionsinerten Lösungsmitteln, wie aliphatischen oder aromatischen Kohlenwasserstoffen, z.B. Benzol, Toluol, Xylol, Hexan, Petrolether, Chlorbenzol, Nitrobenzol verwendet werden.

Bedeutet X Chlor oder Brom, so kann man zweckmässig Alkalijodid (wie NaJ oder KJ) zur Beschleunigung der Reaktion zusetzen. Erhöhte Temperaturen von 0 bis 220°C, bevorzugt 80—170°C, sind vorteilhaft. Zweckmässig wird das Reaktionsgemisch unter Rückfluss erhitzt.

In den Fällen, in denen in Formel II Me für Wasserstoff steht, wird das Verfahren in Gegenwart einer Base durchgeführt. Beispiele solcher Basen sind anorganische Basen wie die Oxide, Hydroxide, Hydride, Carbonate und Hydrogencarbonate von Alkali und Erdalkalimetallen sowie z.B. tert. Amine wie Triethylamin, Triethylendiamin, Piperidin, Pyridin, 4-Dimethylaminopyridin oder 4-Pyrrolidylpyridin.

Bei dieser und den folgenden Herstellungsvarianten können die Zwischen- und Endprodukte aus dem Reaktionsmedium isoliert und, falls erwünscht, auf eine der allgemein üblichen Methoden gereinigt werden, z.B. durch Extraktion, Kristallisation, Chromatographie oder Destillation.

Die Ueberführung der Carbonylgruppe in Verbindungen der Formel IV in die Gruppe der Formel V erfolgt durch Umsetzung mit einem Orthocarbonsäure-tri-$C_1$—$C_{12}$-alkylester, dessen $C_1$—$C_{12}$-Alkylgruppen gegebenenfalls durch Halogen oder $C_1$—$C_6$-Alkoxy substituiert sind, oder in Gegenwart einer Säure mit mindestens 2 Mol eines einwertigen Alkohols der Formel U—OH (Va), wobei Verbindungen der Formel I erhalten werden, worin U und V gleiche, gegebenenfalls substituierte $C_1$—$C_{12}$-Alkylgruppen bedeuten, oder durch Umsetzung mit einem Diol der Formel Vb

HO—U - - - - - V—OH (Vb)

wobei Verbindungen der Formel I erhalten werden, worin U und V gemeinsam eine der eingangs definierten Alkylenbrücken darstellen. Dabei haben Ar, Y, $R_a$, $R_b$, U und V die unter Formel I angegebenen Bedeutungen.

Diese Ketalisierungsreaktion kann analog zu bereits bekannten Ketalisierungs-Reaktionen durchgeführt werden, beispielsweise analog zur Herstellung von 2-Brommethyl-2,4-diphenyl-1,3-dioxolan [Synthesis, *1974* (I), 23].

Bei der bevorzugten Ausführung der Ketalisierung werden beide Reaktionspartner mehrere Stunden zusammen mit einem Azeotrop-Bildner in einem der üblichen organischen Lösungsmittel under Rückfluss erhitzt. Als Azeotrop-Bildner kommen z.B. Benzol, Toluol, Xylol, Chloroform oder Tetrachlorkohlenstoff in Frage, wobei zur Beschleunigung der Reaktion ein Zusatz einer starken Säure, wie z.B. p-Toluolsulfonsäure, vorteilhaft sein kann. Verwendbare organische Lösungsmittel sind in diesem Fall z.B. aromatische Kohlenwasserstoffe, wie Benzol, Toluol, Xylol usw., gesättigte Kohlenwasserstoffe, wie n-Hexan oder gesättigte halogenierte Kohlenwasserstoffe wie z.B. 1,1,1-Trichlorethan.

Es sind auch weitere Wege der Ketalisierung durchführbar, z.B. indem man ein Keton IV, das mit einem von Alkanolen bzw. Diolen der Formeln Va bzw. Vb verschiedenen Alkohol oder Phenol ketalisiert ist, umsetzt und dieses durch Reaktion mit überschüssigem Alkanol Va bzw. dem Diol Vb zu (I) umketalisiert. Das Ausgangsprodukt ist z.B. gemäss einer der Verfahrensvarianten A) oder D) zugänglich.

Die Herstellung von Verbindungen der Formel I, worin Substituenten U und V gemäss Variante C) zusammen für $—CH_2—CH(CH_2ZR_7)—$ stehen, erfolgt beispielsweise durch Reaktion einer Verbindung der Formel VI mit einer Verbindung der Formel VII, worin $X_1$ eine Gruppe —ZH und $X_2$ eine Gruppe X bedeutet. Die Reaktion wird bevorzugt in reaktions-inerten organischen Lösungsmitteln durchgeführt. Es eignen sich hierzu z.B. N,N-Dimethylformamid, N,N-Dimethylacetamid, Hexamethylphosphortriamid, Dimethylsulfoxid oder 4-Methyl-3-pentanon. Auch Gemische mit anderen reaktionsinerten Lösungsmitteln, z.B. mit aromatischen Kohlenwasserstoffen wie Benzol, Toluol oder Xylol können verwendet werden. In manchen Fällen kann es sich als vorteilhaft erweisen, zur Beschleunigung der Reaktionsgeschwindigkeit in Gegenwart einer Base zu arbeiten. Solche Basen sind z.B. Alkalimetallhydride oder Alkalimetallcarbonate.

In gewissen Fällen kann es auch von Vorteil sein, dass man die Verbindung VI zuerst auf bekannte Art in ein geeignetes Metall-Salz überführt.

Dies geschieht vorzugsweise durch Reaktion von VI mit einer Na-Verbindung, z.B. Natriumhydrid oder Natriumhydroxid. Anschliessend wird dieses Salz von VI mit der Verbindung der Formel VII umgesetzt. Zur Erhöhung der Reaktionsgeschwindigkeit kann in manchen Fällen auch bei erhöhter Temperatur, vorzugsweise 80°C bis 130°C, bzw. am Siedepunkt des Lösungsmittels gearbeitet werden.

In analoger Weise kann man auch Verbindungen der Formeln VI und VII, worin $X_1$ eine Gruppe X und $X_2$ eine Gruppe —ZH ist, umsetzen.

Bei der zu Produkten der Formel I, worin Z Sauerstoff ist, führenden Kondensations-Reaktion von Verbindungen der Formeln VI und VII, worin $X_1$ und $X_2$ Hydroxy darstellen, können die Reaktanden in einem geeigneten Lösungsmittel unter Rückfluss erhitzt werden, wobei gleichzeitig das entstehende Wasser azeotrop aus dem Reaktionsgemisch abdestilliert wird. Als Lösungsmittel kommen aromatische Kohlenwasserstoffe, wie Toluol oder Alkohol HO—$R_7$ selbst in Frage. Bei dieser Reaktion arbeitet man zweckmässigerweise in Gegenwart einer starken Säure, z.B. p-Toluolsulfonsäure.

Bei der Variante D) geht man vorzugsweise von Verbindungen der Formeln VIII und IX aus, worin $X_3$ eine Gruppe —OMe und $X_4$ eine nukleofuge Abgangsgruppe ist oder umgekehrt, $X_3$ die nukleofuge Abgangsgruppe darstellt und $X_4$ die Gruppe —OMe bildet. Hierbei haben $R_a$, $R_b$, U, V, Y und Ar die unter Formel I angegebenen Bedeutungen; Me steht bevorzugt für Wasserstoff. Die Reaktion wird vorteilhafterweise unter den bei Variante A) beschriebenen Bedingungen durchgeführt.

Verfahrensgemäss erhältliche Verbindungen können nach an sich bekannten Methoden in andere Verbindungen der Formel I überführt werden.

So kann man beispielsweise verfahrensgemäss erhältliche Verbindungen zu anderen Verbindungen der Formel I umketalisieren. Beispielsweise kann man in Verbindungen der Formel I, worin U und V gleiche, gegebenenfalls substituierte, $C_1$—$C_{12}$-Alkylreste U darstellen, durch Umsetzung mit 1 Mol eines anderen, gegebenenfalls substituierten $C_1$—$C_{12}$-Alkanols der Formel V—OH (Vc), eine Gruppe U durch eine Gruppe V oder durch Umsetzung mit einem Diol der Formel Vb beide Gruppen U durch einen zweiwertigen Rest ersetzen. Die Umketalisierung erfolgt in üblicher Weise, beispielsweise in Gegenwart eines sauren Kondensationsmittels, wie einer Mineral-, Sulfon- oder starken Carbonsäure, z.B. von Chlor- oder Bromwasserstoffsäure, Schwefelsäure, p-Toluolsulfonsäure oder Trifluoressigsäure, vorteilhaft unter destillativer bzw. azeotropdestillativer Entfernung leichtflüchtiger Reaktionsprodukte.

Ferner kann man in die carbocyclischen Arylteile verfahrensgemäss erhältlicher Verbindungen gegebenenfalls zusätzliche Substituenten in den Rest Ar und/oder die Gruppen $R_a$ bzw. $R_b$ einführen. So kann man z.B. durch Umsetzung mit einem Halogen in Gegenwart einer Lewissäure, wie einem Eisen-, Zink-, Bor- oder Antimonhalogenides, oder durch Behandlung mit N-Chlorsuccinimid, Halogen einführen.

Ferner kann man Nitrogruppen, z.B. mittels geeigneter komplexer Hydride, z.B. mit Lithiumaluminiumhydrid, zu Aminen reduzieren, diese, z.B. mittels salpetriger Säure, diazotieren und die gebildete Diazoniumgruppe in üblicher Weise durch Halogen oder Alkoxy ersetzen. Ebenso kann man Halogen durch Umsetzung mit einer Alkylmetallverbindung, z.B. mit einem Alkyllithium oder Alkylmagnesiumhalogenid, durch Alkyl ersetzen.

Werden die Verbindungen der Formel I als Basen erhalten, dann lassen sie sich durch anorganische oder organische Säuren in entsprechende Salze oder durch vorzugsweise äquimolare Mengen von Metallsalzen in Metallkomplexe der Formel I überführen. Umgekehrt lassen sich Salze der Formel I beispielsweise durch Reaktion mit Alkali(hydrogen)carbonat oder Alkalihydroxid in die freien Basen der Formel I überführen.

Die Ausgangsketale der Formel III lassen sich aus dem zugrundeliegenden Methylaryl-keton der Formel XIV

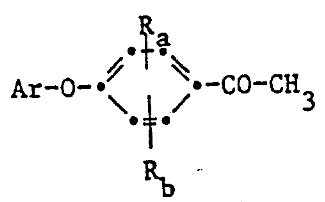

(XIV)

durch Reaktion mit dem gewünschten Diol in einem inerten Lösungsmittel z.B. einem halogenierten Kohlenwasserstoff (wie Methylenchlorid, Ethylenchlorid, Chloroform oder Tetrachlorkohlenstoff) und gleichzeitige oder anschliessende Halogenierung gewinnen. Zur Beschleunigung der Reaktion ist ein Zusatz von p-Toluolsulfonsäure vorteilhaft.

Die Ketone der Formel IV können durch Halogenierung der Ausgangsketone XIV zu XV

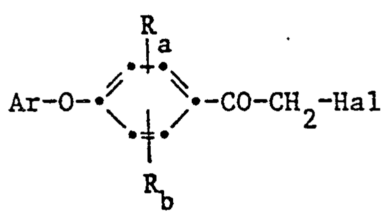

(XV)

und Weiterreaktion von XV, analog zu Variante A, mit einem Azol der Formel II hergestellt werden. Hierbei steht Hal bevorzugt für Chlor oder Brom.

Die Ketale III, VI, IX und X erhält man analog zu Variante B durch Reaktion des Ausgangsketons z.B. der Formel IV mit einem geeigneten Alkohol oder Diol.

Die beschriebenen Herstellungsvarianten sind Bestandteil der Erfindung.

Die Ausgangsstoffe der Formeln III, IV, VI, X und XI, die speziell für die Herstellung der erfindungsgemässen Verbindungen der Formel I entwickelt wurden, sind neu.

Bei allen beschriebenen Ketalisierungs-Reaktionen eines Ketons mit einem substituierten α,β- oder α,γ-Diol entstehen vorwiegend Gemische von Diastereomeren des resultierenden Ketals. Entsprechend bilden sich aus den Ausgangsketonen im allgemeinen Diastereomerengemische der Endprodukte I. Die Verbindungen der Formel I können beispielsweise in nachfolgenden beiden diastereomeren Formen vorliegen:

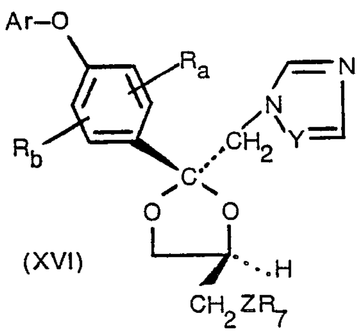

(XVI)

„A–Typen"

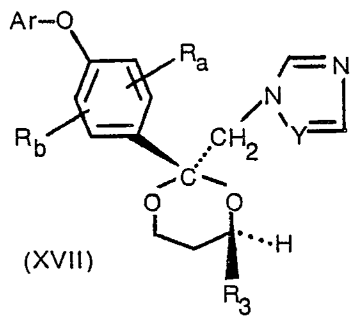

(XVII)

Die Konfiguration vom Typ A soll hier und im folgenden als das *"trans"-Isomere* bezeichnet werden.

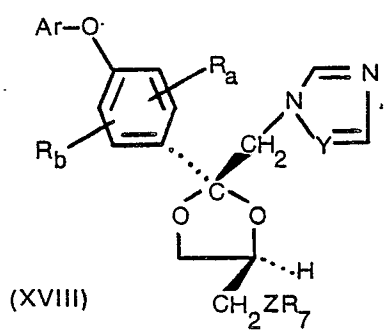

(XVIII)

„B–Typen"

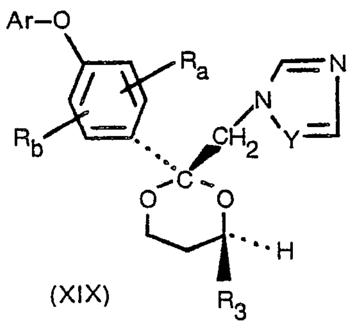

(XIX)

Die Symbole in den räumlich wiedergegebenen Strukturen sollen folgende Bedeutungen haben:

···· = hinter

— = in

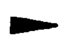 = vor der Zeichenebene.

Die Konfiguration vom Typ B soll entsprechend als das *"cis"-Isomere* bezeichnet werden. Die Trennung der beiden Diastereomeren kann beispielsweise durch fraktionierte Kristallisation oder durch Chromatographie (Dünn-, Dichtschicht-, Säulenchromatographie, Flüssigkeitshochdruckchromatographie usw.) erfolgen. Die beiden Isomeren zeigen unterschiedliche biologische Wirkungen. Im allgemeinen werden für praktische Zwecke die Diastereomerengemische verwendet.

Die Erfindung betrifft sämtliche isomeren Verbindungen der Formel I, ihre Salze und Metallkomplexe.

Das Herstellungsverfahren von Verbindungen der Formel I ist in seinen beschriebenen Varianten A, B, C und D Bestandteil der Erfindung.

Einige der in den Verfahren A, B, C und D verwendeten Ausgangsstoffe und Zwischenprodukte sind bekannt, andere können nach an sich bekannten Methoden hergestellt werden. Einige sind neu; ihre Herstellung wird hierin beschrieben.

1-Imidazolyl-4-arylbutyl-2-ketale werden in der EP—A—49 565 als Pharmazeutika vorgeschlagen.

1-(β-Aryl)-ethylimidazolylketale und 1-(β-Aryl)-ethyl-triazolylketale, worin Aryl für substituiertes Phenyl oder Naphthyl steht, werden in folgenden Referenzen als Fungizide und Bakterizide zitiert: US—PS:

3 575 999, 3 936 470, 4 101 664. Ähnliche Ketale, bei denen Aryl für Diphenyl steht, werden in der EP—A—29 355 vorgeschlagen. Gegenüber diesen und strukturell ähnlichen Ketalen, die als Mikrobizide vorgeschlagen werden und bei denen einige Verbindungen als strukturnächstes Element eine Alkoxyphenylgruppe tragen (US—A—4 160 838, US—A—4 156 008, US—A—4 101 666) besitzen Verbindungen der Formel I vorliegender Erfindung ein sehr günstiges Mikrobizid-Spektrum gegen pflanzenschädigende Mikroorganismen.

Es wurde überraschend festgestellt, dass Verbindungen der Formel I ein für praktische Bedürfnisse sehr günstiges Mikrobizid-Spektrum gegen phytopathogene Pilze und Bakterien aufweisen. So besitzen sie sehr vorteilhafte kurative, präventive und systemische pflanzentherapeutische Eigenschaften und lassen sich zum Schutz von Kulturpflanzen einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben. Die Wirkstoffe sind dabei insbesondere gegen die den folgenden Gruppen angehörenden phytopathogenen Pilze wirksam: Ascomyceten (z.B. Venturia, Podosphaera, Erysiphe, Monilinia, Uncinula); Basidiomyceten (z.B. die Gattungen Hemileia, Rhizoctonia, Puccinia); Fungi imperfecti (z.B. Botrytis, Helminthosporium, Fusarium, Septoria, Cercospora und Alternaria). Ueberdies wirken die Verbindungen der Formel I systemisch. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden. Die erfindungsgemässen Wirkstoffe zeichnen sich durch besonders gute Pflanzenverträglichkeit aus.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen.

Eingeschlossen ist auch ein Verfahren zur Behandlung von Pflanzen, das sich durch Applikation der Verbindungen der Formel I bzw. der neuen Mittel auszeichnet.

Als Zielkulturen für die hierin offenbarten Indikationsgebiete gelten im Rahmen dieser Erfindung beispielsweise folgende Pflanzenarten: Getreide: (Weizen, Gerste, Roggen, Hafer, Reis, Sorghum und Verwandte); Rüben: (Zucker- und Futterrüben); Kern-, Stein- und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächste: (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Compositen).

Wirkstoffe der Formel I werden auf dem Agrarsektor üblicherweise in Form von Zusammensetzungen verwendet und können gleichzeitig oder nacheinander mit weiteren Wirkstoffen auf die zu behandelnde Fläche, Pflanzen oder Pflanzenteile appliziert werden. Diese weiteren Wirkstoffe können bei Pflanzenschutzmitteln sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Bei allen Formulierungen können geeignete Träger und Zusätze fest oder flüssig sein und entsprechen den in der entsprechenden Formulierungstechnik zweckdienlichen, nichttoxischen Stoffen, wie z.B. natürlichen oder regenerierten mineralischen Stoffen, Lösungs-, Dispergier-, Netz-, Haft-, Verdickungs- oder Bindemitteln.

Ein in der Landwirtschaft bevorzugtes Verfahren zum Aufbringen eines Wirkstoffes der Formel I bzw eines agrochemischen Mittels auf eine befallene Pflanze ist das Aufbringen auf das Blattwerk (Blattapplikation). Anzahl der Applikationen und Aufwandmenge richten sich dabei nach dem Befallsdruck für den entsprechenden Erreger (Pilzsorte). Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüberhinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der agrochemischen Formulierungstechnik üblichen Hilfsmittel eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige

Aufwandmengen liegen im allgemeinen bei 50 g bis 5 kg Aktivsubstanz (AS) je ha; bevorzugt 100 g bis 2 kg AS/ha, insbesondere bei 200 g bis 600 g AS/ha.

Die agrochemischen Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegegenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$—$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierhier gehören auch die Salze der Schwefelsäureester und Sulfonsäuren von Fettalkohol-Ethylenoxyd-Addukten. Die sulfonierten Benzimidazolderivate enthalten, vorzugsweise 2-Sulfonsäuregruppen und einen Fettsäurerest mit 8—22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4—14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglycolether, Polypropylen-Polyethylenoxydaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die in der agrochemischen Formulierungstechnik gebräuchlichen Tenside sind u.a. in folgenden Publikationen beschrieben:

"McCutcheon's Detergents and Emulsifiers Annual" MC Publishing Corp., Ringwood New Jersey, 1980

Sisely and Wood, "Encyclopedia of Surface Active Agents", Chemical Publishing Co., Inc. New York, 1980.

Die agrochemischen Zubereitungen enthalten in der Regel 0,1 bis 99%, insbesondere 0,1 bis 95% Wirkstoff der Formel I, 99,9 bis 1%, insbesondere 99,8 bis 5% eines festen oder flüssigen Zusatzstoffes, darunter 0 bis 25%, insbesondere 0,1 bis 25%, eines Tensides.

Während als Handelsware eher konzentrierte Mittel bevorzugt werden, verwendet der Endverbraucher in der Regel verdünnte Mittel.

Die agrochemischen Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen der näheren Erläuterung der Erfindung. Temperaturen sind in Celsiusgraden, Drucke in mbar angegeben. Prozente und Teile beziehen sich auf das Gewicht. RT bedeutet Raumtemperatur, h steht für Stunde.

## Herstellungsbeispiele:
### Beispiel 1

Herstellung von

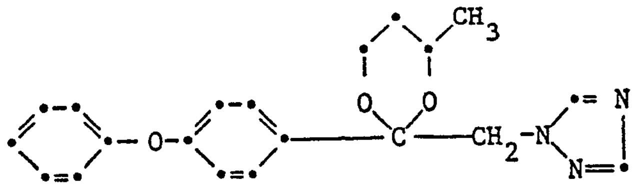

(6.8)

2-[p-(Phenoxy)-phenyl]-2-[1-(1H-1,2,4-triazolyl)-methyl]-4-methyl-1,3-dioxan

a) Herstellung des Zwischenproduktes

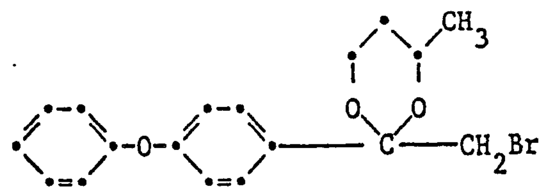

(XX)

2-[p-(Phenoxy)-phenyl]-2-brommethyl-4-methyl-1,3-dioxan

10 Teile 2-[p-(Phenoxy)-phenyl]-2-oxi-1-bromethan und 4 Teile 1,3-Butandiol werden in 40 ml absolutem Toluol in Gegenwart von 0,2 Teilen katalytisch wirkender p-Toluolsulfonsäure 3 h unter Rückfluss erhitzt, wobei gebildetes Wasser mit einem Wasserabscheider abgetrennt wird. Nach dem Abkühlen auf RT wird das Reaktionsgemisch zweimal mit je 20 ml Wasser gewaschen, über Natriumsulfat getrocknet, filtriert, das Lösungsmittel verdampft und das Rohprodukt aus Isopropanol umkristallisiert. Farblose Kristalle. Smp. 96—106°.

b) Herstellung des Endproduktes

3,3 Teile 1,2,4-Triazol-Natriumsalz und eine katalytisch wirkende Menge Kaliumjodid werden in 40 ml Dimethylsulfoxid zusammen mit 10,2 Teilen des nach a) hergestellten 2-[p-(Phenoxy)-phenyl]-2-brommethyl-4-methyl-1,3-dioxan 30 h bei einer Innentemperatur von +120° gerührt. Nach dem Abkühlen auf Raumtemperatur werden 300 ml Wasser zugegeben, dreimal mit je 30 ml Ethylacetat extrahiert, die vereinigten Extrakte zweimal mit je 20 ml Wasser gewaschen, über Natriumsulfat getrocknet, filtriert und das Lösungsmittel verdampft. Der ölige Rückstand wird säulenchromatographisch (Kieselgel/Ethylacetat) gereinigt. Nach dem Verdampfen des Laufmittels kristallisiert der ölige Rückstand nach Zugabe von Petrolether. Bräunliche Kristalle. Smp. 99,5—101°.

### Beispiel 2

Herstellung von

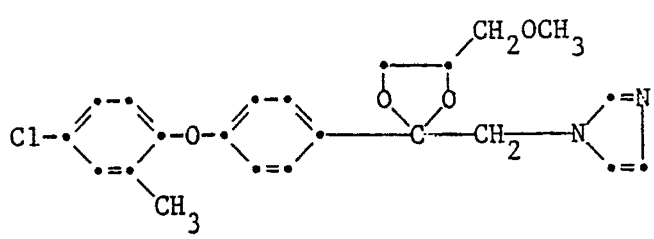

(3.226)

2-[p-(4-Chlor-2-methylphenoxy)-phenyl]-2-(1-imidazolylmethyl)-4-methoxymethyl-1,3-dioxolan

16 Teile 2-[p-(4-Chlor-2-methylphenoxy)-phenyl]-2-(1-imidazolylmethyl)-4-hydroxymethyl-1,3-dioxolan werden in 150 ml N,N-Dimethylformamid gelöst und unter Durchleiten von Stickstoff und Rühren mit 1,9 Teilen 55%iger Natriumhydrid-Dispersion versetzt und 2 h auf 80° erwärmt. Nach dem Abkühlen auf RT werden unter Rühren innerhalb 1 h 6,3 Teile Methyljodid zugetropft, das Reaktionsgemisch 2 h auf 60° erwärmt und mit 800 ml Eiswasser verdünnt und dreimal mit je 300 ml Ethylacetat extrahiert. Die vereinigten Extrakte werden zweimal mit je 50 ml Wasser gewaschen, über Natriumsulfat getrocknet, filtriert und das Lösungsmittel verdampft. Der Rückstand wird säulenchromatographisch (Kieselgel/Aceton-Ethylacetat (1:1)) gereinigt. Nach dem Verdampfen des Laufmittelgemisches wird das Diastereomerengemisch durch Behandeln mit Hexan zur Kristallisation gebracht. Beige Kristalle; Smp. 92—106°.

## Beispiel 3

Herstellung von

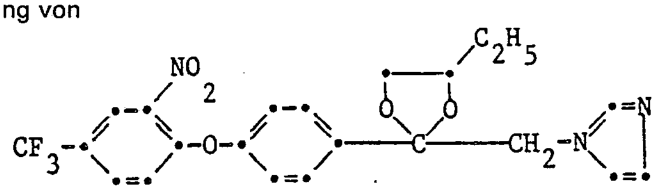

(3.263)

2-[p-(2-Nitro-4-trifluormethylphenoxy)-phenyl]-2-(1-imidazolylmethyl)-4-ethyl-1,3-dioxolan

8,3 Teile 2-(p-Hydroxyphenyl)-2-(1-imidazolylmethyl)-4-ethyl-1,3-dioxolan werden in 300 ml Dimethysulfoxid gelöst und mit 1,84 Teilen gemahlenem Kaliumhydroxid versetzt, wobei die Temperatur von 23° auf 36° ansteigt. Nach 2-stündigem Erwärmen auf 70° werden unter Rühren bei 70° 7,4 Teile 4-Chlor-3-nitrobenzotrifluorid in 100 ml Dimethylsulfoxid zugetropft und 3 h bei 70° gerührt. Nach dem Abkühlen auf RT wird das Reaktionsgemisch auf 2000 ml Wasser gegossen und zweimal mit je 200 ml Diethylether extrahiert. Die vereinigten Extrakte werden zweimal mit je 70 ml Wasser gewaschen, über Natriumsulfat getrocknet und das Lösungsmittel verdampft. Der ölige Rückstand wird säulenchromatographisch (Kieselgel/Ethylacetat) gereinigt. Nach dem Verdampfen des Laufmittels verbleibt das Diastereomerengemisch als zähflüssige Masse.

## Beispiel 4

Synthese von

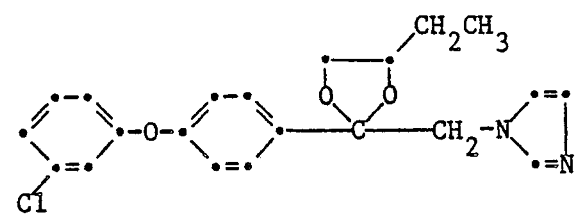

(3.12)

2-[p-(3-Chlorphenoxy)-phenyl]-2-(1-imidazolylmethyl)-4-ethyl-1,3-dioxolan

1,2 Teile Imidazol-Natriumsalz und eine katalytisch wirkende Menge Kaliumjodid werden in 50 ml Dimethylformamid zusammen mit 4 Teilen 2-[p-(3-Chlorphenoxy)-phenyl]-2-brommethyl-4-ethyl-1,3-dioxolan 17 h bei einer Innentemperatur von 125° gerührt. Nach dem Abkühlen wird das braune Reaktionsgemisch mit 150 ml Wasser versetzt, dreimal mit je 50 ml Ethylacetat extrahiert, die vereinigten Extrakte zweimal mit je 50 ml Wasser gewaschen, über Natriumsulfat getrocknet, filtriert und das Lösungsmittel verdampft. Das ölige Rohprodukt wird über eine 35 cm lange Kieselgelsäule mittels Aceton/Ethylacetat (1:1) chromatographiert. Nach dem Verdampfen des Laufmittels kristallisiert der ölige Rückstand nach Zugabe von Petrolether. Leicht gelbliche Kristalle. Smp. 69—71°C.

## Beispiel 5

Herstellung von

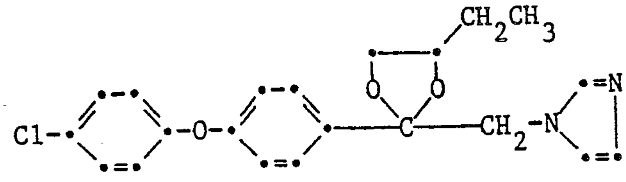

(3.7)

2-[p-(4-Chlorphenoxy)-phenyl]-2-(1-imidazolylmethyl)-4-ethyl-1,3-dioxolan

a) Synthese der Zwischenprodukte

α) Herstellung von

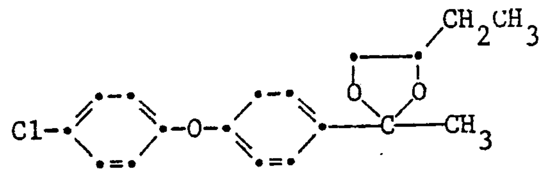

2-[p-(4-Chlorphenoxy)-phenyl]-2-methyl-4-ethyl-1,3-dioxolan

37 Teile 4-(p-Chlorphenoxy)-acetophenon und 18 Teile 1,2-Butandiol werden in 400 ml absolutem Toluol in Gegenwart von 2 Teilen katalytisch wirkender p-Toluolsulfonsäure 14 h am Wasserabscheider unter Rückfluss erhitzt. Nach den Abkühlen auf RT wird das Reaktionsgemisch zweimal mit je 400 ml Wasser gewaschen, über Natriumsulfat getrocknet, filtriert, das Lösungsmittel verdampft und das Rohprodukt zur Reinigung über eine 1 m lange Kieselgelsäule mittels Ligroin/Hexan/Ethylacetat/Toluol (5:3:1:1) chromatographiert. Das Produkt wird als leicht gelbliches Oel erhalten. $n_D^{22}$: 1,5527.

β) Herstellung von

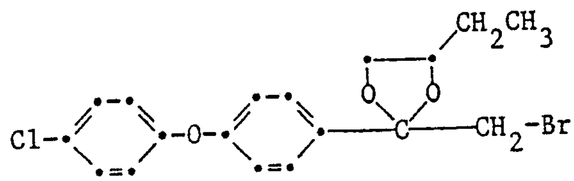

2-[p-(4-Chlorphenoxy)-phenyl]-2-brommethyl-4-ethyl-1,3-dioxolan

36,8 Teile des unter α) hergestellten 2-[p-(4-Chlorphenoxy)-phenyl]-2-methyl-4-ethyl-1,3-dioxolans werden in 350 ml Chloroform zum Sieden erhitzt. Unter Beleuchtung mittels einer 150 Watt Spotlampe werden 19,4 Teile Brom, gelöst in 50 ml Chloroform, zugetropft und anschliessend 2 h unter Rückfluss erhitzt. Nach dem Abkühlen auf RT wird das Reaktionsgemisch zweimal mit je 200 ml Wasser gewaschen, über Natriumsulfat getrocknet, filtriert und am Wasserstrahlvakuum das Lösungsmittel abgezogen. Zur Reinigung wird das Rohprodukt über eine 1 m lange Kieselgelsäule mittels Toluol chromatographiert. Das Produkt wird als gelbes Oel erhalten. $n_D^{23}$: 1,5805.

b) Synthese des Endproduktes

4,4 Teile Imidazol-Natriumsalz und eine katalytisch wirkende Menge Kaliumjodid werden in 80 ml Dimethylformamid zusammen mit 14,7 Teilen des nach β) hergestellten 2-[p-(4-Chlorphenoxy)-phenyl]-2-brommethyl-4-ethyl-1,3-dioxolan 17 h bei einer Badtemperatur von 125°C gerührt. Nach dem Abkühlen auf Raumtemperatur wird das Reaktionsgemisch auf 600 ml Wasser gegossen, dreimal mit je 200 ml Ethylacetat extrahiert, die vereinigten organischen Phasen zweimal mit je 200 ml Wasser gewaschen, über Natriumsulfat getrocknet, filtriert und das Lösungsmittel verdampft. Der ölige Rückstand wird über eine 50 cm lange Kieselgelsäule mittels Aceton/Ethylacetat (1:1) chromatographiert. Nach Verdampfen des Laufmittels wird das Produkt als braunes Oel erhalten; $n_D^{25}$: 1,5750.

## Beispiel 6

Synthese von

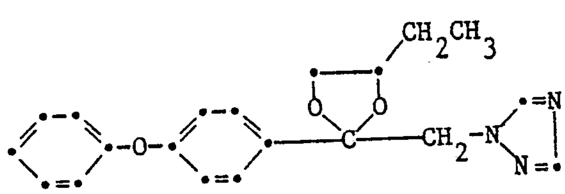

(1.9)

2-[p-(Phenoxy)-phenyl]-2-(1H-1,2,4-triazolylmethyl)-4-ethyl-1,3-dioxolan

17 Teile 2-[p-(Phenoxy)-phenyl]-3-brommethyl-4-ethyl-1,3-dioxolan, 8,4 Teile Kaliumcarbonat, 4,2 Teile 1,2,4-Triazol und eine katalytisch wirkende Menge Natriumjodid werden in 100 ml Dimethylsulfoxid 24 h bei einer Innentemperatur von 125° gerührt. Nach dem Abkühlen auf RT wird das Reaktionsgemisch auf 600 ml Wasser gegossen, dreimal mit je 200 ml Ethylacetat extrahiert, die vereinigten Extrakte zweimal mit je 200 ml Wasser gewaschen, über Natriumsulfat getrocknet, filtriert und das Lösungsmittel verdampft. Der ölige Rückstand wird über eine 50 cm lange Kieselgelsäule mittels Chloroform/Ether (1:1) chromatographiert. Nach dem Verdampfen des Laufmittels kristallisiert der ölige Rückstand nach Zugabe von Petrolether. Weisse Kristalle; Smp. 81,5—83,5°.

Beispiel 7

Synthese von

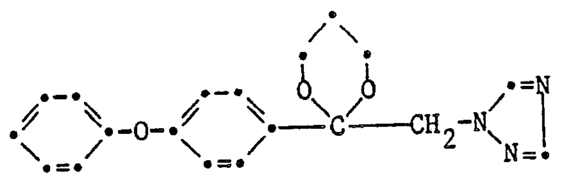

(6.1)

2-[p-(Phenoxy)-phenyl]-2-(1H-1,2,4-triazolylmethyl)-1,3-dioxan

14 Teile 2-[p-Phenoxyphenyl]-2-brommethyl-1,3-dioxan, 7,2 Teile Kaliumcarbonat, 3,6 Teile 1,2,4-Triazol und eine katalytisch wirkende Menge Kaliumjodid werden in 100 ml Dimethylsulfoxid 20 h bei einer Innentemperatur von 140° gerührt. Nach dem Abkühlen auf RT werden 600 ml Wasser zugegeben, dreimal mit je 200 ml Ether extrahiert, die vereinigten Extrakte zweimal mit je 200 ml Wasser gewaschen, über Natriumsulfat getrocknet, filtriert und das Lösungsmittel verdampft. Der ölige Rückstand wird über eine 50 cm lange Kieselgelsäule mittels Chloroform/Ether (1:1) chromatographiert. Nach Verdampfen des Laufmittels kristallisiert der Rückstand nach Zugabe von Petrolether. Weisse Kristalle; Smp. 129—130°.

Beispiel 8

Synthese von

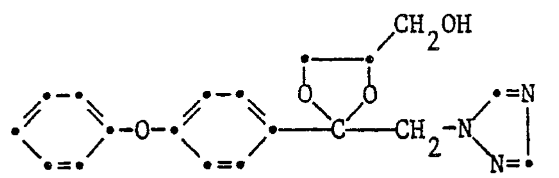

(1.23)

2-[p-(Phenoxy)-phenyl]-2-(1H-1,2,4-triazolylmethyl)-4-hydroxymethyl-1,3-dioxolan

4,5 Teile 2-[p-Phenoxyphenyl]-2-brommethyl-4-hydroxymethyl-1,3-dioxolan, 2,2 Teile Kaliumcarbonat, 1,1 Teile 1,2,4-Triazol und eine katalytisch wirkende Menge Kaliumjodid werden in 50 ml Dimethylsulfoxid 4 h bei einer Innentemperatur von 140° gerührt. Nach dem Abkühlen auf RT werden 600 ml Wasser zugegeben, zweimal mit je 200 ml Ethylacetat extrahiert, die vereinigten Extrakte zweimal mit je 200 ml Wasser gewaschen, über Natriumsulfat getrocknet, filtriert und das Lösungsmittel verdampft. Der ölige Rückstand wird über eine 50 cm lange Kieselgelsäule mittels Aceton chromatographiert. Nach dem Verdampfen des Laufmittels kristallisiert der ölige Rückstand nach Zugabe von Petrolether. Beige Kristalle; Smp. 111—122°.

Beispiel 9

Synthese von

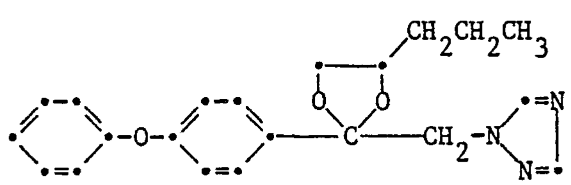

(1.17)

2-[p-(Phenoxy)-phenyl]-2-(1H-1,2,4-triazolylmethyl-4-n-propyl-1,3-dioxolan

10,3 Teile des Nitrats vom 1-(p-Phenoxyphenyl)-2-(1,2,4-triazolyl-1-yl)äthanon, 6,1 Teile 1,2-Pentandiol, 6,9 Teile p-Toluolsulfonsäure, 20 Teile 1-Pentanol und 200 Teile Xylol werden 6 Tage am Wasserabscheider unter Rückfluss erhitzt und nach dem Abkühlen auf RT zweimal mit je 200 ml verdünnter Natronlauge und zweimal mit je 200 ml Wasser gewaschen. Die organische Phase wird über Natriumsulfat getrocknet, filtriert und das Lösungsmittel verdampft. Der ölige Rückstand wird über eine 1 m lange Kieselgelsäule mittels Ethylacetat chromatographiert. Nach dem Verdampfen des Laufmittels kristallisiert der ölige Rückstand langsam aus. Beige Kristalle; Smp. 68,5—71°.

Auf analoge Weise lassen sich auch die nachfolgenden Endprodukte (falls nicht besonders vermerkt Diastereomerengemische mit unterschiedlichen Mischungsverhältnissen) der Formel I herstellen:

In den nachfolgenden Tabellen steht das Symbol A für ein Diastereomeres des Typs A und B entsprechend für ein Diastereomeres des Typs B.

Tabelle 1: Verbindungen der Formel

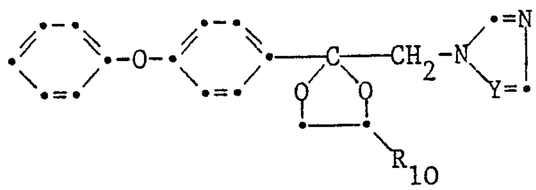

(XXI)

wobei isomere Formen mit umfasst sein sollen:

| Verb. Nr. | $R_{10}$ | Y | Salz | Physik. Konstante |
|---|---|---|---|---|
| 1.1 | H | N | - | Smp. 100-102° |
| 1.2 | H | N | $HNO_3$ | |
| 1.3 | H | CH | - | |
| 1.4 | $CH_3$ | N | - | Smp. 76,5-81° |
| 1.5 | $CH_3$ | N | HCl | |
| 1.6 | $CH_3$ | N | $CuCl_2$ | |
| 1.7 | $CH_3$ | CH | - | |
| 1.8 | $CH_3$ | N | $Mn(NO_3)_2$ | |
| 1.9 | $C_2H_5$ | N | - | Smp. 81,5-83,5° |
| 1.10 | $C_2H_5$ | N | $HNO_3$ | |
| 1.11 | $C_2H_5$ | N | $ZnCl_2$ | |
| 1.12 | $C_2H_5$ | N | $Mn(NO_3)_2$ | |
| 1.13 | $C_2H_5$ | N | $FeCl_3$ | |
| 1.14 | $C_2H_5$ | CH | - | hellbraunes Oel |
| 1.15 | $C_2H_5$ | CH | $CuCl_2$ | |
| 1.16 | $C_3H_7$-n | CH | - | |
| 1.17 | $C_3H_7$-n | N | - | Smp. 68,5-71° |
| 1.18 | $C_3H_7$-n | N | $ZnCl_2$ | |
| 1.19 | $C_3H_7$-n | N | HCl | |

Fortsetzung von Tabelle 1:

| Verb. Nr. | $R_{10}$ | Y | Salz | Physik. Konstante |
|---|---|---|---|---|
| 1.20 | $C_4H_9$-n | N | - | |
| 1.21 | $C_4H_9$-n | CH | - | |
| 1.22 | $CH_2Cl$ | N | - | |
| 1.23 | $CH_2OH$ | N | - | Smp. 111-122° |
| 1.24 | 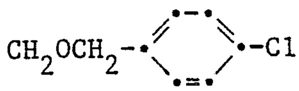 | N | - | zähes Oel; $n_D^{26}$=1.5865 A |
| 1.25 | 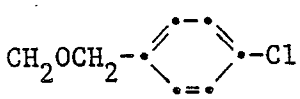 | N | - | Smp. 83-85° B |
| 1.26 | 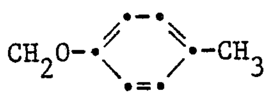 | N | - | Smp. 107-109° A |
| 1.27 | 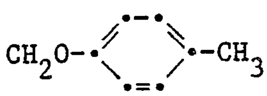 | N | - | Smp. 90-94° B |
| 1.28 | $CH_2OH$ | CH | - | |
| 1.29 | $CH_2OCH_3$ | CH | - | |
| 1.30 | $C_2H_5$ | N | 1/2$CuSO_4$ | |
| 1.31 | $CH_2OC_2H_5$ | N | - | |
| 1.32 | $CH_2OCH_3$ | N | - | |
| 1.33 | $CH_2OCH_2CH_2OCH_3$ | N | - | |

Tabelle 2: Verbindungen der Formel

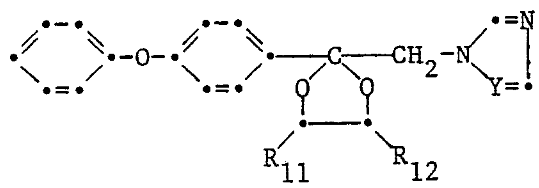

(XXII)

wobei die isomeren Formen mit umfasst sein sollen:

| Verb. Nr. | $R_{11}$ | $R_{12}$ | Y | Salz | Physik. Konstante |
|---|---|---|---|---|---|
| 2.1 | $CH_3$ | $C_2H_5$ | CH | - | |
| 2.2 | $CH_3$ | $C_2H_5$ | N | - | |
| 2.3 | $CH_3$ | $C_2H_5$ | CH | $HNO_3$ | |
| 2.4 | $CH_3$ | $C_2H_5$ | N | $HNO_3$ | |
| 2.5 | $CH_3$ | $C_3H_7$-n | CH | - | |
| 2.6 | $CH_3$ | $C_3H_7$-n | N | - | |
| 2.7 | $CH_3$ | $C_3H_7$-n | N | $HNO_3$ | |
| 2.8 | $CH_3$ | $C_3H_7$-n | N | $Mn(NO_3)_2$ | |
| 2.9 | $CH_3$ | $CH_3$ | CH | - | |
| 2.10 | $CH_3$ | $CH_3$ | CH | $CuCl_2$ | |
| 2.11 | $CH_3$ | $C_2H_5$ | CH | $Mn(NO_3)_2$ | |
| 2.12 | $CH_3$ | $C_2H_5$ | CH | $CuCl_2$ | |
| 2.13 | $CH_3$ | $C_2H_5$ | N | $CuCl_2$ | |
| 2.14 | $CH_3$ | $C_2H_5$ | N | $ZnCl_2$ | |
| 2.15 | $CH_3$ | $C_2H_5$ | N | $Mn(NO_3)_2$ | |
| 2.16 | $CH_3$ | $C_2H_5$ | N | $FeCl_3$ | |
| 2.17 | $CH_3$ | $CH_3$ | N | - | Oel; $n_D^{23} = 1.5643$ |
| 2.18 | $CH_3$ | $CH_3$ | N | $HNO_3$ | |
| 2.19 | $C_2H_5$ | $CH_3$ | CH | $MnCl_2$ | |
| 2.20 | $C_2H_5$ | $CH_3$ | N | $MnCl_2$ | |
| 2.21 | $C_2H_5$ | $CH_3$ | CH | $H_2SO_4$ | |
| 2.22 | $C_2H_5$ | $CH_3$ | CH | $ZnCl_2$ | |

Fortsetzung von Tabelle 2:

| Verb. Nr. | $R_{11}$ | $R_{12}$ | Y | Salz |
|---|---|---|---|---|
| 2.23 | $C_2H_5$ | $C_2H_5$ | CH | - |
| 2.24 | $C_2H_5$ | $C_2H_5$ | CH | $H_2SO_4$ |
| 2.25 | $C_2H_5$ | $C_2H_5$ | N | - |
| 2.26 | $C_2H_5$ | $C_2H_5$ | N | $HNO_3$ |
| 2.27 | $C_2H_5$ | $C_2H_5$ | N | HCl |
| 2.28 | $C_2H_5$ | $C_3H_7$-n | N | - |
| 2.29 | $C_2H_5$ | $C_3H_7$-i | N | - |
| 2.30 | $C_2H_5$ | $C_3H_7$-n | CH | - |
| 2.31 | $C_2H_5$ | $C_3H_7$-n | N | HCl |
| 2.32 | $C_2H_5$ | $C_2H_5$ | N | $Mn(NO_3)_2$ |
| 2.33 | $CH_3$ | $C_2H_5$ | N | $(COOH)_2$ |
| 2.34 | $CH_3$ | $C_2H_5$ | CH | $(COOH)_2$ |
| 2.35 | $CH_3$ | $C_3H_7$-i | N | - |
| 2.36 | $CH_3$ | $C_3H_7$-i | N | $H_2SO_4$ |
| 2.37 | $-(CH_2)_4-$ | | CH | - |
| 2.38 | $-(CH_2)_4-$ | | CH | $HNO_3$ |
| 2.39 | $-(CH_2)_4-$ | | N | - |
| 2.40 | $-(CH_2)_4-$ | | N | $Mn(NO_3)_2$ |
| 2.41 | $-(CH_2)_4-$ | | N | $(COOH)_2$ |
| 2.42 | $-(CH_2)_4-$ | | N | $ZnCl_2$ |
| 2.43 | $-(CH_2)_4-$ | | N | HCl |
| 2.44 | $-(CH_2)_4-$ | | CH | $ZnCl_2$ |

Tabelle 3: Verbindungen der Formel

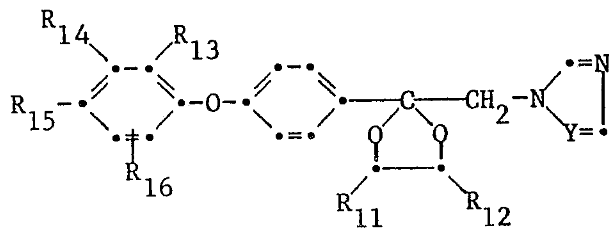

(XXIII)

wobei die isomeren Formen mit umfasst sein sollen:

| Verb. Nr. | $R_{11}$ | $R_{12}$ | $R_{13}$ | $R_{14}$ | $R_{15}$ | $R_{16}$ | Y | Salz | Physik. Konst. |
|---|---|---|---|---|---|---|---|---|---|
| 3.1 | H | $C_2H_5$ | Cl | H | Cl | H | N | - | Oel; $n_D^{23}$=1.5778 |
| 3.2 | H | $C_2H_5$ | $CH_3$ | H | $CH_3$ | H | N | - | Smp. 74–76° |
| 3.3 | H | $C_2H_5$ | $CH_3$ | H | $CH_3$ | H | N | $HNO_3$ | |
| 3.4 | H | $C_2H_5$ | Cl | H | Cl | H | CH | - | |
| 3.5 | H | $C_2H_5$ | Cl | H | Cl | H | N | $CuCl_2$ | |
| 3.6 | H | $C_2H_5$ | H | H | Cl | H | N | - | Smp. 74–80° |
| 3.7 | H | $C_2H_5$ | H | H | Cl | H | CH | - | Oel; $n_D^{25}$= 1.5750 |
| 3.8 | H | $C_2H_5$ | H | Cl | H | 5-Cl | N | - | Smp. 111,5-114° |
| 3.9 | H. | $C_2H_5$ | H | Cl | H | 6-Cl | CH | - | |
| 3.10 | $CH_3$ | $CH_3$ | H | Cl | H | 5-Cl | N | $Mn(NO_3)_2$ | |
| 3.11 | H | $C_2H_5$ | H | Cl | H | H | N | - | Smp. 96–98° |

Fortsetzung von Tabelle 3:

| Verb. Nr. | $R_{11}$ | $R_{12}$ | $R_{13}$ | $R_{14}$ | $R_{15}$ | $R_{16}$ | Y | Salz | Physik. Konst. |
|---|---|---|---|---|---|---|---|---|---|
| 3.12 | H | $C_2H_5$ | H | Cl | H | H | CH | - | Smp. 69-71° |
| 3.13 | H | $CH_3$ | H | Br | H | H | N | - | |
| 3.14 | $CH_3$ | $CH_3$ | Cl | H | Cl | H | N | - | |
| 3.15 | $CH_3$ | $CH_3$ | Cl | H | Cl | H | CH | - | |
| 3.16 | H | $C_3H_7$(n) | H | Cl | H | H | N | - | |
| 3.17 | H | $C_3H_7$(n) | H | Cl | H | 5-Cl | N | - | |
| 3.18 | H | $C_3H_7$(i) | H | Cl | H | 5-Cl | N | - | |
| 3.19 | H | $C_3H_7$(i) | H | Cl | H | 5-Cl | CH | - | |
| 3.20 | H | $C_3H_7$(i) | H | Cl | H | H | N | $HNO_3$ | |
| 3.21 | $C_2H_5$ | $C_2H_5$ | H | H | $CH_3$ | H | N | - | |
| 3.22 | $C_2H_5$ | $C_2H_5$ | $CH_3$ | H | $CH_3$ | H | N | - | |
| 3.23 | $CH_3$ | $CH_3$ | $CH_3$ | H | $CH_3$ | H | N | - | |
| 3.24 | H | $CH_3$ | H | $(CH=CH)_2$ | | H | N | - | |
| 3.25 | H | $CH_3$ | H | $(CH=CH)_2$ | | H | CH | - | |
| 3.26 | H | $C_2H_5$ | H | $(CH=CH)_2$ | | H | N | - | Oel; $n_D^{23}$=1.6086 |
| 3.27 | H | $C_2H_5$ | H | $(CH=CH)_2$ | | H | CH | - | |
| 3.28 | H | $C_2H_5$ | H | $(CH=CH)_2$ | | H | N | HCl | |
| 3.29 | H | $C_2H_5$ | H | $(CH=CH)_2$ | | H | N | $(COOH)_2$ | |

Fortsetzung von Tabelle 3:

| Verb. Nr. | $R_{11}$ | $R_{12}$ | $R_{13}$ | $R_{14}$ | $R_{15}$ | $R_{16}$ | Y | Salz |
|---|---|---|---|---|---|---|---|---|
| 3.30 | H | $C_2H_5$ | Cl | $(CH=CH)_2$ | | H | N | - |
| 3.31 | $CH_3$ | $CH_3$ | $CH_3$ | $(CH=CH)_2$ | | H | N | - |
| 3.32 | $CH_3$ | $CH_3$ | H | $(CH=CH)_2$ | | H | N | $CuCl_2$ |
| 3.33 | H | $CH_3$ | $(CH=CH)_2$ | | H | 2-Cl | N | - |
| 3.34 | H | $C_2H_5$ | $(CH=CH)_2$ | | H | 3-Cl | N | - |
| 3.35 | H | $C_2H_5$ | $(CH=CH)_2$ | | H | H | N | - |
| 3.36 | H | $C_2H_5$ | $(CH=CH)_2$ | | H | H | CH | - |
| 3.37 | H | $C_2H_5$ | $(CH=CH)_2$ | | H | H | N | $Mn(NO)_3$ |
| 3.38 | H | $C_2H_5$ | $(CH=CH)_2$ | | H | $2-CH_3$ | N | - |
| 3.39 | $CH_3$ | $CH_3$ | $(CH=CH)_2$ | | H | H | N | - |
| 3.40 | $CH_3$ | $CH_3$ | $(CH=CH)_2$ | | H | H | CH | - |
| 3.41 | H | $C_3H_7$-i | $(CH=CH)_2$ | | H | H | N | - |
| 3.42 | H | $C_2H_5$ | $(CH=CH)_2$ | | H | $2-CH_3$ | CH | HCl |
| 3.43 | $C_2H_5$ | $C_2H_5$ | $(CH=CH)_2$ | | H | H | N | - |
| 3.44 | H | $C_2H_5$ | F | H | H | H | CH | - |
| 3.45 | H | $C_2H_5$ | F | H | H | H | N | - |
| 3.46 | H | $C_2H_5$ | H | F | H | H | CH | - |

Fortsetzung von Tabelle 3:

| Verb. Nr. | $R_{11}$ | $R_{12}$ | $R_{13}$ | $R_{14}$ | $R_{15}$ | $R_{16}$ | Y | Salz | Physik. Konst. |
|---|---|---|---|---|---|---|---|---|---|
| 3.47 | H | $C_2H_5$ | H | F | H | H | N | - | |
| 3.48 | H | $CH_2OCH_3$ | F | H | H | H | CH | - | |
| 3.49 | H | $CH_2OCH_3$ | H | F | H | H | CH | - | |
| 3.50 | H | $CH_2OCH_3$ | H | F | H | H | N | - | |
| 3.51 | H | $C_2H_5$ | H | H | F | H | CH | - | Oel |
| 3.52 | H | $C_2H_5$ | H | H | F | H | N | - | |
| 3.53 | H | $CH_2OH$ | Cl | H | H | H | CH | - | |
| 3.54 | H | $CH_2OH$ | H | H | F | H | CH | - | |
| 3.55 | H | $CH_2OCH_3$ | H | H | F | H | N | - | zähe Masse |
| 3.56 | H | $C_2H_5$ | Cl | H | H | H | CH | - | |
| 3.57 | H | $CH_2OCH_3$ | Cl | H | H | H | CH | - | |
| 3.58 | H | $CH_2OCH_3$ | Cl | H | H | H | N | - | |
| 3.59 | H | $C_2H_5$ | Cl | H | H | H | N | - | |
| 3.60 | H | $CH_3$ | H | Cl | H | H | CH | - | |
| 3.63 | H | $CH_2OH$ | H | Cl | H | H | CH | - | |
| 3.64 | H | $CH_2OC_2H_5$ | H | Cl | H | H | CH | - | |
| 3.65 | H | $CH_2OCH_3$ | H | Cl | H | H | N | - | |
| 3.66 | $CH_3$ | $CH_3$ | H | Cl | H | H | CH | - | |

Fortsetzung von Tabelle 3:

| Verb. Nr. | $R_{11}$ | $R_{12}$ | $R_{13}$ | $R_{14}$ | $R_{15}$ | $R_{16}$ | Y | Salz | Physik. Konst. |
|---|---|---|---|---|---|---|---|---|---|
| 3.67 | H | $C_3H_7$-n | H | Cl | H | H | CH | - | |
| 3.68 | H | $CH_2OCH_3$ | H | Cl | H | H | CH | - | |
| 3.69 | H | $C_6H_{13}$-n | H | H | Cl | H | CH | - | |
| 3.70 | $CH_3$ | $CH_3$ | H | H | Cl | H | CH | - | Oel |
| 3.71 | H | $C_6H_5$ | H | H | Cl | H | CH | - | |
| 3.72 | H | $CH_2OC_2H_5$ | H | H | Cl | H | CH | - | |
| 3.73 | H | $CH_2OH$ | H | H | Cl | H | CH | - | |
| 3.74 | H | H | H | H | Cl | H | CH | - | |
| 3.75 | H | $C_3H_7$-n | H | H | Cl | H | CH | - | zähe Masse |
| 3.76 | H | $CH_3$ | H | H | Cl | H | CH | - | |
| 3.77 | H | $CH_2Cl$ | H | H | Cl | H | CH | - | |
| 3.78 | H | $CH_2OCH_3$ | H | H | Cl | H | CH | - | Oel |
| 3.79 | H | $CH_2OCH_2CH{=}CH_2$ | H | H | Cl | H | CH | - | |
| 3.80 | H | $CH_2OCH_2C{\equiv}CH$ | H | H | Cl | H | CH | - | |
| 3.81 | H | $CH_3$ | H | H | Cl | H | N | - | |
| 3.82 | H | $C_2H_5$ | H | H | Cl | H | N | $HNO_3$ | |
| 3.83 | H | $CH_2OCH_3$ | H | H | Cl | H | N | - | Oel |
| 3.84 | H | $CH_2O(CH_2)_2OCH_3$ | H | H | Cl | H | CH | - | |

Fortsetzung von Tabelle 3:

| Verb. Nr. | $R_{11}$ | $R_{12}$ | $R_{13}$ | $R_{14}$ | $R_{15}$ | $R_{16}$ | Y | Salz | Physik. Konst. |
|---|---|---|---|---|---|---|---|---|---|
| 3.85 | $CH_3$ | $CH_3$ | H | H | Cl | H | N | - | Oel |
| 3.86 | H | $C_2H_5$ | H | H | Cl | H | N | 1/2$CuSO_4$ | •$H_2O$ Smp.85-90° |
| 3.87 | H | $C_3H_7$-n | H | H | Cl | H | N | - | |
| 3.88 | H | $CH_2OC_2H_5$ | H | H | Cl | H | N | - | |
| 3.89 | H | $CH_2OCH_2CH{=}CH_2$ | H | H | Cl | H | N | - | |
| 3.90 | H | $CH_2O(CH_2)_2OCH_3$ | H | H | Cl | H | N | - | |
| 3.91 | H | $CH_2OCH_3$ | Br | H | H | H | CH | - | |
| 3.92 | H | $CH_2OCH_2C{\equiv}CH$ | H | H | Cl | H | N | - | |
| 3.93 | H | $CH_2O(CH_2)_2OCH_3$ | H | H | Cl | H | N | 1/2$CuSO_4$ | |
| 3.94 | H | $CH_2OCH_3$ | Br | H | H | H | N | - | |
| 3.95 | H | $C_2H_5$ | H | Br | H | H | CH | - | |
| 3.96 | H | $CH_2OCH_3$ | H | H | Br | H | N | - | |
| 3.97 | H | $CH_2OCH_3$ | H | H | J | H | CH | - | |
| 3.98 | H | $C_2H_5$ | H | H | J | H | N | - | |
| 3.99 | H | $CH_2OCH_3$ | H | Br | H | H | CH | - | |
| 3.100 | H | $C_2H_5$ | H | Br | H | H | N | - | |
| 3.101 | H | $CH_2OH$ | H | H | Br | H | CH | - | |
| 3.102 | H | $CH_2OCH_3$ | H | H | Br | H | CH | - | |

Fortsetzung von Tabelle 3:

| Verb. Nr. | $R_{11}$ | $R_{12}$ | $R_{13}$ | $R_{14}$ | $R_{15}$ | $R_{16}$ | Y | Salz |
|---|---|---|---|---|---|---|---|---|
| 3.103 | H | $C_2H_5$ | H | H | Br | H | N | – |
| 3.104 | H | $C_2H_5$ | $CH_3$ | H | H | H | N | – |
| 3.105 | H | $C_2H_5$ | H | $CH_3$ | H | H | N | – |
| 3.106 | H | $CH_2OCH_3$ | H | H | $CH_3$ | H | CH | – |
| 3.107 | $CH_3$ | $CH_3$ | $CH_3$ | H | H | H | CH | – |
| 3.108 | H | $CH_2OCH_3$ | H | $CH_3$ | H | H | CH | – |
| 3.109 | H | $CH_2OCH_3$ | H | $CH_3$ | H | H | N | – |
| 3.110 | H | $CH_2OH$ | H | H | $CH_3$ | H | CH | – |
| 3.111 | H | $C_2H_5$ | H | H | $CH_3$ | H | N | – |
| 3.112 | H | $CH_2OCH_3$ | $C_2H_5$ | H | H | H | CH | – |
| 3.113 | H | $C_2H_5$ | $C_2H_5$ | H | H | H | N | – |
| 3.114 | H | $CH_2OCH_3$ | H | $C_2H_5$ | H | H | CH | – |
| 3.115 | H | $C_3H_7$-n | H | $C_2H_5$ | H | H | N | – |
| 3.116 | H | $CH_2OH$ | H | H | $C_2H_5$ | H | CH | – |
| 3.117 | H | $C_2H_5$ | H | $C_2H_5$ | H | H | CH | – |
| 3.118 | H | $CH_2OCH_3$ | H | H | $C_2H_5$ | H | CH | – |
| 3.119 | H | $CH_2OCH_3$ | H | H | $C_2H_5$ | H | N | – |
| 3.120 | H | $CH_2OCH_3$ | $C_3H_7$-n | H | H | H | CH | – |

Fortsetzung von Tabelle 3:

| Verb. Nr. | $R_{11}$ | $R_{12}$ | $R_{13}$ | $R_{14}$ | $R_{15}$ | $R_{16}$ | Y | Salz | Physik. Konst. |
|---|---|---|---|---|---|---|---|---|---|
| 3.121 | H | $C_2H_5$ | H | $C_3H_7$-i | H | H | CH | - | |
| 3.122 | H | $CH_2OCH_3$ | H | $C_3H_7$-i | H | H | CH | - | |
| 3.123 | H | $CH_2OCH_3$ | H | H | $C_3H_7$-i | H | CH | - | |
| 3.124 | H | $CH_2OCH_3$ | H | H | $C_3H_7$-i | H | N | - | |
| 3.125 | H | $CH_2OCH_3$ | $C_4H_9$-t | H | H | H | N | - | |
| 3.126 | H | $C_2H_5$ | H | $C_4H_9$-t | H | H | N | - | |
| 3.127 | H | $C_2H_5$ | H | H | $C_4H_9$-t | H | CH | - | $n_D^{22,5} = 1.5508$ |
| 3.128 | H | $CH_2OCH_3$ | H | H | $C_2H_5C(CH_3)_2$ | H | CH | - | |
| 3.129 | H | $C_2H_5$ | H | H | $C_3H_7$-i | H | CH | - | |
| 3.130 | H | $CH_2OCH_3$ | H | $C_3H_7$-i | H | H | N | - | |
| 3.131 | H | $C_2H_5$ | H | H | $C_3H_7$-i | H | N | - | |
| 3.132 | H | $CH_2OCH_3$ | H | $C_4H_9$-t | H | H | N | - | |
| 3.133 | H | $C_2H_5$ | H | H | $C_4H_9$-t | H | N | - | $n_D^{22} = 1.5499$ |
| 3.134 | H | $CH_2OCH_3$ | H | H | $C_4H_9$-t | H | CH | - | |
| 3.135 | H | $CH_2OCH_3$ | $C_2H_5CH(CH_3)_2$ | H | H | H | CH | - | |
| 3.136 | H | $C_2H_5$ | H | H | $C_2H_5CH(CH_3)_2$ | H | CH | - | |
| 3.137 | H | $CH_2OCH_3$ | $OCH_3$ | H | H | H | CH | - | |
| 3.138 | H | $CH_2OCH_3$ | $OCH_3$ | H | H | H | N | - | |

Fortsetzung von Tabelle 3:

| Verb. Nr. | $R_{11}$ | $R_{12}$ | $R_{13}$ | $R_{14}$ | $R_{15}$ | $R_{16}$ | Y | Salz | Physik. Konst. |
|---|---|---|---|---|---|---|---|---|---|
| 3.139 | H | $CH_2OCH_3$ | H | H | $OC_4H_9$-n | H | CH | - | |
| 3.140 | H | $CH_2OCH_3$ | H | H | $OC_7H_{15}$-n | H | CH | - | |
| 3.141 | H | $C_2H_5$ | $OCH_3$ | H | H | H | N | - | |
| 3.142 | $CH_3$ | $CH_3$ | H | H | $OC_4H_9$-n | H | N | - | |
| 3.143 | H | $C_2H_5$ | H | H | $OC_7H_{15}$-n | H | N | - | |
| 3.144 | H | $C_2H_5$ | $NO_2$ | H | H | H | CH | - | |
| 3.145 | H | $CH_2OCH_3$ | $NO_2$ | H | H | H | CH | - | |
| 3.146 | H | $C_3H_7$-n | $NO_2$ | H | H | H | N | - | |
| 3.147 | H | $C_2H_5$ | H | $NO_2$ | H | H | N | - | |
| 3.148 | H | $CH_2OCH_3$ | H | H | $NO_2$ | H | CH | - | |
| 3.149 | H | $CH_2OCH_3$ | $CF_3$ | H | H | H | CH | - | |
| 3.150 | H | $C_3H_7$-n | H | $CF_3$ | H | H | CH | - | |
| 3.151 | H | $CH_2OCH_3$ | H | $NO_2$ | H | H | CH | - | |
| 3.152 | H | $C_2H_5$ | H | H | $NO_2$ | H | CH | - | |
| 3.153 | H | $C_2H_5$ | H | H | $NO_2$ | H | N | - | |
| 3.154 | H | $CH_2OCH_3$ | H | $CF_3$ | H | H | CH | - | |
| 3.155 | H | $C_2H_5$ | H | $CF_3$ | H | H | CH | - | zähe Masse |
| 3.156 | H | $C_2H_5$ | H | $CF_3$ | H | H | N | - | Smp. 80-83° |

Fortsetzung von Tabelle 3:

| Verb. Nr. | $R_{11}$ | $R_{12}$ | $R_{13}$ | $R_{14}$ | $R_{15}$ | $R_{16}$ | Y | Salz | Physik. Konstante |
|---|---|---|---|---|---|---|---|---|---|
| 3.157 | H | $CH_2OCH_3$ | H | $CF_3$ | H | H | N | - | |
| 3.158 | H | $CH_2OCH_3$ | H | H | $CF_3$ | H | CH | - | |
| 3.159 | H | $CH_2OCH_3$ | H | H | $CF_3$ | H | N | - | |
| 3.160 | H | $C_2H_5$ | H | H | $CF_3$ | H | CH | - | |
| 3.161 | H | $CH_2OCH_3$ | Cl | H | Cl | H | CH | - | |
| 3.162 | H | $C_2H_5$ | Cl | H | H | 5-Cl | CH | - | $n_D^{23} = 1.5837$ |
| 3.163 | H | $CH_2OCH_3$ | Cl | H | H | 5-Cl | N | - | |
| 3.164 | H | $C_2H_5$ | Cl | H | Cl | H | CH | - | $n_D^{22} = 1.5779$ |
| 3.165 | H | H | Cl | H | Cl | H | N | - | |
| 3.166 | H | $CH_2OCH_3$ | Cl | H | Cl | H | N | - | |
| 3.167 | H | $CH_2OCH_3$ | Cl | H | H | 5-Cl | CH | - | |
| 3.168 | H | $C_2H_5$ | Cl | H | H | 5-Cl | N | - | $n_D^{23} = 1.5788$ |
| 3.169 | H | $CH_2OCH_3$ | Cl | H | H | 6-Cl | CH | - | |
| 3.170 | H | $CH_2OCH_3$ | Cl | H | H | 6-Cl | N | - | |
| 3.171 | H | $C_2H_5$ | H | Cl | Cl | H | CH | - | $n_D^{23} = 1.5835$ |
| 3.172 | H | $C_2H_5$ | H | Cl | Cl | H | N | - | Smp. 90-97° |
| 3.173 | H | $C_2H_5$ | H | Cl | H | 5-Cl | CH | - | |
| 3.174 | H | $C_2H_5$ | Cl | H | H | 6-Cl | CH | - | |

Fortsetzung von Tabelle 3:

| Verb. Nr. | $R_{11}$ | $R_{12}$ | $R_{13}$ | $R_{14}$ | $R_{15}$ | $R_{16}$ | Y | Salz | Physik. Konst. |
|---|---|---|---|---|---|---|---|---|---|
| 3.175 | H | $C_2H_5$ | Cl | H | H | 6-Cl | N | – | |
| 3.176 | H | $CH_2OCH_3$ | H | Cl | Cl | H | CH | – | $n_D^{23} = 1.5836$ |
| 3.177 | H | $CH_2OCH_3$ | H | Cl | Cl | H | N | – | Smp. 48-60° |
| 3.178 | H | $CH_3$ | H | Cl | H | 5-Cl | CH | – | |
| 3.179 | H | $CH_2OCH_3$ | H | Cl | H | 5-Cl | CH | – | |
| 3.180 | H | $CH_2OCH_3$ | H | Cl | H | 5-Cl | N | – | |
| 3.181 | H | $CH_2OH$ | Br | H | Br | H | CH | – | |
| 3.182 | H | $C_2H_5$ | Br | H | Br | H | CH | – | |
| 3.183 | H | $C_2H_5$ | Br | H | Br | H | N | – | |
| 3.184 | H | $C_2H_5$ | Br | H | Cl | H | CH | – | |
| 3.185 | H | $C_2H_5$ | Br | H | Cl | H | N | – | |
| 3.186 | $CH_3$ | $CH_3$ | Br | H | Br | H | CH | – | |
| 3.187 | $CH_3$ | $CH_3$ | F | H | H | H | CH | – | |
| 3.188 | H | $CH_2OCH_3$ | Br | H | Br | H | N | – | |
| 3.189 | H | $CH_2OCH_3$ | Br | H | Cl | H | CH | – | |
| 3.190 | H | $CH_2OCH_3$ | Br | H | Cl | H | N | – | |
| 3.191 | H | $CH_2OCH_3$ | Cl | H | Br | H | CH | – | |
| 3.192 | H | $CH_2OCH_3$ | Cl | H | Br | H | N | – | |

Fortsetzung von Tabelle 3:

| Verb. Nr. | $R_{11}$ | $R_{12}$ | $R_{13}$ | $R_{14}$ | $R_{15}$ | $R_{16}$ | Y | Salz | Physik. Konst. |
|---|---|---|---|---|---|---|---|---|---|
| 3.193 | H | $C_2H_5$ | $CH_3$ | H | $CH_3$ | H | CH | - | Smp. 84-90° |
| 3.194 | H | $C_2H_5$ | Cl | H | Br | H | CH | - | |
| 3.195 | H | $C_2H_5$ | Cl | H | Br | H | N | - | |
| 3.196 | H | $CH_2OCH_3$ | $CH_3$ | H | $CH_3$ | H | CH | - | |
| 3.197 | H | $CH_2OCH_3$ | $CH_3$ | H | $CH_3$ | H | N | - | |
| 3.198 | H | $CH_2OC_2H_5$ | $CH_3$ | H | H | 5-$CH_3$ | N | - | |
| 3.199 | H | $C_2H_5$ | H | $CH_3$ | H | 5-$CH_3$ | CH | - | |
| 3.200 | H | $CH_2OCH_3$ | $C_4H_9$-t | H | $CH_3$ | H | CH | - | |
| 3.201 | H | $C_2H_5$ | $C_4H_9$-t | H | $C_4H_9$-t | H | N | - | |
| 3.202 | H | $CH_2OCH_3$ | $C_3H_7$-i | H | H | 5-$CH_3$ | CH | - | |
| 3.203 | H | $CH_2O(CH_2)_2OCH_3$ | $NO_2$ | H | $NO_2$ | H | CH | - | |
| 3.204 | H | $CH_2OC_2H_5$ | $CH_3$ | H | H | 5-$CH_3$ | CH | - | |
| 3.205 | H | $C_2H_5$ | H | $CH_3$ | H | 5-$CH_3$ | N | - | |
| 3.206 | H | $CH_2OCH_3$ | $C_4H_9$-t | H | $CH_3$ | H | N | - | |
| 3.207 | H | $CH_2OCH_3$ | $C_4H_9$-t | H | $C_4H_9$-t | H | CH | - | |
| 3.208 | H | $CH_2OCH_3$ | $C_3H_7$-i | H | H | 5-$CH_3$ | N | - | |
| 3.209 | H | $CH_2OC_2H_5$ | $NO_2$ | H | $NO_2$ | H | N | - | |
| 3.210 | H | $CH_2OCH_3$ | H | Cl | Cl | 6-Cl | CH | - | |

Fortsetzung von Tabelle 3:

| Verb. Nr. | $R_{11}$ | $R_{12}$ | $R_{13}$ | $R_{14}$ | $R_{15}$ | $R_{16}$ | Y | Salz | Physik. Konst. |
|---|---|---|---|---|---|---|---|---|---|
| 3.211 | H | $CH_2OCH_3$ | H | Cl | Cl | 6-Cl | N | - | |
| 3.212 | H | $C_2H_5$ | H | Cl | Cl | 6-Cl | CH | - | zähe Masse |
| 3.213 | H | $C_2H_5$ | H | Cl | Cl | 6-Cl | N | - | Oel; $n_D^{23}$ = 1.5863 |
| 3.214 | H | $CH_2OCH_3$ | Cl | H | Cl | 6-Cl | CH | - | |
| 3.215 | H | $CH_2OCH_3$ | H | Cl | Cl | 5-Cl | CH | - | |
| 3.216 | H | $CH_2OCH_3$ | H | Cl | Cl | 5-Cl | N | - | |
| 3.217 | $CH_3$ | $CH_3$ | Br | H | Br | 6-Br | N | - | |
| 3.218 | H | $CH_2OCH_3$ | Br | H | Br | 6-Br | N | - | |
| 3.219 | H | $CH_2OCH_3$ | $CH_3$ | $CH_3$ | H | 6-$CH_3$ | N | - | |
| 3.220 | H | $C_2H_5$ | $CH_3$ | H | Cl | H | CH | - | Smp. 94-96° |
| 3.221 | H | $C_2H_5$ | $CH_3$ | H | Cl | H | N | - | Smp. 78-80° |
| 3.222 | H | $CH_2OCH_3$ | Cl | H | Cl | 6-Cl | N | - | |
| 3.223 | H | $CH_2OH$ | H | Cl | Cl | 5-Cl | CH | - | |
| 3.224 | H | $CH_2OCH_3$ | Br | H | Br | 6-Br | CH | - | |
| 3.225 | H | $CH_2OCH_3$ | $CH_3$ | $CH_3$ | H | 5-$CH_3$ | CH | - | |
| 3.226 | H | $CH_2OCH_3$ | $CH_3$ | H | Cl | H | CH | - | Smp. 92-106° |
| 3.227 | H | $CH_2OCH_3$ | $CH_3$ | H | Cl | H | N | - | Smp. 101-103° |
| 3.228 | $CH_3$ | $CH_3$ | H | $CH_3$ | Cl | H | N | - | |

Fortsetzung von Tabelle 3:

| Verb. Nr. | $R_{11}$ | $R_{12}$ | $R_{13}$ | $R_{14}$ | $R_{15}$ | $R_{16}$ | Y | Salz | Physik. Konst. |
|---|---|---|---|---|---|---|---|---|---|
| 3.229 | H | $CH_2OCH_3$ | $C_4H_9$-t | H | Cl | H | N | - | |
| 3.230 | H | $CH_2OCH_3$ | H | $CH_3$ | H | 6-Cl | CH | - | |
| 3.231 | H | $CH_2OCH_3$ | H | $CH_3$ | Cl | H | CH | - | $n_D^{22,5}$= 1.5752,Oel |
| 3.232 | H | $C_2H_5$ | H | $CH_3$ | Cl | H | CH | - | $n_D^{22,5}$= 1.5748,Oel |
| 3.233 | H | $CH_2OCH_3$ | H | $CH_3$ | Cl | H | N | - | Smp. 88-93° |
| 3.234 | H | $C_2H_5$ | H | $CH_3$ | Cl | H | N | - | Smp. 72-75° |
| 3.235 | H | $C_3H_7$-n | $C_4H_9$-t | H | Cl | H | CH | - | |
| 3.236 | H | $CH_2OCH_3$ | $C_4H_9$-t | H | Cl | H | CH | - | |
| 3.237 | H | $CH_2OCH_3$ | H | $CH_3$ | H | 6-Cl | N | - | |
| 3.238 | H | $CH_2OCH_3$ | Br | H | $CH_3$ | H | CH | - | |
| 3.239 | H | $CH_2OCH_3$ | Br | H | $CH_3$ | H | N | - | |
| 3.240 | H | $CH_2OCH_3$ | $CH_3$ | H | Br | H | CH | - | |
| 3.241 | H | $CH_2OCH_3$ | $CH_3$ | H | Br | H | N | - | |
| 3.242 | H | $CH_2OCH_2CH=CH_2$ | H | $OCH_3$ | H | 6-Cl | CH | - | |
| 3.243 | H | $CH_2OCH_3$ | Cl | H | $NO_2$ | H | CH | - | |
| 3.244 | H | $CH_2OCH_3$ | Cl | H | $NO_2$ | H | N | - | |
| 3.245 | H | H | Br | H | $CH_3$ | H | N | - | |
| 3.246 | H | $CH_2Cl$ | Br | H | Br | H | CH | - | |

Fortsetzung von Tabelle 3:

| Verb. Nr. | $R_{11}$ | $R_{12}$ | $R_{13}$ | $R_{14}$ | $R_{15}$ | $R_{16}$ | Y | Salz | Physik.Konst. |
|---|---|---|---|---|---|---|---|---|---|
| 3.247 | H | $C_6H_5$ | $CH_3$ | H | Br | H | N | - | |
| 3.248 | H | $CH_2OCH_3$ | H | $OCH_3$ | H | 6-Cl | CH | - | |
| 3.249 | H | $CH_2OCH_3$ | H | $OCH_3$ | H | 6-Cl | N | - | |
| 3.250 | H | $CH_2OCH_2C{\equiv}CH$ | Cl | H | $NO_2$ | H | CH | - | |
| 3.251 | H | $CH_2OC_6H_5$ | $NO_2$ | H | Cl | H | CH | - | |
| 3.252 | H | $C_2H_5$ | $NO_2$ | H | Cl | H | CH | - | |
| 3.253 | H | $C_2H_5$ | $NO_2$ | H | Cl | H | N | - | |
| 3.254 | H | $C_2H_5$ | Cl | H | $CF_3$ | H | CH | - | |
| 3.255 | H | $CH_2OCH_3$ | Cl | H | $CF_3$ | H | CH | - | |
| 3.256 | H | $CH_2OCH_3$ | Cl | H | $CF_3$ | H | N | - | |
| 3.257 | H | $CH_2OCH_3$ | $NO_2$ | H | $CF_3$ | H | CH | - | |
| 3.258 | H | $CH_2OCH_3$ | $NO_2$ | H | Cl | H | CH | - | |
| 3.259 | H | $CH_2OCH_3$ | $NO_2$ | H | Cl | H | N | - | |
| 3.260 | H | $C_2H_5$ | H | H | $CF_3$ | H | N | - | |
| 3.261 | H | $C_2H_5$ | H | H | $NO_2$ | H | CH | - | |
| 3.262 | H | $C_2H_5$ | H | H | $NO_2$ | H | N | - | |
| 3.263 | H | $C_2H_5$ | $NO_2$ | H | $CF_3$ | H | CH | - | zähflüssige Masse |
| 3.264 | $CH_3$ | $CH_3$ | $NO_2$ | H | $CF_3$ | H | N | - | |

Fortsetzung von Tabelle 3:

| Verb. Nr. | $R_{11}$ | $R_{12}$ | $R_{13}$ | $R_{14}$ | $R_{15}$ | $R_{16}$ | Y | Salz | Physik. Konst. |
|---|---|---|---|---|---|---|---|---|---|
| 3.265 | H | $CH_2OCH_3$ | $NO_2$ | H | $CF_3$ | H | N | - | |
| 3.266 | H | $CH_2OCH_3$ | $CF_3$ | H | $NO_2$ | H | CH | - | |
| 3.267 | H | $C_2H_5$ | H | $CH_3$ | Cl | 5-$CH_3$ | CH | - | Smp. 75-78° |
| 3.268 | H | $CH_2OCH_3$ | H | $CH_3$ | Cl | 5-$CH_3$ | CH | - | |
| 3.269 | H | $CH_2OCH_3$ | H | $CH_3$ | Cl | 5-$CH_3$ | N | - | |
| 3.270 | H | $C_2H_5$ | H | $CH_3$ | Br | 5-$CH_3$ | CH | - | |
| 3.271 | H | $C_2H_5$ | $NO_2$ | H | $CF_3$ | H | N | - | |
| 3.272 | H | $CH_2OCH_3$ | $CF_3$ | H | $NO_2$ | H | N | - | |
| 3.273 | H | $CH_2OC_6H_5$ | H | $CH_3$ | Cl | 5-$CH_3$ | CH | - | |
| 3.274 | H | $C_2H_5$ | H | $CH_3$ | Cl | 5-$CH_3$ | N | - | Smp.64-73° |
| 3.275 | H | $CH_2OCH_3$ | H | $CH_3$ | Cl | 5-$CH_3$ | CH | - | |
| 3.276 | H | $C_2H_5$ | H | $CH_3$ | Br | 5-$CH_3$ | N | - | |
| 3.277 | H | $CH_2OCH_2C{\equiv}CH$ | H | $CH_3$ | Br | 5-$CH_3$ | N | - | |
| 3.278 | H | $C_2H_5$ | Cl | H | Cl | 5-$CH_3$ | N | - | |
| 3.279 | H | $C_2H_5$ | Cl | H | Cl | 6-$CH_3$ | CH | - | Smp. 70-74° |
| 3.280 | H | $CH_2OCH_3$ | Cl | H | Cl | 6-$CH_3$ | N | - | |
| 3.281 | H | $CH_3$ | Cl | H | Cl | 6-$CH_3$ | N | - | |
| 3.282 | H | $C_2H_5$ | $CH_3$ | H | Br | 6-Cl | CH | - | |

Fortsetzung von Tabelle 3:

| Verb. Nr. | $R_{11}$ | $R_{12}$ | $R_{13}$ | $R_{14}$ | $R_{15}$ | $R_{16}$ | Y | Salz | Physik. Konst. |
|---|---|---|---|---|---|---|---|---|---|
| 3.283 | H | $CH_2OCH_3$ | H | $CH_3$ | Br | 5-$CH_3$ | N | - | |
| 3.284 | H | $C_2H_5$ | Cl | H | Cl | 5-$CH_3$ | CH | - | |
| 3.285 | H | $CH_2OC_6H_5$ | Cl | H | Cl | 5-$CH_3$ | N | - | |
| 3.286 | H | $CH_2OCH_3$ | Cl | H | Cl | 6-$CH_3$ | CH | - | |
| 3.287 | H | $C_2H_5$ | Cl | H | Cl | 6-$CH_3$ | N | - | Smp. 98-101° |
| 3.288 | H | $C_3H_7$-n | Cl | H | Cl | 6-$CH_3$ | N | - | |
| 3.289 | H | $CH_2OCH_3$ | $CH_3$ | H | Br | 6-Cl | CH | - | |
| 3.290 | H | $CH_2OCH_3$ | $CH_3$ | H | Br | 6-Cl | N | - | |
| 3.291 | H | $CH_2OCH_3$ | $CF_3$ | H | $NO_2$ | 6-$CF_3$ | CH | - | |
| 3.292 | H | $C_2H_5$ | $NO_2$ | H | $NO_2$ | 6-$CF_3$ | CH | - | |
| 3.293 | H | $C_2H_5$ | $NO_2$ | H | $NO_2$ | 6-$CF_3$ | N | - | |
| 3.294 | H | $C_2H_5$ | $CH_3$ | H | Br | 6-Cl | N | - | |
| 3.295 | $CH_3$ | $CH_3$ | $CF_3$ | H | $NO_2$ | 6-$CF_3$ | CH | - | |
| 3.296 | H | $CH_3$ | $CF_3$ | H | $NO_2$ | 6-$CF_3$ | N | - | |
| 3.297 | H | $CH_2OCH_3$ | $CF_3$ | H | $NO_2$ | 6-$CF_3$ | N | - | |
| 3.298 | $CH_3$ | $CH_3$ | $NO_2$ | H | $NO_2$ | 6-$CF_3$ | CH | - | |
| 3.299 | H | H | $NO_2$ | H | $NO_2$ | 6-$CF_3$ | N | - | |
| 3.300 | H | $CH_2OCH_3$ | $NO_2$ | H | $NO_2$ | 6-$CF_3$ | N | - | |

Fortsetzung von Tabelle 3:

| Verb. Nr. | $R_{11}$ | $R_{12}$ | $R_{13}$ | $R_{14}$ | $R_{15}$ | $R_{16}$ | Y | Salz | Physik. Konst. |
|---|---|---|---|---|---|---|---|---|---|
| 3.301 | H | $CH_2OH$ | $(CH=CH)_2$ | | H | H | CH | - | |
| 3.302 | H | $CH_2OCH_3$ | $(CH=CH)_2$ | | H | H | CH | - | |
| 3.303 | H | $C_3H_7$-n | $(CH_2=CH)_2$ | | H | H | N | - | |
| 3.304 | H | $CH_2OCH_3$ | $(CH_2=CH)_2$ | | H | H | N | - | |
| 3.305 | H | $CH_2OH$ | $(CH_2=CH)_2$ | | Cl | H | CH | - | |
| 3.306 | H | $C_2H_5$ | $(CH_2=CH)_2$ | | Cl | H | N | - | |
| 3.307 | H | $CH_2OCH_3$ | $(CH_2=CH)_2$ | | Cl | H | CH | - | |
| 3.308 | H | $CH_2OCH_3$ | $(CH_2=CH)_2$ | | Cl | H | N | - | |
| 3.309 | H | $CH_2OCH_3$ | H | $(CH=CH)_2$ | | H | CH | - | |
| 3.310 | H | $CH_2OCH_3$ | H | $(CH=CH)_2$ | | H | N | - | |
| 3.311 | H | $C_2H_5$ | Cl | Cl | H | H | CH | - | Oel; $n_D^{23}$=1.5832 |
| 3.312 | $CH_3$ | $CH_3$ | Cl | Cl | H | H | CH | - | |
| 3.313 | H | $CH_2OCH_3$ | Cl | Cl | H | H | CH | - | |
| 3.314 | H | $C_2H_5$ | Cl | Cl | H | H | N | - | Oel; $n_D^{23}$=1.5796 |
| 3.315 | H | $CH_2OCH_3$ | Cl | Cl | H | H | N | - | |
| 3.316 | H | $CH_2OCH_3$ | $CH_3$ | $CH_3$ | H | H | CH | - | |
| 3.317 | H | $CH_2OCH_3$ | $CH_3$ | $CH_3$ | H | H | N | - | |
| 3.318 | $-(CH_2)_4-$ | | Cl | H | Br | H | N | - | |

Fortsetzung von Tabelle 3:

| Verb. Nr. | $R_{11}$ | $R_{12}$ | $R_{13}$ | $R_{14}$ | $R_{15}$ | $R_{16}$ | Y | Salz |
|---|---|---|---|---|---|---|---|---|
| 3.319 | H | $C_2H_5$ | $CH_3$ | $CH_3$ | H | H | CH | – |
| 3.320 | H | $CH_2OCH_3$ | $CH_3$ | $CH_3$ | H | H | N | – |
| 3.321 | H | $C_2H_5$ | Cl | Cl | Cl | H | CH | – |
| 3.322 | H | $C_2H_5$ | Cl | Cl | Cl | H | N | – |
| 3.323 | H | $CH_2OCH_3$ | Cl | Cl | Cl | H | CH | – |
| 3.324 | H | $CH_2OCH_3$ | Cl | Cl | H | 6-Cl | CH | – |
| 3.325 | H | $CH_2OCH_3$ | Cl | Cl | H | 6-Cl | N | – |
| 3.326 | $CH_3$ | $CH_3$ | Cl | Cl | H | 6-Cl | CH | – |
| 3.327 | H | $CH_2OCH_3$ | Cl | Cl | H | 6-Cl | N | – |
| 3.328 | H | $CH_2OCH_3$ | H | $CH_3$ | H | 5-$CH_3$ | N | – |
| 3.329 | H | $C_3H_7$-n | F | H | H | H | CH | – |
| 3.330 | H | $CH_2OH$ | F | H | H | H | CH | – |
| 3.331 | H | $CH_2OC_2H_5$ | F | H | H | H | CH | – |
| 3.332 | H | $C_3H_7$-n | F | H | H | H | N | – |
| 3.333 | H | $CH_2OCH_3$ | F | H | H | H | N | – |
| 3.334 | H | $CH_3$ | H | F | H | H | CH | – |
| 3.335 | $CH_3$ | $CH_3$ | H | F | H | H | CH | – |
| 3.336 | H | $C_3H_7$-n | H | F | H | H | CH | – |

Fortsetzung von Tabelle 3:

| Verb. Nr. | $R_{11}$ | $R_{12}$ | $R_{13}$ | $R_{14}$ | $R_{15}$ | $R_{16}$ | Y | Salz | Physik. Konst. |
|---|---|---|---|---|---|---|---|---|---|
| 3.337 | H | $CH_2OH$ | H | F | H | H | CH | - | |
| 3.338 | H | $C_3H_7$-n | H | F | H | H | N | - | |
| 3.339 | H | $CH_2OH$ | H | F | H | H | N | - | |
| 3.340 | H | $CH_2OCH_3$ | H | H | F | H | CH | - | Oel |
| 3.341 | H | H | H | H | F | H | CH | - | |
| 3.342 | H | $CH_3$ | H | H | F | H | CH | - | |
| 3.343 | $CH_3$ | $CH_3$ | H | H | F | H | CH | - | |
| 3.344 | H | $C_3H_7$-n | H | H | F | H | CH | - | |
| 3.345 | H | H | H | H | F | H | N | - | |
| 3.346 | H | $CH_3$ | H | H | F | H | N | - | |
| 3.347 | $CH_3$ | $CH_3$ | H | H | F | H | N | - | |
| 3.348 | H | $CH_2OH$ | H | H | F | H | N | - | zähe Masse |
| 3.349 | H | $CH_2OC_2H_5$ | H | H | F | H | N | - | |
| 3.350 | H | $CH_2OC_2H_5$ | Cl | H | H | H | CH | - | |
| 3.351 | H | $CH_3$ | Cl | H | H | H | N | - | |
| 3.352 | $CH_3$ | $CH_3$ | Cl | H | H | H | N | - | |
| 3.353 | H | $CH_3$ | Br | H | H | H | CH | - | |
| 3.354 | $CH_3$ | $CH_3$ | Br | H | H | H | CH | - | |

Fortsetzung von Tabelle 3:

| Verb. Nr. | $R_{11}$ | $R_{12}$ | $R_{13}$ | $R_{14}$ | $R_{15}$ | $R_{16}$ | Y | Salz |
|---|---|---|---|---|---|---|---|---|
| 3.355 | H | $C_2H_5$ | Br | H | H | H | CH | – |
| 3.356 | H | $CH_2OH$ | Br | H | H | H | CH | – |
| 3.357 | H | $CH_3$ | Br | H | H | H | N | – |
| 3.358 | $CH_3$ | $CH_3$ | Br | H | H | H | N | – |
| 3.359 | H | $C_2H_5$ | Br | H | H | H | N | – |
| 3.360 | H | $CH_2OH$ | Br | H | H | H | N | – |
| 3.361 | H | $CH_2OC_2H_5$ | Br | H | H | H | CH | – |
| 3.362 | H | $CH_2OC_2H_5$ | Br | H | H | H | N | – |
| 3.363 | H | H | H | Br | H | H | CH | – |
| 3.364 | H | $CH_3$ | H | Br | H | H | CH | – |
| 3.365 | H | $C_3H_7$-n | H | Br | H | H | CH | – |
| 3.366 | H | $CH_2OH$ | H | Br | H | H | CH | – |
| 3.367 | H | $CH_3$ | H | Br | H | H | N | – |
| 3.368 | $CH_3$ | $CH_3$ | H | Br | H | H | N | – |
| 3.369 | H | $C_3H_7$-n | H | Br | H | H | N | – |
| 3.370 | H | $CH_2OH$ | H | Br | H | H | N | – |
| 3.371 | H | $CH_2OCH_3$ | H | Br | H | H | N | – |

Fortsetzung von Tabelle 3:

| Verb. Nr. | $R_{11}$ | $R_{12}$ | $R_{13}$ | $R_{14}$ | $R_{15}$ | $R_{16}$ | Y | Salz | Physik. Konst. |
|---|---|---|---|---|---|---|---|---|---|
| 3.372 | H | $C_2H_5$ | H | H | Br | H | CH | - | Oel |
| 3.373 | H | $C_3H_7$-n | H | H | Br | H | CH | - | |
| 3.374 | H | $CH_2OC_2H_5$ | H | H | Br | H | CH | - | |
| 3.375 | $CH_3$ | $CH_3$ | H | H | Br | H | N | - | |
| 3.376 | H | $C_3H_7$-n | H | H | Br | H | N | - | Oel |
| 3.377 | H | $CH_2OH$ | H | H | Br | H | N | - | |
| 3.378 | H | $CH_2OC_2H_5$ | H | H | Br | H | N | - | |
| 3.379 | H | $C_2H_5$ | H | H | J | H | CH | - | |
| 3.380 | H | $CH_2OH$ | H | H | J | H | CH | - | |
| 3.381 | H | $CH_2OCH_3$ | H | H | J | H | N | - | |
| 3.382 | H | $CH_2OH$ | H | H | J | H | N | - | |

Tabelle 4: Verbindungen der Formel

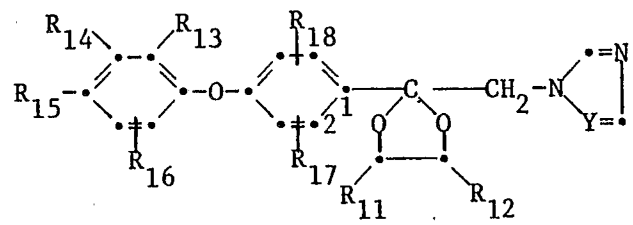

(XXIV)

wobei die isomeren Formen mit umfasst sein sollen:

| Verb. Nr. | $R_{11}$ | $R_{12}$ | $R_{13}$ | $R_{14}$ | $R_{15}$ | $R_{16}$ | Y | $R_{17}$ | $R_{18}$ | Salz |
|---|---|---|---|---|---|---|---|---|---|---|
| 4.1 | H | $C_2H_5$ | Cl | H | Cl | H | N | 3-$NO_2$ | H | - |
| 4.2 | H | $C_2H_5$ | $CH_3$ | H | H | H | N | 2-$CH_3$ | H | - |
| 4.3 | H | $C_2H_5$ | $CH_3$ | H | $CH_3$ | H | N | 3-$CH_3$ | H | $HNO_3$ |
| 4.4 | H | $C_2H_5$ | Cl | H | Cl | H | CH | 2-$CH_3$ | 6-$CH_3$ | - |
| 4.5 | H | $C_2H_5$ | Cl | H | Cl | H | N | 2-Cl | H | $CuCl_2$ |
| 4.6 | H | $C_2H_5$ | H | H | Cl | H | N | 3-$NO_2$ | H | - |
| 4.7 | H | $C_2H_5$ | H | H | Cl | H | CH | 3-$NO_2$ | H | - |
| 4.8 | H | $C_2H_5$ | H | Cl | H | 5-Cl | N | 2-Cl | H | - |
| 4.9 | $C_2H_5$ | H | H | Cl | H | 6-Cl | CH | 2-$NO_2$ | H | - |
| 4.10 | $CH_3$ | $CH_3$ | H | Cl | H | 6-Cl | N | 2-$NO_2$ | H | $Mn(NO_3)_2$ |
| 4.11 | H | $C_2H_5$ | Cl | H | H | H | N | 2-Cl | H | - |

Fortsetzung von Tabelle 4:

| Verb. Nr. | $R_{11}$ | $R_{12}$ | $R_{13}$ | $R_{14}$ | $R_{15}$ | $R_{16}$ | Y | $R_{17}$ | $R_{18}$ | Salz | Physik. Konstante |
|---|---|---|---|---|---|---|---|---|---|---|---|
| 4.12 | H | $C_2H_5$ | H | Cl | H | H | CH | 2-Br | H | - | |
| 4.13 | H | $CH_3$ | H | Br | H | H | N | 2-$CH_3$ | H | - | |
| 4.14 | $CH_3$ | $CH_3$ | Cl | H | Cl | H | N | 2-Cl | 6-Cl | - | |
| 4.15 | H | $CH_3$ | H | H | Cl | H | CH | 2-$CH_3$ | H | - | Harz |
| 4.16 | H | $C_3H_7$-n | Cl | H | H | H | N | 2-Cl | H | - | |
| 4.17 | H | $C_3H_7$-n | H | Cl | H | 6-Cl | N | 2-Cl | H | - | |
| 4.18 | H | $C_3H_7$-i | H | Cl | H | 6-Cl | N | 2-$OCH_3$ | H | - | |
| 4.19 | H | $C_3H_7$-i | H | Cl | H | 5-Cl | CH | 2-$OCH_3$ | 6-$OCH_3$ | - | |
| 4.20 | H | $C_3H_7$-i | H | Cl | H | H | N | 2-Cl | H | $HNO_3$ | |
| 4.21 | $C_2H_5$ | $C_2H_5$ | H | H | $CH_3$ | H | N | 2-Cl | H | - | |
| 4.22 | $C_2H_5$ | $C_2H_5$ | $CH_3$ | H | $CH_3$ | H | N | 2-Cl | H | - | |
| 4.23 | $CH_3$ | $CH_3$ | H | F | H | H | N | 2-$CH_3$ | H | - | braungelbes Harz |
| 4.24 | H | $CH_3$ | $(CH=CH)_2$ | | H | H | N | 2-Cl | H | - | |
| 4.25 | H | $C_2H_5$ | $(CH=CH)_2$ | | H | H | N | 2-Cl | 6-Cl | - | |
| 4.26 | H | $CH_3$ | $(CH=CH)_2$ | | H | H | CH | 2-$NO_2$ | H | - | |
| 4.27 | H | $C_2H_5$ | $(CH=CH)_2$ | | H | H | N | 2-Cl | H | - | |
| 4.28 | H | $C_3H_7$-i | $(CH=CH)_2$ | | H | H | N | 2-Cl | H | - | |
| 4.29 | $CH_3$ | $CH_3$ | $(CH=CH)_2$ | | H | H | N | 3-Cl | H | - | |

Fortsetzung von Tabelle 4:

| Verb. Nr. | $R_{11}$ | $R_{12}$ | $R_{13}$ | $R_{14}$ | $R_{15}$ | $R_{16}$ | Y | $R_{17}$ | $R_{18}$ | Salz |
|---|---|---|---|---|---|---|---|---|---|---|
| 4.30 | $CH_3$ | $CH_3$ | $(CH=CH)_2$ | | H | H | N | 2-Cl | H | - |
| 4.31 | $CH_3$ | $CH_3$ | $(CH=CH)_2$ | | H | 2-Cl | N | 2-Br | H | - |
| 4.32 | H | $C_2H_5$ | $(CH=CH)_2$ | | H | H | N | 2-Cl | H | HCl |
| 4.33 | H | $CH_3$ | $(CH=CH)_2$ | | $CH_3$ | H | N | 3-Cl | H | - |
| 4.34 | H | $CH_3$ | $(CH=CH)_2$ | | H | 2-$CH_3$ | N | H | H | HCl |
| 4.35 | H | $CH_3$ | $(CH=CH)_2$ | | H | H | N | H | H | - |
| 4.36 | H | $CH_3$ | $(CH=CH)_2$ | | H | H | CH | H | H | - |
| 4.37 | H | $CH_3$ | H | $(CH=CH)_2$ | | H | N | 3-$NO_2$ | H | - |
| 4.38 | H | $CH_3$ | H | $(CH=CH)_2$ | | H | CH | 3-$NO_2$ | H | - |
| 4.39 | H | $CH_3$ | H | $(CH=CH)_2$ | | H | N | H | H | $CuCl_2$ |
| 4.40 | H | $CH_3$ | Cl | $(CH=CH)_2$ | | H | N | 2-Cl | H | - |
| 4.41 | $CH_3$ | $CH_3$ | Cl | $(CH=CH)_2$ | | H | N | 2-Cl | H | - |
| 4.42 | H | $C_2H_5$ | Cl | $(CH=CH)_2$ | | H | N | 3-Cl | H | - |
| 4.43 | H | $C_3H_7$-i | $CH_3$ | $(CH=CH)_2$ | | H | N | H | H | - |
| 4.44 | H | $CH_3$ | $CH_3$ | $(CH=CH)_2$ | | H | N | 2-Cl | 6-Cl | - |
| 4.45 | H | $CH_3$ | Cl | $(CH=CH)_2$ | | 3-Cl | N | 2-Cl | H | - |
| 4.46 | H | $CH_3$ | $CH_3$ | $(CH=CH)_2$ | | 3-$CH_3$ | N | H | H | - |
| 4.47 | H | $CH_3$ | $CH_3$ | $(CH=CH)_2$ | | H | N | 2-Cl | H | - |

Fortsetzung von Tabelle 4:

| Verb. Nr. | $R_{11}$ | $R_{12}$ | $R_{13}$ | $R_{14}$ | $R_{15}$ | $R_{16}$ | Y | $R_{17}$ | $R_{18}$ | Salz | Physik. Konstante |
|---|---|---|---|---|---|---|---|---|---|---|---|
| 4.48 | $CH_3$ | H | $CH_3$ | H | H | H | N | 2-$CH_3$ | H | - | dunkelgelbes Harz |
| 4.49 | $CH_3$ | $CH_3$ | $CH_3$ | H | $OCH_3$ | H | N | 2-$CH_3$ | H | - | |
| 4.50 | $CH_3$ | H | H | H | $OCH_3$ | H | N | 2-$CH_3$ | H | - | Harz |
| 4.51 | $C_3H_7$-n | H | $CH_3$ | H | H | H | N | 2-$CH_3$ | H | - | |
| 4.52 | $C_3H_7$-n | H | $CH_3$ | H | $OCH_3$ | H | N | 2-$CH_3$ | H | - | |
| 4.53 | $C_2H_5$ | H | $CH_3$ | H | $OCH_3$ | H | N | 2-$CH_3$ | H | - | |
| 4.54 | H | $CH_2OCH_3$ | F | H | H | H | CH | 2-Cl | H | - | |
| 4.55 | H | $C_2H_5$ | F | H | H | H | N | 2-$CH_3$ | H | - | Harz |
| 4.56 | H | $CH_2OCH_3$ | H | F | H | H | CH | 2-Cl | H | - | |
| 4.57 | H | $C_2H_5$ | H | H | F | H | N | 2-$CH_3$ | H | - | hellbraunes Harz |
| 4.58 | H | $C_2H_5$ | H | F | H | H | N | 2-Cl | H | - | |
| 4.59 | H | $C_2H_5$ | H | H | F | H | CH | 2-$CH_3$ | H | - | braunes Harz |
| 4.60 | H | $C_2H_5$ | H | H | F | H | CH | 2-Cl | H | - | |
| 4.61 | H | $CH_2OCH_3$ | Cl | H | H | H | CH | 2-Cl | H | - | |
| 4.62 | H | $CH_2OCH_3$ | Cl | H | H | H | N | 2-Cl | H | - | |
| 4.63 | H | $CH_2OH$ | H | Cl | H | H | CH | 2-Cl | H | - | |
| 4.64 | H | $C_2H_5$ | H | H | Cl | H | CH | 2-$CH_3$ | H | - | |
| 4.65 | H | $CH_3$ | H | H | F | H | N | 2-$CH_3$ | H | - | viskoses Oel |

Fortsetzung von Tabelle 4:

| Verb. Nr. | $R_{11}$ | $R_{12}$ | $R_{13}$ | $R_{14}$ | $R_{15}$ | $R_{16}$ | Y | $R_{17}$ | $R_{18}$ | Salz | Physik. Konstante |
|---|---|---|---|---|---|---|---|---|---|---|---|
| 4.66 | H | $CH_3$ | H | H | F | H | CH | 2-$CH_3$ | H | – | |
| 4.67 | H | $CH_2OCH_3$ | H | H | Cl | H | N | 2-Cl | H | – | |
| 4.68 | H | $C_2H_5$ | H | Br | H | H | CH | 2-$CH_3$ | H | – | |
| 4.69 | H | $C_2H_5$ | F | H | H | H | CH | 2-$CH_3$ | H | – | |
| 4.70 | H | $CH_3$ | H | H | F | H | CH | 2-$CH_3$ | H | – | hellbraunes Harz |
| 4.71 | H | $CH_2OCH_3$ | Br | H | H | H | N | 2-Cl | H | – | |
| 4.72 | H | $CH_2OCH_3$ | H | Br | H | H | CH | 2-Cl | H | – | |
| 4.73 | H | $CH_2OCH_3$ | H | H | Br | H | CH | 2-Cl | H | – | |
| 4.74 | H | $C_2H_5$ | H | H | Br | H | N | 2-Cl | H | – | |
| 4.75 | H | $CH_2OCH_3$ | H | H | J | H | CH | 2-Cl | H | – | |
| 4.76 | H | $CH_2OCH_3$ | H | H | J | H | N | 2-Cl | H | – | |
| 4.77 | H | $C_3H_7$-n | $CH_3$ | H | H | H | N | 2-$CH_3$ | H | $HNO_3$ | Smp. 105–110° |
| 4.78 | H | $CH_3$ | $CH_3$ | H | H | H | N | 2-$CH_3$ | H | $HNO_3$ | Smp. 92–95° B |
| 4.79 | H | $C_2H_7$-n | H | $CH_3$ | H | H | CH | 2-Cl | H | – | |
| 4.80 | H | $CH_2OCH_3$ | $CH_3$ | H | H | H | CH | 2-Cl | H | – | |
| 4.81 | H | $CH_3$ | $CH_3$ | H | H | H | N | 2-$CH_3$ | H | $HNO_3$ | Smp. 129–134° A |
| 4.82 | H | $CH_2OCH_3$ | H | H | $CH_3$ | H | N | 2-Cl | H | – | |
| 4.83 | H | $CH_2OCH_3$ | H | H | $CH_3$ | H | CH | 2-$CH_3$ | H | – | |

Fortsetzung von Tabelle 4:

| Verb. Nr. | $R_{11}$ | $R_{12}$ | $R_{13}$ | $R_{14}$ | $R_{15}$ | $R_{16}$ | Y | $R_{17}$ | $R_{18}$ | Salz | Physik. Konstante |
|---|---|---|---|---|---|---|---|---|---|---|---|
| 4.84 | H | $C_2H_5$ | H | H | $C_2H_5$ | H | N | 2-Cl | H | – | |
| 4.85 | H | $CH_2OCH_3$ | $C_3H_7$-i | H | H | H | CH | H | H | – | |
| 4.86 | H | $CH_2OCH_3$ | $C_3H_7$-i | H | H | H | CH | 2-$NO_2$ | H | – | |
| 4.87 | H | $CH_2OCH_3$ | H | $C_2H_5$ | H | H | CH | 3-$CH_3$ | H | – | |
| 4.88 | H | $CH_2OCH_3$ | H | $C_2H_5$ | H | H | N | 2-Cl | H | – | |
| 4.89 | $CH_3$ | $CH_3$ | H | H | $OCH_3$ | H | N | 2-$CH_3$ | H | – | Harz B |
| 4.90 | H | $C_3H_7$-n | H | H | $OCH_3$ | H | N | 2-$CH_3$ | H | – | Oel |
| 4.91 | H | $C_3H_7$-n | H | H | $OCH_3$ | H | N | 2-$CH_3$ | H | – | Oel B |
| 4.92 | $CH_3$ | $CH_3$ | H | H | $OCH_3$ | H | N | 2-$CH_3$ | H | – | Harz A |
| 4.93 | H | $C_2H_5$ | H | H | $OCH_3$ | H | N | 2-$CH_3$ | H | – | Harz |
| 4.94 | H | $C_3H_7$-n | H | H | $OCH_3$ | H | N | 2-$CH_3$ | H | – | Oel A |
| 4.95 | H | $CH_2OC_2H_5$ | $NO_2$ | H | H | H | CH | 2-Br | H | – | |
| 4.96 | H | $CH_2OCH_3$ | H | H | $NO_2$ | H | CH | 2-Cl | H | – | |
| 4.97 | H | $CH_2OH$ | H | $CF_3$ | H | H | CH | 3-$CH_3$ | H | – | |
| 4.98 | H | $CH_2OCH_3$ | H | H | $CF_3$ | H | CH | 2-Cl | H | – | |
| 4.99 | H | $CH_2OCH_3$ | H | Cl | H | H | CH | 2-Cl | H | – | |
| 4.100 | H | $CH_2OCH_3$ | Br | H | Br | H | CH | 2-Cl | H | – | |
| 4.101 | H | $CH_2OCH_3$ | Cl | H | Cl | H | CH | 2-Cl | H | – | |

Fortsetzung von Tabelle 4:

| Verb. Nr. | $R_{11}$ | $R_{12}$ | $R_{13}$ | $R_{14}$ | $R_{15}$ | $R_{16}$ | Y | $R_{17}$ | $R_{18}$ | Salz |
|---|---|---|---|---|---|---|---|---|---|---|
| 4.102 | $CH_2OH$ | H | $(CH=CH)_2$ | | H | H | CH | 2-Cl | H | - |
| 4.103 | $C_2H_5$ | H | $CH_3$ | H | Cl | H | CH | 2-$OCH_3$ | 6-$OCH_3$ | - |
| 4.104 | $C_2H_5$ | H | $(CH=CH)_2$ | | H | H | CH | 2-Cl | H | - |
| 4.105 | $CH_2OCH_3$ | H | $(CH=CH)_2$ | | H | H | CH | 2-Cl | H | - |
| 4.106 | $CH_2OCH_3$ | H | $(CH=CH)_2$ | | H | H | N | H | H | - |
| 4.107 | $C_2H_5$ | H | H | $(CH=CH)_2$ | | H | CH | 2-Cl | H | - |
| 4.108 | $C_2H_5$ | H | H | $(CH=CH)_2$ | | H | N | 2-Cl | H | - |
| 4.109 | $C_2H_5$ | H | $(CH=CH)_2$ | | Cl | H | CH | 2-Cl | H | - |
| 4.110 | $C_2H_5$ | H | $(CH=CH)_2$ | | Cl | H | N | 2-Cl | H | - |
| 4.111 | $(CH_2)_4$ | | H | H | Cl | H | CH | H | H | - |
| 4.112 | $CH_2OCH_3$ | H | Cl | Cl | H | H | CH | 2-Cl | H | - |
| 4.113 | $CH_3$ | H | H | H | Cl | H | N | 2-$CH_3$ | H | - |
| 4.114 | $C_2H_5$ | H | H | H | Cl | H | N | 2-$OCH_3$ | 6-$OCH_3$ | - |
| 4.115 | $C_2H_5$ | H | H | H | Cl | H | CH | 2-$OCH_3$ | 6-$OCH_3$ | - |
| 4.116 | $C_2H_5$ | H | H | H | Cl | H | N | 2-$CH_3$ | H | - |
| 4.117 | $CH_3$ | $CH_3$ | H | H | F | H | N | 2-$CH_3$ | H | - |
| 4.118 | $CH_3$ | $CH_3$ | H | H | F | H | CH | 2-$CH_3$ | H | - |
| 4.119 | $CH_3$ | $C_2H_5$ | H | H | F | H | N | 2-$CH_3$ | H | - |

Fortsetzung von Tabelle 4:

| Verb. Nr. | $R_{11}$ | $R_{12}$ | $R_{13}$ | $R_{14}$ | $R_{15}$ | $R_{16}$ | Y | $R_{17}$ | $R_{18}$ | Salz | Physik. Konstante |
|---|---|---|---|---|---|---|---|---|---|---|---|
| 4.120 | $CH_3$ | $C_2H_5$ | H | H | F | H | CH | 2-$CH_3$ | H | – | |
| 4.121 | H | H | H | H | F | H | N | 2-$CH_3$ | H | – | |
| 4.122 | H | H | H | H | F | H | CH | 2-$CH_3$ | H | – – | |
| 4.123 | H | -$C_3H_7$-n | H | H | F | H | N | 2-$CH_3$ | H | – | Harz |
| 4.124 | H | -$C_3H_7$-n | H | H | F | H | CH | 2-$CH_3$ | H | – | Harz |
| 4.125 | H | $CH_2OCH_3$ | H | H | F | H | N | 2-$CH_3$ | H | – | |
| 4.126 | H | $CH_2OCH_3$ | H | H | F | H | CH | 2-$CH_3$ | H | – | |
| 4.127 | H | H | H | H | Cl | H | N | 2-$CH_3$ | H | – | |
| 4.128 | H | H | H | H | Cl | H | CH | 2-$CH_3$ | H | – | hochviskoses Oel |
| 4.129 | H | $CH_3$ | H | H | Cl | H | N | 3-$CH_3$ | H | – | |
| 4.130 | H | $C_2H_5$ | H | H | Cl | H | N | 3-$CH_3$ | H | – | |
| 4.131 | H | H | H | H | Cl | H | N | 3-$CH_3$ | H | – | |
| 4.132 | H | $CH_3$ | H | H | Cl | H | CH | 3-$CH_3$ | H | – | |
| 4.133 | H | $C_2H_5$ | H | H | Cl | H | CH | 3-$CH_3$ | H | – | |
| 4.134 | H | $C_3H_7$-n | H | H | Cl | H | CH | 3-$CH_3$ | H | – | |
| 4.135 | H | $CH_2OCH_3$ | H | H | Cl | H | CH | 3-$CH_3$ | H | – | |

Tabelle 5: Verbindungen der Formel

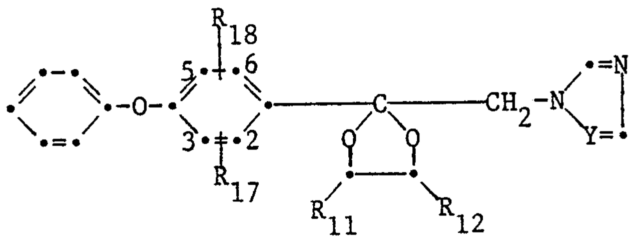

wobei die isomeren Formen mit umfasst sein sollen:

| Verb. Nr. | $R_{11}$ | $R_{12}$ | $R_{17}$ | $R_{18}$ | Y | Salz | Physik. Konstante |
|---|---|---|---|---|---|---|---|
| 5.1 | $CH_3$ | $C_2H_5$ | 2-Cl | H | CH | - | |
| 5.2 | $CH_3$ | $C_2H_5$ | 2-Cl | H | N | - | |
| 5.3 | $CH_3$ | $C_2H_5$ | 2-Cl | 6-Cl | CH | $HNO_3$ | |
| 5.4 | $CH_3$ | $C_2H_5$ | 2-$CH_3$ | H | N | $HNO_3$ | |
| 5.5 | $CH_3$ | $C_3H_7$-n | 2-Cl | H | CH | - | |
| 5.6 | $CH_3$ | $C_3H_7$-n | 2-Cl | H | N | - | |
| 5.7 | $CH_3$ | $C_3H_7$-n | 3-Cl | H | N | $HNO_3$ | |
| 5.8 | $CH_3$ | $C_3H_7$-n | 3-Cl | 6-Cl | N | $Mn(NO_3)_2$ | |
| 5.9 | $CH_3$ | $CH_3$ | 2-Cl | H | CH | - | |
| 5.10 | $CH_3$ | $CH_3$ | 2-Cl | 6-Cl | CH | $CuCl_2$ | |
| 5.11 | $CH_3$ | $C_2H_5$ | 2-Cl | H | CH | $Mn(NO_3)_2$ | |
| 5.12 | $CH_3$ | $C_2H_5$ | 2-$CH_3$ | 6-$CH_3$ | CH | $CuCl_2$ | |
| 5.13 | $CH_3$ | $CH_3$ | 2-$CH_3$ | H | N | $HNO_3$ | Smp. 158-160° |
| 5.14 | $CH_3$ | $CH_3$ | 2-$CH_3$ | H | N | HBr | Smp. 192-204° |
| 5.15 | $CH_3$ | $C_2H_5$ | 2-$CH_3$ | H | N | $Mn(NO_3)_2$ | |
| 5.16 | $CH_3$ | $C_2H_5$ | 2-$NO_2$ | H | N | $FeCl_3$ | |
| 5.17 | $CH_3$ | $CH_3$ | 2-Cl | H | N | - | |
| 5.18 | $CH_3$ | $CH_3$ | 2-Cl | 5-Cl | N | $HNO_3$ | |
| 5.19 | $C_2H_5$ | $CH_3$ | 3-Cl | H | CH | $MnCl_2$ | |
| 5.20 | $C_2H_5$ | $CH_3$ | 2-$CH_3$ | H | N | $MnCl_2$ | |
| 5.21 | $C_2H_5$ | $CH_3$ | 2-$CH_3$ | H | N | $CuCl_2$ | |
| 5.22 | $C_2H_5$ | $CH_3$ | 2-Cl | 5-Cl | N | $ZnCl_2$ | |
| 5.23 | $C_2H_5$ | $C_2H_5$ | 2-Br | H | CH | - | |
| 5.24 | H | $C_2H_5$ | 2-$OCH_3$ | 6-$OCH_3$ | CH | - | |

Fortsetzung von Tabelle 5:

| Verb. Nr. | $R_{11}$ | $R_{12}$ | $R_{17}$ | $R_{18}$ | Y | Salz | Physik. Konstante |
|---|---|---|---|---|---|---|---|
| 5.25 | H | H | 2-Cl | H | N | - | |
| 5.26 | H | H | 2-Cl | H | CH | - | |
| 5.27 | H | H | 2-Cl | 6-Cl | CH | - | |
| 5.28 | H | H | 2-Cl | H | N | $Mn(NO_3)_2$ | |
| 5.29 | H | H | 2-Cl | 6-Cl | N | - | |
| 5.30 | H | $CH_3$ | 2-Cl | H | N | - | |
| 5.31 | H | $CH_3$ | 2-Cl | H | N | $HNO_3$ | |
| 5.32 | H | $CH_3$ | 2-Cl | 6-Cl | N | - | |
| 5.33 | H | $CH_3$ | 2-Cl | H | CH | - | |
| 5.34 | H | $CH_3$ | 2-$OCH_3$ | 6-$OCH_3$ | CH | - | |
| 5.35 | H | $CH_3$ | 3-Cl | H | N | - | |
| 5.36 | H | $CH_3$ | 2-$CH_3$ | H | N | $HNO_3$ | Smp. 132-134° |
| 5.37 | H | $CH_3$ | 3-$CH_3$ | H | N | - | |
| 5.38 | H | $C_2H_5$ | 3-$CH_3$ | H | CH | $CuCl_2$ | |
| 5.39 | H | $C_2H_5$ | 2-$CH_3$ | 6-$CH_3$ | N | - | |
| 5.40 | H | $C_2H_5$ | 2-$CH_3$ | H | N | $HNO_3$ | Smp. 108-110° |
| 5.41 | H | $C_2H_5$ | 2-Br | 5-Br | N | - | |
| 5.42 | H | $C_2H_5$ | 2-Cl | H | N | - | |
| 5.43 | H | $C_3H_7$-n | 2-Cl | H | N | - | |
| 5.44 | H | $C_3H_7$-n | 2-Cl | 6-Cl | N | - | |
| 5.45 | H | $C_3H_7$-i | 2-$CH_3$ | H | N | - | |
| 5.46 | H | $C_3H_7$-i | 2-$CH_3$ | H | CH | - | |
| 5.47 | H | $C_3H_7$-i | 2-$OCH_3$ | 6-$OCH_3$ | N | $MnCl_2$ | |
| 5.48 | $C_2H_5$ | $C_2H_5$ | 2-Cl | H | N | - | |
| 5.49 | $C_2H_5$ | $C_2H_5$ | 2-Cl | H | N | $HNO_3$ | |
| 5.50 | $C_2H_5$ | $C_2H_5$ | 2-Cl | 6-Cl | N | HCl | |
| 5.51 | H | H | 2-$OCH_3$ | 6-$OCH_3$ | CH | - | |
| 5.52 | $C_2H_5$ | $C_3H_7$-i | 2-$CH_3$ | H | N | - | |

Fortsetzung von Tabelle 5:

| Verb. Nr. | $R_{11}$ | $R_{12}$ | $R_{17}$ | $R_{18}$ | Y | Salz | Physik. Konstante |
|---|---|---|---|---|---|---|---|
| 5.53 | H | $C_2H_5$ | 2-$OCH_3$ | 6-$OCH_3$ | N | - | |
| 5.54 | H | $C_2H_5$ | 2-$OCH_3$ | 6-$OCH_3$ | N | - | |
| 5.55 | H | H | 2-$OCH_3$ | 6-$OCH_3$ | N | - | |
| 5.56 | $CH_3$ | $C_2H_5$ | 3-Cl | H | N | $(COOH)_2$ | |
| 5.57 | $CH_3$ | $C_2H_5$ | 2-$CH_3$ | 6-$CH_3$ | CH | $(COOH)_2$ | |
| 5.58 | $CH_3$ | $C_3H_7$-i | 2-Cl | H | N | - | |
| 5.59 | $CH_3$ | $C_3H_7$-i | 2-Cl | 6-Cl | N | $H_2SO_4$ | |
| 5.60 | $-(CH_2)_4-$ | | 2-Cl | H | CH | - | |
| 5.61 | $-(CH_2)_4-$ | | 2-Cl | H | CH | $HNO_3$ | |
| 5.62 | $-(CH_2)_4-$ | | 2-Cl | H | N | - | |
| 5.63 | $-(CH_2)_4-$ | | 3-Cl | H | N | $Mn(NO_3)_2$ | |
| 5.64 | $-(CH_2)_4-$ | | 3-Cl | 6-Cl | N | $(COOH)_2$ | |
| 5.65 | $-(CH_2)_4-$ | | 3-Cl | 5-Cl | N | $ZnCl_2$ | |
| 5.66 | $-(CH_2)_4-$ | | 3-Cl | 6-Cl | N | HCl | |
| 5.67 | $-(CH_2)_4-$ | | 2-Cl | H | CH | $ZnCl_2$ | |
| 5.68 | H | H | 2-$CH_3$ | H | CH | - | |
| 5.69 | $CH_3$ | $CH_3$ | 2-$CH_3$ | H | CH | - | |
| 5.70 | H | $C_3H_7$-n | 2-$CH_3$ | H | CH | - | gelbes Harz |
| 5.71 | H | $CH_2OCH_3$ | 2-$CH_3$ | H | CH | - | Harz |
| 5.72 | H | $CH_3$ | 2-$CH_3$ | H | CH | - | |
| 5.73 | H | $C_2H_5$ | 2-$CH_3$ | H | CH | - | |
| 5.74 | H | $CH_2OH$ | 2-$CH_3$ | H | CH | - | |
| 5.75 | H | $CH_2OCH_3$ | 2-$CH_3$ | H | N | - | Oel |
| 5.76 | H | $C_2H_5$ | 2-$CH_3$ | H | N | - | Harz |
| 5.77 | H | H | 2-$CH_3$ | H | N | - | Smp. 103-105° |
| 5.78 | H | $C_3H_7$-n | 2-$CH_3$ | H | N | - | Harz |
| 5.79 | H | $CH_2OH$ | 2-Cl | H | CH | - | |
| 5.80 | H | $C_2H_5$ | 2-Br | H | CH | - | |
| 5.81 | H | $C_2H_5$ | 2-Cl | H | CH | - | |

Fortsetzung von Tabelle 5:

| Verb. Nr. | $R_{11}$ | $R_{12}$ | $R_{17}$ | $R_{18}$ | Y | Salz |
|---|---|---|---|---|---|---|
| 5.82 | H | $CH_2OCH_3$ | 2-Cl | H | CH | - |
| 5.83 | H | $CH_2OCH_3$ | 2-Cl | H | N | - |
| 5.84 | H | $CH_2OH$ | 2-Br | H | CH | - |
| 5.85 | H | $CH_2OCH_3$ | 2-Br | H | CH | - |
| 5.86 | $CH_3$ | $CH_3$ | 2-Br | H | N | - |
| 5.87 | H | $CH_2OCH_3$ | 2-Br | H | N | - |
| 5.88 | H | $CH_2OH$ | 3-$NO_2$ | H | CH | - |
| 5.89 | H | $C_3H_7$-n | 3-$NO_2$ | H | N | - |
| 5.90 | H | $C_2H_5$ | 2-Br | H | N | - |
| 5.91 | H | $C_2H_5$ | 3-$NO_2$ | H | CH | - |
| 5.92 | H | $CH_3$ | 3-$NO_2$ | H | CH | - |
| 5.93 | H | $C_2H_5$ | 3-$NO_2$ | H | N | - |
| 5.94 | H | $CH_3$ | 3-$NO_2$ | H | N | - |
| 5.95 | H | $CH_2OH$ | 3-$CH_3$ | H | CH | - |
| 5.96 | H | $CH_2OCH_3$ | 3-$CH_3$ | H | N | - |
| 5.97 | H | $C_2H_5$ | 2-$NO_2$ | H | CH | - |
| 5.98 | H | $CH_2OCH_3$ | 2-$NO_2$ | H | CH | - |
| 5.99 | H | $CH_2OCH_3$ | 2-$NO_2$ | H | N | - |
| 5.100 | H | $C_2H_5$ | 3-$CH_3$ | H | CH | - |
| 5.101 | H | $CH_2OCH_3$ | 3-$CH_3$ | H | CH | - |
| 5.102 | H | $C_2H_5$ | 3-$CH_3$ | H | N | - |
| 5.103 | H | $CH_2OH$ | 2-$NO_2$ | H | CH | - |
| 5.104 | H | $C_2H_5$ | 2-$NO_2$ | H | N | - |

Tabelle 6: Verbindungen der Formel

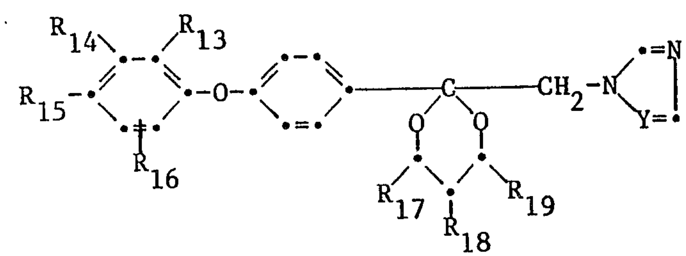

(XXVI)

wobei isomere Formen mit umfasst sein sollen:

| Verb. Nr. | $R_{13}$ | $R_{14}$ | $R_{15}$ | $R_{16}$ | $R_{17}$ | $R_{18}$ | $R_{19}$ | Y | Salz | Physik. Konstante |
|---|---|---|---|---|---|---|---|---|---|---|
| 6.1 | H | H | H | H | H | H | H | N | - | Smp. 129-130° |
| 6.2 | H | H | H | H | H | $C_2H_5$ | H | N | - | |
| 6.3 | H | H | Cl | H | H | $CH_3$ | H | CH | - | |
| 6.4 | H | H | H | H | $CH_3$ | $CH_3$ | H | N | - | |
| 6.5 | H | H | H | H | $CH_3$ | $CH_3$ | H | N | $HNO_3$ | |
| 6.6 | H | H | H | H | $CH_3$ | $CH_3$ | $CH_3$ | N | - | |
| 6.7 | H | H | H | H | $CH_3$ | $CH_3$ | $CH_3$ | N | $HNO_3$ | |
| 6.8 | H | H | H | H | $CH_3$ | H | H | N | - | Smp. 99,5-101° |
| 6.9 | Cl | H | Cl | H | $CH_3$ | $CH_3$ | $CH_3$ | N | $Mn(NO_3)_2$ | |
| 6.10 | $CH_3$ | H | $CH_3$ | H | $CH_3$ | $CH_3$ | $CH_3$ | N | $CuCl_2$ | |

Fortsetzung von Tabelle 6:

| Verb. Nr. | $R_{13}$ | $R_{14}$ | $R_{15}$ | $R_{16}$ | $R_{17}$ | $R_{18}$ | $R_{19}$ | Y | Salz | Physik. Konstante |
|---|---|---|---|---|---|---|---|---|---|---|
| 6.11 | $CH_3$ | H | $CH_3$ | H | $CH_3$ | $CH_3$ | $CH_3$ | N | $(COOH)_2$ | |
| 6.12 | Cl | H | H | 6-Cl | $CH_3$ | $CH_3$ | $CH_3$ | CH | $HNO_3$ | |
| 6.13 | Cl | H | H | 5-Cl | $CH_3$ | $CH_3$ | $CH_3$ | CH | $CuCl_2$ | |
| 6.14 | Cl | H | Cl | 6-Cl | $CH_3$ | $CH_3$ | $CH_3$ | CH | $FeCl_3$ | |
| 6.15 | Cl | H | Cl | H | $CH_3$ | H | $CH_3$ | N | - | |
| 6.16 | Cl | Cl | Cl | H | $CH_3$ | H | $C_2H_5$ | N | - | |
| 6.17 | H | H | H | H | $C_2H_5$ | H | H | N | - | Smp. 103-109° |
| 6.18 | $CH_3$ | H | H | H | $CH_3$ | H | H | N | - | |
| 6.19 | $CH_3$ | H | H | 6-$CH_3$ | $CH_3$ | H | H | N | $HNO_3$ | |
| 6.20 | H | H | H | 5-$CH_3$ | $CH_3$ | $C_2H_5$ | H | N | - | |
| 6.21 | H | Cl | H | 6-Cl | $CH_3$ | $C_2H_5$ | H | CH | - | |
| 6.22 | H | Cl | H | 6-Cl | $C_2H_5$ | $C_2H_5$ | $CH_3$ | N | - | |
| 6.23 | H | H | $CH_3$ | H | $C_2H_5$ | $C_2H_5$ | $C_2H_5$ | N | $Mn(NO_3)_2$ | |
| 6.24 | H | H | H | H | $C_3H_7$-n | H | $C_3H_7$-n | N | - | |
| 6.25 | H | $CH_3$ | H | H | $C_3H_7$-n | H | $C_3H_7$-n | CH | - | |
| 6.26 | Cl | H | H | H | $C_3H_7$-n | $C_3H_7$-n | H | N | - | |
| 6.27 | Cl | H | Cl | H | $C_3H_7$-n | $C_2H_5$ | $CH_3$ | N | - | |
| 6.28 | Cl | Cl | Cl | 6-Cl | $C_3H_7$-n | $C_2H_5$ | $CH_3$ | CH | - | |

Fortsetzung von Tabelle 6:

| Verb. Nr. | $R_{13}$ | $R_{14}$ | $R_{15}$ | $R_{16}$ | $R_{17}$ | $R_{18}$ | $R_{19}$ | Y | Salz |
|---|---|---|---|---|---|---|---|---|---|
| 6.29 | H | H | Cl | H | $C_3H_7$-n | $C_2H_5$ | $CH_3$ | N | $ZnCl_2$ |
| 6.30 | H | H | Br | H | $C_3H_7$-n | $CH_3$ | $C_2H_5$ | N | - |
| 6.31 | $CH_3$ | H | $CH_3$ | H | $C_2H_5$ | $C_3H_7$-n | $CH_3$ | N | - |
| 6.32 | H | H | $CH_3$ | H | $C_4H_9$-n | $CH_3$ | H | N | - |
| 6.33 | Cl | H | H | 6-Cl | $C_4H_9$-n | H | $CH_3$ | N | $FeCl_3$ |
| 6.34 | Cl | H | H | 6-Cl | $C_3H_7$-n | H | $CH_3$ | CH | - |
| 6.35 | H | H | Cl | H | $CH_3$ | H | $C_4H_9$-n | N | - |
| 6.36 | F | H | H | H | H | H | $C_4H_9$-n | CH | - |
| 6.37 | H | H | H | H | $C_4H_9$-n | H | H | CH | - |
| 6.38 | H | H | H | H | $C_4H_9$-n | H | H | N | $HNO_3$ |
| 6.39 | Cl | H | H | H | H | $C_4H_9$-n | H | N | - |
| 6.40 | Cl | H | Cl | $CH_3$ | H | $C_4H_9$-n | H | CH | HCl |
| 6.41 | H | Cl | H | H | $CH_3$ | $C_4H_9$-n | H | N | - |
| 6.42 | H | Cl | H | H | H | $C_4H_9$-sek | H | N | $HNO_3$ |
| 6.43 | H | Cl | Cl | H | H | $C_4H_9$-sek | H | N | - |
| 6.44 | H | Br | H | H | $CH_3$ | $C_4H_9$-sek | H | N | - |
| 6.45 | H | Br | H | 6-Br | $CH_3$ | $C_3H_7$-sek | H | CH | - |
| 6.46 | $CH_3$ | H | H | 5-Br | $CH_3$ | $C_3H_7$-i | H | N | - |

Fortsetzung von Tabelle 6:

| Verb. Nr. | $R_{13}$ | $R_{14}$ | $R_{15}$ | $R_{16}$ | $R_{17}$ | $R_{18}$ | $R_{19}$ | Y | Salz |
|---|---|---|---|---|---|---|---|---|---|
| 6.47 | H | H | $CH_3$ | H | $CH_3$ | $C_3H_7$-i | H | N | $Mn(NO_3)_2$ |
| 6.48 | H | Cl | H | H | $CH_3$ | $C_3H_7$-i | $CH_3$ | N | - |
| 6.49 | Cl | H | Cl | H | $C_2H_5$ | $C_3H_7$-i | H | N | - |
| 6.50 | $(CH=CH)_2$ | | H | H | H | H | H | N | - |
| 6.51 | $(CH=CH)_2$ | | H | H | H | $CH_3$ | H | CH | $Mn(NO_3)_2$ |
| 6.52 | $(CH=CH)_2$ | | H | 6-Cl | H | $CH_3$ | H | N | - |
| 6.53 | $(CH=CH)_2$ | | H | 6-$CH_3$ | $CH_3$ | $CH_3$ | H | N | - |
| 6.54 | $(CH=CH)_2$ | | Cl | 6-Cl | H | $CH_3$ | H | N | - |
| 6.55 | $(CH=CH)_2$ | | H | 6-Cl | $CH_3$ | $CH_3$ | $CH_3$ | N | - |
| 6.56 | $(CH=CH)_2$ | | H | 6-Cl | $CH_3$ | $CH_3$ | $CH_3$ | CH | - |
| 6.57 | $(CH=CH)_2$ | | Cl | 6-Cl | $C_3H_7$-i | H | H | N | HCl |
| 6.58 | $(CH=CH)_2$ | | H | 6-Cl | H | $C_2H_5$ | H | N | - |
| 6.59 | $(CH=CH)_2$ | | H | H | $CH_3$ | $CH_3$ | $CH_3$ | N | - |
| 6.60 | $(CH=CH)_2$ | | H | H | $CH_3$ | $CH_3$ | $CH_3$ | CH | - |
| 6.61 | H | $(CH=CH)_2$ | | H | H | H | H | N | - |
| 6.62 | H | $(CH=CH)_2$ | | H | H | H | H | N | $CuCl_2$ |
| 6.63 | H | $(CH=CH)_2$ | | H | H | $CH_3$ | H | N | - |
| 6.64 | H | $(CH=CH)_2$ | | H | $CH_3$ | H | H | N | - |

Fortsetzung von Tabelle 6:

| Verb. Nr. | $R_{13}$ | $R_{14}$ | $R_{15}$ | $R_{16}$ | $R_{17}$ | $R_{18}$ | $R_{19}$ | Y | Salz | Physik. Konstante |
|---|---|---|---|---|---|---|---|---|---|---|
| 6.65 | H | $(CH=CH)_2$ | | H | $CH_3$ | $CH_3$ | $CH_3$ | N | - | |
| 6.66 | H | $(CH=CH)_2$ | | H | H | H | H | CH | - | |
| 6.67 | Cl | $(CH=CH)_2$ | | H | H | H | H | N | - | |
| 6.68 | Cl | $(CH=CH)_2$ | | H | $CH_3$ | $CH_3$ | $CH_3$ | N | - | |
| 6.69 | Cl | $(CH=CH)_2$ | | H | $C_2H_5$ | H | H | N | - | |
| 6.70 | Cl | $(CH=CH)_2$ | | 6-Cl | $CH_3$ | $CH_3$ | $CH_3$ | N | - | |
| 6.71 | H | $(CH=CH)_2$ | | 6-Cl | $CH_3$ | $CH_3$ | $CH_3$ | N | $HNO_3$ | |
| 6.72 | Br | $(CH=CH)_2$ | | H | $CH_3$ | $C_2H_5$ | $CH_3$ | N | - | |
| 6.73 | $CH_3$ | $(CH=CH)_2$ | | H | $CH_3$ | $CH_3$ | $CH_3$ | N | - | |
| 6.74 | Cl | Cl | H | 5-Cl | H | H | H | N | - | |
| 6.75 | Cl | H | Cl | 5-Cl | H | H | H | N | - | |
| 6.76 | $CH_3$ | $CH_3$ | H | H | H | H | H | CH | - | |
| 6.77 | F | H | H | H | $CH_3$ | H | H | N | - | |
| 6.78 | H | F | H | H | H | H | H | N | - | |
| 6.79 | H | H | F | H | H | H | H | CH | - | Oel |
| 6.80 | H | H | F | H | $CH_3$ | H | H | CH | - | zähe Masse |
| 6.81 | H | H | F | H | $CH_3$ | H | H | N | - | |
| 6.82 | Cl | H | H | H | $CH_3$ | H | H | N | - | |

Fortsetzung von Tabelle 6:

| Verb. Nr. | $R_{13}$ | $R_{14}$ | $R_{15}$ | $R_{16}$ | $R_{17}$ | $R_{18}$ | $R_{19}$ | Y | Salz |
|---|---|---|---|---|---|---|---|---|---|
| 6.83 | Br | H | H | H | $CH_3$ | H | H | N | – |
| 6.84 | Br | H | H | H | H | H | H | N | – |
| 6.85 | H | Br | H | H | H | H | H | CH | – |
| 6.86 | H | Br | H | H | H | H | H | N | – |
| 6.87 | H | H | Br | H | H | H | H | CH | – |
| 6.88 | H | H | Br | H | $CH_3$ | H | H | CH | – |
| 6.89 | H | H | Br | H | H | H | H | N | – |
| 6.90 | H | H | J | H | H | H | H | CH | – |
| 6.91 | H | H | J | H | H | H | H | N | – |

Tabelle 7: Verbindungen der Formel

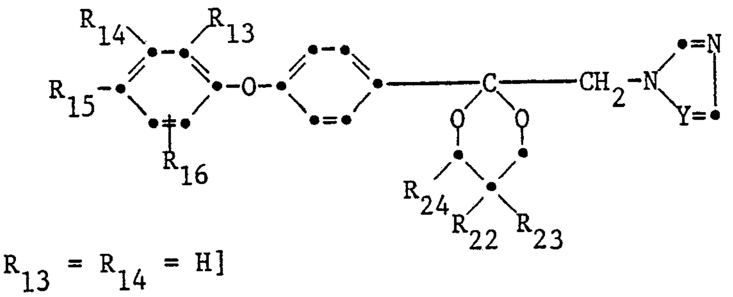

(XXVII)

$[R_{13} = R_{14} = H]$
wobei isomere Formen mit umfasst sein sollen:

| Verb. Nr. | $R_{22}$ | $R_{23}$ | $R_{24}$ | $R_{15}$ | $R_{16}$ | Y | Salz | Physik. Könst. |
|---|---|---|---|---|---|---|---|---|
| 7.1 | H | $CH_3$ | H | H | H | N | - | |
| 7.2 | H | $CH_3$ | H | H | H | CH | - | |
| 7.3 | $CH_3$ | $CH_3$ | H | H | H | N | - | Smp. 122-124° |
| 7.4 | $CH_3$ | $CH_3$ | H | H | H | N | $HNO_3$ | |
| 7.5 | $CH_3$ | $CH_3$ | $CH_3$ | H | H | N | $Mn(NO_3)_2$ | |
| 7.6 | $CH_3$ | $CH_3$ | $CH_3$ | H | H | CH | - | |
| 7.7 | $CH_3$ | $CH_3$ | H | Cl | 6-Cl | | - | Oel; $n_D^{23} = 1.5782$ |
| 7.8 | $CH_3$ | $C_2H_5$ | H | H | H | N | - | |
| 7.9 | $CH_3$ | $C_2H_5$ | H | H | H | N | $CuCl_2$ | |
| 7.10 | $CH_3$ | $C_2H_5$ | $CH_3$ | $CH_3$ | 6-$CH_3$ | CH | $(COOH)_2$ | |
| 7.11 | $C_2H_5$ | $C_2H_5$ | H | H | H | N | $HNO_3$ | |
| 7.12 | $C_2H_5$ | $C_2H_5$ | H | H | H | N | - | |
| 7.13 | $C_2H_5$ | $C_2H_5$ | H | H | H | CH | HCl | |
| 7.14 | $C_2H_5$ | $C_2H_5$ | H | H | H | CH | $FeCl_3$ | |
| 7.15 | $C_2H_5$ | $C_2H_5$ | H | H | H | CH | - | |
| 7.16 | H | $C_2H_5$ | H | $CH_3$ | H | N | - | |
| 7.17 | H | $C_2H_5$ | H | H | H | N | $HNO_3$ | |
| 7.18 | H | $C_2H_5$ | H | H | H | CH | - | |
| 7.19 | H | $C_3H_7$-n | H | H | H | N | $CuCl_2$ | |
| 7.20 | H | $C_3H_7$-n | H | H | H | CH | - | |
| 7.21 | $CH_3$ | $CH_3$ | $C_3H_7$-n | H | H | N | - | Smp. 119-121° |
| 7.22 | H | H | H | H | H | CH | - | |
| 7.23 | $CH_3$ | $CH_3$ | H | H | H | CH | - | |

Fortsetzung von Tabelle 7:

| Verb. Nr. | $R_{22}$ | $R_{23}$ | $R_{24}$ | $R_{15}$ | $R_{16}$ | Y | Salz | Physik. Konst. |
|---|---|---|---|---|---|---|---|---|
| 7.24 | H | H | $CH_3$ | H | H | CH | - | |
| 7.25 | H | H | $CH_3$ | H | H | N | 1/2 $CuSO_4$ | |
| 7.26 | H | H | H | H | 6-F | N | - | |
| 7.27 | H | H | H | F | H | N | - | |
| 7.28 | H | H | H | H | 6-Cl | N | - | |
| 7.29 | H | H | $CH_3$ | H | 6-Cl | CH | - | |
| 7.30 | H | H | H | H | 5-Cl | CH | - | |
| 7.31 | H | H | H | H | 5-Cl | N | - | |
| 7.32 | H | H | $CH_3$ | Cl | H | CH | - | |
| 7.33 | H | H | H | Cl | H | CH | - | zähe Masse |
| 7.34 | H | H | H | Cl | H | N | - | Oel |
| 7.35 | H | H | $CH_3$ | Cl | H | N | - | |
| 7.36 | H | H | H | $CH_3$ | H | N | - | |
| 7.37 | H | H | H | $C_3H_7$-i | H | N | - | |
| 7.38 | H | H | H | $NO_2$ | H | N | - | |
| 7.39 | H | H | H | H | 6-$CF_3$ | N | - | |
| 7.40 | H | H | $CH_3$ | H | 5-$CF_3$ | N | - | |
| 7.41 | H | H | H | $CF_3$ | H | N | - | |
| 7.42 | H | H | H | Cl | 5-Cl | N | - | |
| 7.43 | H | H | H | Cl | 5-$CH_3$ | CH | - | |
| 7.44 | $CH_3$ | $C_2H_5$ | H | $NO_2$ | 5-$CH_3$ | N | - | |
| 7.45 | $CH_3$ | $CH_3$ | H | $CF_3$ | 6-Cl | CH | - | |
| 7.46 | $CH_3$ | $CH_3$ | $C_3H_7$-n | $CF_3$ | 6-$NO_2$ | CH | - | |

Tabelle 8: Verbindungen der Formel

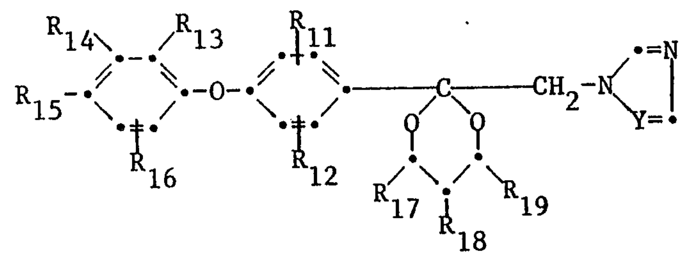

(XXVIII)

wobei die isomeren Formen mit umfasst sein sollen:

| Verb. Nr. | $R_{11}$ | $R_{12}$ | $R_{13}$ | $R_{14}$ | $R_{15}$ | $R_{16}$ | $R_{17}$ | $R_{18}$ | $R_{19}$ | Y | Salz |
|---|---|---|---|---|---|---|---|---|---|---|---|
| 8.1 | 2-Cl | H | $CH_3$ | H | H | H | H | H | H | N | - |
| 8.2 | 2-Cl | H | H | H | H | H | H | $C_2H_5$ | H | N | - |
| 8.3 | 2-Cl | H | H | H | H | H | H | $CH_3$ | H | CH | - |
| 8.4 | 2-Cl | 5-Cl | H | H | H | H | $CH_3$ | $CH_3$ | H | N | - |
| 8.5 | 2-$CH_3$ | H | H | H | H | H | $CH_3$ | H | H | N | $HNO_3$ |
| 8.6 | 2-$CH_3$ | H | H | F | H | H | H | H | H | N | - |
| 8.7 | 2-Cl | H | Cl | H | H | H | $CH_3$ | $CH_3$ | $CH_3$ | N | $HNO_3$ |
| 8.8 | 2-Cl | 6-Cl | Cl | H | Cl | H | $CH_3$ | H | H | N | - |
| 8.9 | 2-Br | H | H | H | H | H | $CH_3$ | $CH_3$ | $CH_3$ | N | $Mn(NO_3)_2$ |
| 8.10 | 2-$CH_3$ | H | $CH_3$ | H | H | H | $CH_3$ | H | H | N | - |
| 8.11 | 2-$OCH_3$ | 6-$OCH_3$ | H | H | Cl | H | H | H | H | CH | - |

Fortsetzung von Tabelle 8:

| Verb. Nr. | $R_{11}$ | $R_{12}$ | $R_{13}$ | $R_{14}$ | $R_{15}$ | $R_{16}$ | $R_{17}$ | $R_{18}$ | $R_{19}$ | Y | Salz |
|---|---|---|---|---|---|---|---|---|---|---|---|
| 8.12 | 2-Cl | H | H | Cl | Cl | 6-Cl | $CH_3$ | $CH_3$ | $CH_3$ | CH | $HNO_3$ |
| 8.13 | 3-Cl | H | H | H | H | H | $CH_3$ | $CH_3$ | $CH_3$ | CH | $CuCl_2$ |
| 8.14 | 3-Br | H | H | H | H | H | $CH_3$ | $CH_3$ | $CH_3$ | CH | $FeCl_3$ |
| 8.15 | 3-Br | 5-Br | H | H | H | H | $CH_3$ | H | $CH_3$ | N | - |
| 8.16 | 2-Cl | H | H | H | H | H | $CH_3$ | H | $C_2H_5$ | N | - |
| 8.17 | 2-Cl | 6-Cl | H | Cl | H | H | $C_2H_5$ | H | $C_2H_5$ | N | - |
| 8.18 | 2-Cl | 6-Cl | H | H | H | H | $CH_3$ | H | H | N | - |
| 8.19 | 2-Cl | 5-Cl | H | Cl | H | H | $CH_3$ | H | H | N | $HNO_3$ |
| 8.20 | 2-$OCH_3$ | 6-$OCH_3$ | H | H | Cl | H | H | H | H | N | - |
| 8.21 | 2-$CH_3$ | 6-$CH_3$ | $CH_3$ | H | $CH_3$ | H | $CH_3$ | $C_2H_5$ | H | CH | - |
| 8.22 | 2-Br | 6-$CH_3$ | | H | H | H | $C_2H_5$ | $C_2H_5$ | $CH_3$ | N | - |
| 8.23 | 2-Cl | H | Cl | H | Cl | H | $C_2H_5$ | $C_2H_5$ | $C_2H_5$ | N | $Mn(NO_3)_2$ |
| 8.24 | 3-Cl | H | $(CH=CH)_2$ | | H | H | H | H | H | N | - |
| 8.25 | H | H | $(CH=CH)_2$ | | H | H | H | H | H | CH | $Mn(NO_3)_2$ |
| 8.26 | 2-Cl | H | $(CH=CH)_2$ | | H | H | H | H | H | N | - |
| 8.27 | 2-Cl | H | $(CH=CH)_2$ | | H | 2-Cl | H | $CH_3$ | H | N | - |
| 8.28 | 2-Cl | 6-Cl | $(CH=CH)_2$ | | Cl | H | $CH_3$ | $CH_3$ | $CH_3$ | N | - |
| 8.29 | 2-Cl | H | $(CH=CH)_2$ | | H | H | $CH_3$ | $CH_3$ | $CH_3$ | N | - |

Fortsetzung von Tabelle 8:

| Verb. Nr. | $R_{11}$ | $R_{12}$ | $R_{13}$ | $R_{14}$ | $R_{15}$ | $R_{16}$ | $R_{17}$ | $R_{18}$ | $R_{19}$ | Y | Salz |
|---|---|---|---|---|---|---|---|---|---|---|---|
| 8.30 | 2-Cl | 6-Cl | $(CH=CH)_2$ | | H | H | $CH_3$ | $CH_3$ | $CH_3$ | N | $CuCl_2$ |
| 8.31 | 2-$CH_3$ | H | $(CH=CH)_2$ | | H | H | $CH_3$ | $CH_3$ | $CH_3$ | N | - |
| 8.32 | 2-$CH_3$ | 6-$CH_3$ | $(CH=CH)_2$ | | H | H | $CH_3$ | $CH_3$ | $CH_3$ | CH | HCl |
| 8.33 | 2-Br | H | $(CH=CH)_2$ | | H | 2-Br | H | $CH_3$ | H | N | - |
| 8.34 | 2-Cl | 5-Cl | $(CH=CH)_2$ | | H | H | $CH_3$ | $CH_3$ | $CH_3$ | N | - |
| 8.35 | 2-Cl | 5-Cl | $(CH=CH)_2$ | | H | H | $CH_3$ | $CH_3$ | H | N | - |
| 8.36 | H | H | H | $(CH=CH)_2$ | | H | H | H | H | CH | $MnCl_2$ |
| 8.37 | 3-Cl | H | H | $(CH=CH)_2$ | | H | H | H | H | N | - |
| 8.38 | 2-Cl | H | H | $(CH=CH)_2$ | | H | $CH_3$ | H | H | N | - |
| 8.39 | 2-Cl | H | H | $(CH=CH)_2$ | | H | $CH_3$ | $CH_3$ | H | N | - |
| 8.40 | 2-Cl | H | Cl | $(CH=CH)_2$ | | 3-Cl | H | $CH_3$ | H | N | - |
| 8.41 | 2-Cl | 6-Cl | H | $(CH=CH)_2$ | | 4-Cl | $CH_3$ | $CH_3$ | H | CH | - |
| 8.42 | 2-Cl | H | Cl | $(CH=CH)_2$ | | H | $CH_3$ | $CH_3$ | $CH_3$ | N | - |
| 8.43 | 2-Cl | 5-Cl | H | $(CH=CH)_2$ | | H | $CH_3$ | $CH_3$ | $CH_3$ | N | - |
| 8.44 | 2-Cl | 3-Cl | H | $(CH=CH)_2$ | | 4-Br | $CH_3$ | H | $CH_3$ | N | - |
| 8.45 | 2-$CH_3$ | 3-Cl | Cl | $(CH=CH)_2$ | | H | $CH_3$ | $CH_3$ | $CH_3$ | H | - |
| 8.46 | 2-$CH_3$ | 5-$CH_3$ | H | $(CH=CH)_2$ | | H | $CH_3$ | $CH_3$ | H | N | HCl |
| 8.47 | 2-$CH_3$ | H | $CH_3$ | $(CH=CH)_2$ | | H | $CH_3$ | H | $CH_3$ | N | - |

Fortsetzung von Tabelle 8:

| Verb. Nr. | $R_{11}$ | $R_{12}$ | $R_{13}$ | $R_{14}$ | $R_{15}$ | $R_{16}$ | $R_{17}$ | $R_{18}$ | $R_{19}$ | Y | Salz |
|---|---|---|---|---|---|---|---|---|---|---|---|
| 8.48 | 2-Cl | H | Cl | $(CH=CH)_2$ | | H | H | $C_2H_5$ | H | N | – |
| 8.49 | 2-Cl | H | Cl | $(CH=CH)_2$ | | H | $C_2H_5$ | $CH_3$ | H | N | – |
| 8.50 | 2-$CH_3$ | H | H | H | H | H | H | H | H | CH | – |
| 8.51 | 2-$CH_3$ | H | H | H | H | H | $CH_3$ | H | H | CH | – |
| 8.52 | 2-$CH_3$ | H | H | H | H | H | $CH_3$ | H | H | N | – |
| 8.53 | 2-$CH_3$ | H | H | H | H | H | H | H | H | N | – |
| 8.54 | 2-Cl | H | H | H | H | H | H | H | H | CH | – |
| 8.55 | 2-Cl | H | H | H | H | H | H | H | H | N | – |
| 8.56 | 2-Cl | H | H | H | H | H | $CH_3$ | H | H | N | – |
| 8.57 | 3-$NO_2$ | H | H | H | H | H | H | H | H | CH | – |
| 8.58 | 2-Br | H | H | H | H | H | H | H | H | CH | – |
| 8.59 | 2-Br | H | H | H | H | H | H | H | H | N | – |
| 8.60 | 3-$CH_3$ | H | H | H | H | H | H | H | H | CH | – |
| 8.61 | 3-$CH_3$ | H | H | H | H | H | H | H | H | N | – |
| 8.62 | 2-$NO_2$ | H | H | H | H | H | H | H | H | N | – |
| 8.63 | 2-Cl | H | H | H | Cl | H | H | H | H | N | – |
| 8.64 | H | H | Cl | H | H | 5-Cl | H | H | H | N | – |
| 8.65 | H | H | H | Cl | H | 5-Cl | H | H | H | N | – |

Fortsetzung von Tabelle 8:

| Verb. Nr. | $R_{11}$ | $R_{12}$ | $R_{13}$ | $R_{14}$ | $R_{15}$ | $R_{16}$ | $R_{17}$ | $R_{18}$ | $R_{19}$ | Y | Salz | Physik. Konstante |
|---|---|---|---|---|---|---|---|---|---|---|---|---|
| 8.66 | 2-$CH_3$ | H | H | F | H | H | H | H | H | CH | – | braunes Harz |
| 8.67 | 2-$CH_3$ | H | F | H | H | H | H | H | H | N | – | |
| 8.68 | 2-$CH_3$ | H | H | H | Cl | H | H | H | H | N | – | |
| 8.69 | 2-$CH_3$ | H | H | H | Cl | H | H | H | H | CH | – | braunes Harz |
| 8.70 | 2-$CH_3$ | H | H | H | Cl | H | $CH_3$ | H | H | N | – | |
| 8.71 | 2-$CH_3$ | H | H | H | Cl | H | $CH_3$ | H | H | CH | – | |
| 8.72 | 2-$CH_3$ | H | H | H | F | H | H | H | H | N | – | Harz |
| 8.73 | 2-$CH_3$ | H | H | H | F | H | H | H | H | CH | – | |
| 8.74 | 2-$CH_3$ | H | H | H | F | H | $CH_3$ | H | H | N | – | |
| 8.75 | 2-$CH_3$ | H | H | H | F | H | $CH_3$ | H | H | CH | – | |

Tabelle 9: Verbindungen der Formel

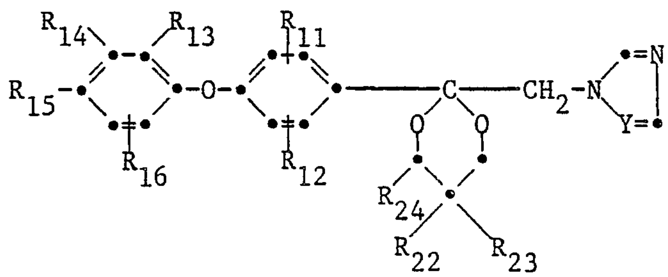

, (XXIX)

wobei isomere Formen mit umfasst sein sollen:

| Verb. Nr. | $R_{11}$ | $R_{12}$ | $R_{13}$ | $R_{14}$ | $R_{15}$ | $R_{16}$ | $R_{22}$ | $R_{23}$ | $R_{24}$ | Y | Salz |
|---|---|---|---|---|---|---|---|---|---|---|---|
| 9.1 | 2-Cl | H | H | H | H | H | H | $CH_3$ | H | N | - |
| 9.2 | 2-Cl | H | H | Cl | H | 6-Cl | H | $CH_3$ | H | CH | - |
| 9.3 | 2-Br | H | Br | H | H | H | $CH_3$ | $CH_3$ | H | N | - |
| 9.4 | 2-$CH_3$ | 6-$CH_3$ | H | H | H | H | $CH_3$ | $CH_3$ | H | N | $HNO_3$ |
| 9.5 | 2-Cl | 6-Cl | Cl | H | H | 6-Cl | $CH_3$ | $CH_3$ | $CH_3$ | N | $Mn(NO_3)_2$ |
| 9.6 | 2-Cl | 6-Cl | H | Cl | H | H | $CH_3$ | $CH_3$ | $CH_3$ | CH | - |
| 9.7 | 2-Cl | 5-Cl | H | H | Cl | H | $CH_3$ | $CH_3$ | H | | - |
| 9.8 | 2-Cl | H | H | Cl | Cl | H | $CH_2$ | $C_2H_5$ | H | N | - |
| 9.9 | 2-Cl | H | Cl | H | H | H | $CH_3$ | $C_2H_5$ | H | N | $CuCl_2$ |
| 9.10 | 3-Cl | 5-Cl | Cl | H | H | 6-Cl | $CH_3$ | $C_2H_5$ | $CH_3$ | CH | $(COOH)_2$ |
| 9.11 | 2-Cl | 6-Cl | H | H | Cl | H | $C_2H_5$ | $C_2H_5$ | H | N | $HNO_3$ |
| 9.12 | 3-$CH_3$ | 5-$CH_3$ | H | H | H | H | $C_2H_5$ | $C_2H_5$ | H | N | - |
| 9.13 | 2-$CH_3$ | H | $CH_3$ | $CH_3$ | H | H | $C_2H_5$ | $C_2H_5$ | H | CH | HCl |
| 9.14 | 2-Cl | H | H | $CH_3$ | H | H | $C_2H_5$ | $C_2H_5$ | H | CH | $FeCl_3$ |
| 9.15 | 2-Cl | 5-Cl | Cl | H | H | H | $C_2H_5$ | $C_2H_5$ | H | CH | - |
| 9.16 | 2-Cl | H | Cl | Cl | Cl | 5-Cl | H | $C_2H_5$ | H | N | - |
| 9.17 | 2-Cl | H | H | Cl | Cl | H | H | $C_2H_5$ | H | N | $HNO_3$ |
| 9.18 | 2-$CH_3$ | 5-$CH_3$ | $CH_3$ | H | H | 6-$CH_3$ | H | $C_2H_5$ | H | CH | - |
| 9.19 | 2-$CH_3$ | 6-$CH_3$ | H | H | $CH_3$ | H | H | $C_3H_7$-n | H | N | $CuCl_2$ |
| 9.20 | 2-Cl | 6-Cl | H | H | $CH_3$ | H | H | $C_3H_7$-n | H | CH | - |
| 9.21 | 3-$NO_2$ | H | H | H | Cl | H | H | H | $CH_3$ | N | - |
| 9.22 | H | H | Cl | H | Cl | 6-$CH_3$ | $CH_3$ | $CH_3$ | H | N | - |
| 9.23 | H | H | $CH_3$ | H | Br | 6-Cl | $CH_3$ | $CH_3$ | $C_3H_7$-n | CH | - |
| 9.24 | H | H | $CH_3$ | H | Br | 6-Cl | $CH_3$ | $C_2H_5$ | H | N | |

Tabelle 10: Verbindungen der Formel

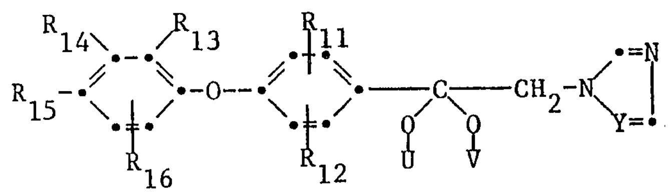

(XXX)

wobei die isomeren Formen mit umfasst sein sollen:

| Verb. Nr. | $R_{11}$ | $R_{12}$ | $R_{13}$ | $R_{14}$ | $R_{15}$ | $R_{16}$ | Y | U | V | Salz |
|---|---|---|---|---|---|---|---|---|---|---|
| 10.1 | H | H | Cl | H | Cl | H | N | $CH_3$ | $CH_3$ | – |
| 10.2 | H | H | Cl | H | Cl | H | N | $CH_3$ | $CH_3$ | $Mn(NO_3)_2$ |
| 10.3 | H | H | $CH_3$ | H | $CH_3$ | H | CH | $CH_3$ | $C_2H_5$ | – |
| 10.4 | H | H | $CH_3$ | H | $CH_3$ | 5-$CH_3$ | N | $C_2H_5$ | $C_2H_5$ | – |
| 10.5 | H | H | H | Cl | H | 5-Cl | N | $C_4H_9$-n | $C_4H_9$-n | – |
| 10.6 | H | H | H | Cl | H | 6-Cl | N | $C_3H_7$-i | $C_3H_7$-i | $CuCl_2$ |
| 10.7 | H | H | H | H | H | H | N | $CH_3$ | $CH_3$ | – |
| 10.8 | H | H | H | H | F | H | CH | $CH_3$ | $CH_3$ | – |
| 10.9 | H | H | H | H | H | H | N | $C_3H_7$-n | $C_3H_7$-n | $(COOH)_2$ |
| 10.10 | 2-Cl | H | H | H | Cl | H | N | $CH_3$ | $CH_3$ | – |
| 10.11 | 2-Cl | 6-Cl | H | H | H | H | N | $CH_3$ | $CH_3$ | – |
| 10.12 | 3-Cl | H | H | H | H | H | N | $C_2H_5$ | $C_2H_5$ | – |
| 10.13 | 2-Cl | 6-Cl | H | H | Cl | H | N | $C_8H_{17}$-n | $C_8H_{17}$-n | – |

Fortsetzung Tabelle 10:

| Verb. Nr. | $R_{11}$ | $R_{12}$ | $R_{13}$ | $R_{14}$ | $R_{15}$ | $R_{16}$ | Y | U | V | Salz |
|---|---|---|---|---|---|---|---|---|---|---|
| 10.14 | 2-$CH_3$ | H | H | $CH_3$ | H | H | CH | $C_2H_5$ | $C_2H_5$ | HCl |
| 10.15 | 2-$CH_3$ | 6-$CH_3$ | $CH_3$ | H | H | H | N | $C_3H_7$-i | $C_3H_7$-i | - |
| 10.16 | 2-$CH_3$ | H | $CH_3$ | $CH_3$ | H | H | N | $C_5H_{11}$-n | $C_5H_{11}$-n | - |
| 10.17 | 2-Cl | 5-Cl | Cl | H | H | 6-Cl | N | $CH_3$ | $CH_3$ | - |
| 10.18 | 2-Cl | 5-Cl | H | H | Cl | H | N | $C_4H_9$-s | $C_4H_9$-s | - |
| 10.19 | 2-Cl | 6-Cl | Cl | Cl | Cl | 6-Cl | CH | $CH_3$ | $CH_3$ | - |
| 10.20 | 2-Cl | H | $(CH{=}CH)_2$ | | H | H | N | $CH_3$ | $CH_3$ | - |
| 10.21 | 2-$CH_3$ | H | $(CH{=}CH)_2$ | | H | H | N | $CH_3$ | $C_3H_7$-i | - |
| 10.22 | H | H | H | H | H | H | CH | $CH_3$ | $CH_3$ | - |
| 10.23 | H | H | H | H | H | H | CH | $C_2H_5$ | $C_2H_5$ | - |
| 10.24 | H | H | H | H | H | H | CH | $C_4H_9$-n | $C_4H_9$-n | - |
| 10.25 | H | H | H | H | H | H | CH | $(CH_2)_2OCH_3$ | $(CH_2)_2OCH_3$ | - |
| 10.26 | H | H | H | H | H | H | CH | $(CH_2)_2OC_2H_5$ | $(CH_2)_2OC_2H_5$ | - |
| 10.27 | H | H | H | H | H | H | CH | $C_8H_{17}$-n | $C_8H_{17}$-n | - |
| 10.28 | H | H | H | H | H | H | N | $(CH_2)_2OCH_3$ | $(CH_2)_2OCH_3$ | - |
| 10.29 | 2-$CH_3$ | H | H | H | H | H | CH | $CH_3$ | $CH_3$ | |
| 10.30 | 2-$CH_3$ | H | H | H | H | H | N | $(CH_2)_2OCH_3$ | $(CH_2)_2OCH_3$ | - |
| 10.31 | 2-Cl | H | H | H | H | H | CH | $(CH_2)_2OCH_3$ | $(CH_2)_2OCH_3$ | |

Fortsetzung Tabelle 10:

| Verb. Nr. | $R_{11}$ | $R_{12}$ | $R_{13}$ | $R_{14}$ | $R_{15}$ | $R_{16}$ | Y | U | V | Salz |
|---|---|---|---|---|---|---|---|---|---|---|
| 10.32 | 2-$CH_3$ | H | H | H | H | H | N | $CH_3$ | $CH_3$ | – |
| 10.33 | 2-Cl | H | H | H | H | H | CH | $CH_3$ | $CH_3$ | – |
| 10.34 | 2-Cl | H | H | H | H | H | N | $(CH_2)_2OCH_3$ | $(CH_2)_2OCH_3$ | – |
| 10.35 | 2-Br | H | H | H | H | H | CH | $CH_3$ | $CH_3$ | – |
| 10.36 | 2-Br | H | H | H | H | H | N | $C_2H_5$ | $C_2H_5$ | – |
| 10.37 | 3-$NO_2$ | H | H | H | H | H | CH | $C_2H_5$ | $C_2H_5$ | – |
| 10.38 | 2-Br | H | H | H | H | H | N | $(CH_2)_2OCH_3$ | $(CH_2)_2OCH_3$ | – |
| 10.39 | 3-$CH_3$ | H | H | H | H | H | CH | $CH_3$ | $CH_3$ | – |
| 10.40 | H | H | Cl | H | H | H | CH | $CH_3$ | $CH_3$ | – |
| 10.41 | H | H | Cl | H | H | H | CH | $(CH_2)_2OCH_3$ | $(CH_2)_2OCH_3$ | – |
| 10.42 | H | H | Cl | H | H | H | CH | $C_4H_9$-n | $C_4H_9$-n | – |
| 10.43 | H | H | Cl | H | H | H | N | $(CH_2)_2OCH_3$ | $(CH_2)_2OCH_3$ | – |
| 10.44 | H | H | H | Cl | H | H | CH | $CH_3$ | $CH_3$ | – |
| 10.45 | H | H | H | Cl | H | H | CH | $C_4H_9$-n | $C_4H_9$-n | – |
| 10.46 | H | H | H | Cl | H | H | CH | $C_8H_{17}$-n | $C_8H_{17}$-n | – |
| 10.47 | H | H | Cl | H | H | H | N | $C_4H_9$-n | $C_4H_9$-n | – |
| 10.48 | H | H | H | Cl | H | H | CH | $C_2H_5$ | $C_2H_5$ | – |
| 10.49 | H | H | H | Cl | H | H | CH | $(CH_2)_2OCH_3$ | $(CH_2)_2OCH_3$ | – |

Fortsetzung Tabelle 10:

| Verb. Nr. | $R_{11}$ | $R_{12}$ | $R_{13}$ | $R_{14}$ | $R_{15}$ | $R_{16}$ | Y | U | V | Salz |
|---|---|---|---|---|---|---|---|---|---|---|
| 10.50 | H | H | H | Cl | H | H | CH | $(CH_2)_2OC_2H_5$ | $(CH_2)_2OC_2H_5$ | - |
| 10.51 | H | H | H | H | Cl | H | CH | $CH_3$ | $CH_3$ | - |
| 10.52 | H | H | H | H | Cl | H | CH | $C_4H_9$-n | $C_4H_9$-n | - |
| 10.53 | H | H | H | H | Cl | H | CH | $(CH_2)_2OCH_3$ | $(CH_2)_2OCH_3$ | - |
| 10.54 | H | H | H | H | Cl | H | CH | $C_2H_5$ | $C_2H_5$ | - |
| 10.55 | H | H | H | H | Cl | H | CH | $C_2H_4Cl$ | $C_2H_4Cl$ | - |
| 10.56 | H | H | H | H | Cl | H | CH | $(CH_2)_2OC_2H_5$ | $(CH_2)_2OC_2H_5$ | - |
| 10.57 | H | H | H | H | Cl | H | N | $C_2H_5$ | $C_2H_5$ | - |
| 10.58 | H | H | H | H | Cl | H | N | $CH_3$ | $CH_3$ | - |
| 10.59 | H | H | H | H | Cl | H | N | $(CH_2)_2OCH_3$ | $(CH_2)_2OCH_3$ | - |
| 10.60 | H | H | H | H | Br | H | CH | $(CH_2)_2OCH_3$ | $(CH_2)_2OCH_3$ | - |
| 10.61 | H | H | H | H | Br | H | CH | $CH_3$ | $CH_3$ | |
| 10.62 | H | H | $CH_3$ | H | H | H | CH | $CH_3$ | $CH_3$ | - |
| 10.63 | H | H | $NO_2$ | H | H | H | CH | $CH_3$ | $CH_3$ | - |
| 10.64 | H | H | H | $CF_3$ | H | H | CH | $CH_3$ | $CH_3$ | - |
| 10.65 | H | H | H | $CF_3$ | H | H | CH | $(CH_2)_2OCH_3$ | $(CH_2)_2OCH_3$ | - |
| 10.66 | H | H | Cl | Cl | H | H | CH | $CH_3$ | $CH_3$ | - |
| 10.67 | H | H | Cl | H | Cl | H | CH | $C_2H_5$ | $C_2H_5$ | - |

Fortsetzung Tabelle 10:

| Verb. Nr. | $R_{11}$ | $R_{12}$ | $R_{13}$ | $R_{14}$ | $R_{15}$ | $R_{16}$ | Y | U | V | Salz |
|---|---|---|---|---|---|---|---|---|---|---|
| 10.68 | H | H | Cl | H | H | 5-Cl | N | $C_2H_5$ | $C_2H_5$ | - |
| 10.69 | H | H | Cl | H | H | 5-Cl | CH | $CH_3$ | $CH_3$ | - |
| 10.70 | H | H | Cl | H | H | 6-Cl | CH | $C_4H_9$-n | $C_4H_9$-n | - |
| 10.71 | H | H | H | Cl | Cl | H | N | $CH_3$ | $CH_3$ | - |
| 10.72 | H | H | H | Cl | H | 5-Cl | CH | $CH_3$ | $CH_3$ | - |
| 10.73 | H | H | H | Cl | Cl | H | CH | $CH_3$ | $CH_3$ | |
| 10.74 | H | H | Br | H | Br | H | CH | $CH_3$ | $CH_3$ | - |
| 10.75 | H | H | Cl | H | Br | H | CH | $C_2H_5$ | $C_2H_5$ | - |
| 10.76 | H | H | $CH_3$ | $CH_3$ | H | H | CH | $CH_3$ | $CH_3$ | - |
| 10.77 | H | H | $CH_3$ | H | H | 5-$CH_3$ | CH | $C_2H_5$ | $C_2H_5$ | - |
| 10.78 | H | H | $C_4H_9$-t | H | $C_4H_9$-t | H | CH | $C_2H_5$ | $C_2H_5$ | - |
| 10.79 | H | H | H | $CH_3$ | H | 5-$CH_3$ | CH | $(CH_2)_2OCH_3$ | $(CH_2)_2OCH_3$ | |
| 10.80 | H | H | $C_3H_7$-i | H | H | 5-$CH_3$ | CH | $CH_3$ | $CH_3$ | - |
| 10.81 | H | H | Cl | H | Cl | 5-Cl | CH | $C_2H_5$ | $C_2H_5$ | - |
| 10.82 | H | H | Cl | Cl | Cl | H | CH | $(CH_2)_2OCH_3$ | $(CH_2)_2OCH_3$ | - |
| 10.83 | H | H | Cl | Cl | H | 5-Cl | CH | $C_2H_5$ | $C_2H_5$ | - |
| 10.84 | H | H | Br | H | Br | 6-Br | CH | $(CH_2)_2OC_2H_5$ | $(CH_2)_2OC_2H_5$ | - |
| 10.85 | H | H | $CH_3$ | H | Cl | H | CH | $CH_3$ | $CH_3$ | - |

Fortsetzung Tabelle 10:

| Verb. Nr. | $R_{11}$ | $R_{12}$ | $R_{13}$ | $R_{14}$ | $R_{15}$ | $R_{16}$ | Y | U | V | Salz |
|---|---|---|---|---|---|---|---|---|---|---|
| 10.86 | H | H | H | $CH_3$ | Cl | H | CH | $CH_3$ | $CH_3$ | – |
| 10.87 | H | H | H | $CH_3$ | Cl | H | N | $(CH_2)_2OCH_3$ | $(CH_2)_2OCH_3$ | – |
| 10.88 | H | H | $C_4H_9$-t | H | Cl | H | CH | $CH_3$ | $CH_3$ | – |
| 10.89 | H | H | $C_4H_9$-t | H | Cl | H | N | $C_2H_4Cl$ | $C_2H_4Cl$ | – |
| 10.90 | H | H | Br | H | $CH_3$ | H | CH | $C_8H_{17}$-n | $C_8H_{17}$-n | – |
| 10.91 | H | H | $NO_2$ | H | $CF_3$ | H | CH | $CH_3$ | $CH_3$ | – |
| 10.92 | H | H | H | $CH_3$ | Cl | 5-$CH_3$ | CH | $C_7H_{18}$-n | $C_7H_{18}$-n | – |
| 10.93 | H | H | $CF_3$ | H | $NO_2$ | H | CH | $CH_3$ | $CH_3$ | – |
| 10.94 | H | H | H | $CH_3$ | Cl | 5-$CH_3$ | CH | $C_2H_4Cl$ | $C_2H_4Cl$ | – |
| 10.95 | H | H | $CF_3$ | H | $NO_2$ | 6-$CF_3$ | CH | $CH_3$ | $CH_3$ | – |
| 10.96 | H | H | $CF_3$ | H | $NO_2$ | 6-$CF_3$ | N | $C_2H_5$ | $C_2H_5$ | – |
| 10.97 | H | H | $CF_3$ | H | $NO_2$ | 6-$NO_2$ | CH | $C_2H_5$ | $C_2H_5$ | – |
| 10.98 | H | H | Br | H | $CH_3$ | H | CH | $C_2H_4Cl$ | $C_2H_4Cl$ | – |
| 10.99 | H | H | H | H | F | H | CH | $C_4H_9$-n | $C_4H_9$-n | – |
| 10.100 | H | H | H | H | F | H | CH | $(CH_2)_2OCH_3$ | $(CH_2)_2OCH_3$ | – |
| 10.101 | H | H | H | H | F | H | CH | $C_2H_4Cl$ | $C_2H_4Cl$ | – |
| 10.102 | H | H | H | H | F | H | CH | $C_2H_5$ | $C_2H_5$ | – |
| 10.103 | H | H | H | H | F | H | N | $(CH_2)_2OCH_3$ | $(CH_2)_2OCH_3$ | – |

Formulierungsbeispiele für agrochemische Mittel, enthaltend flüssige Wirkstoffe der Formel I (% = Gewichtzprozent)

| *10. Emulsions-Konzentrate* | a) | b) | c) |
|---|---|---|---|
| Wirkstoff aus den Tabellen 1 bis 10 | 25% | 40% | 50% |
| Ca-Dodecylbenzolsulfonat | 5% | 8% | 6% |
| Ricinusöl-polyethylenglykolether (36 Mol Ethylenoxid) | 5% | — | — |
| Tributylphenoyl-polyethylenglykolether (30 Mol Ethylenoxid) | — | 12% | 4% |
| Cyclohexanon | — | 15% | 20% |
| Xylolgemisch | 65% | 25% | 20% |

Aus solchen Konzentraten können durch Verdünnen mit Wasser Emulsionen jeder gewünsachten Konzentration hergestellt werden.

| *11. Lösungen* | a) | b) | c) | d) |
|---|---|---|---|---|
| Wirkstoff aus den Tabellen 1 bis 10 | 80% | 10% | 5% | 95% |
| Ethylenglykol-monomethyl-ether | 20% | — | — | — |
| Polyethylenglykol M G 400 | — | 70% | — | — |
| N-Methyl-2-pyrrolidon | — | 20% | — | — |
| Epoxydiertes Kokosnussöl | — | — | 1% | 5% |
| Benzin (Siedegrenzen 160—190°C) | — | — | 94% | — |

(MG = Molekulargewicht)

Die Lösungen sind zur Anwendung in Form kleinster Tropfen geeignet.

| *12. Granulate* | a) | b) |
|---|---|---|
| Wirkstoff aus den Tabellen 1 bis 10 | 5% | 10% |
| Kaolin | 94% | — |
| Hochdisperse Kieselsäure | 1% | — |
| Attapulgit | — | 90% |

Der Wirkstoff wird in Methylenchlorid gelöst, auf den Träger aufgesprüht und das Lösungsmittel anschliessend im Vakuum abgedampft.

| *13. Stäubemittel* | da) | b) |
|---|---|---|
| Wirkstoff aus den Tabellen 1 bis 10 | 2% | 5% |
| Hochdisperse Kieselsäure | 1% | 5% |
| Talkum | 97% | — |
| Kaolin | — | 90% |

Durch inniges Vermischen auf Trägerstoffe mit dem Wirkstoff erhält man gebrauchsfertige Stäubemittel.

Formulierungsbeispiele für agrochemische Mittel, enthaltend feste Wirkstoff der Formel I (% = Gewichtsprozent)

*14. Spritzpulver*

| | a) | b) | c) |
|---|---|---|---|
| Wirkstoff aus den Tabellen 1 bis 10 | 25% | 50% | 75% |
| Na-Ligninsulfonat | 5% | 5% | — |
| Na-Laurylsulfat | 3% | — | 5% |
| Na-Diisobutylnaphthalinsulfonat | — | 6% | 10% |
| Octylphenolpolyethylenglykolether (7—8 Mol Ethylenoxid) | — | 2% | — |
| Hochdisperse Kieselsäure | 5% | 10% | 10% |
| Kaolin | 62% | 27% | — |

Der Wirkstoff wird den Zusatzstoffen gut vermischt und in einer geeigneten Mühle gut verhalen. Man erhält Spritzpulver, die sich mit Wasser zu Suspensionen jeder gewünschten Konzentration verdünnen lassen.

*15. Emulsions-Konzentrat*

| | |
|---|---|
| Wirkstoff aus den Tabellen 1 bis 10 | 10% |
| Octylphenolpolyethylenglykolether (4—5 Mol Ethylenoxid) | 3% |
| Ca-Dodecylbenzolsulfonat | 3% |
| Ricinusölpolyglykolether (35 Mol Ethylenoxid) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

*16. Stäubemittel*

| | a) | b) |
|---|---|---|
| Wirkstoff aus den Tabellen 1 bis 10 | 5% | 8% |
| Talkum | 95% | — |
| Kaolin | — | 92% |

Man erhält anwendungsfertige Stäubemittel, indem der Wirkstoff mit den Träger vermischt und auf einer geeigneten Mühle vermahlen wird.

*17. Extruder Granulat*

| | |
|---|---|
| Wirkstoff aus den Tabellen 1 bis 10 | 10% |
| Na-Ligninsulfonat | 2% |
| Carboxymethylcellulose | 1% |
| Kaolin | 87% |

Der Wirkstoff wird mit den Zusatzstoffen vermischt, vermahlen unt mit Wasser angefeuchtet. Dieses Gemisch wird extrudiert und anschliessend im Luftstrom getrocknet.

*18. Umhüllungs-Granulat*

| | |
|---|---|
| Wirkstoff aus den Tabellen 1 bis 10 | 3% |
| Polyethylenglykol (M G (200) | 3% |
| Kaolin | 94% |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

*19. Suspensions-Konzentrat*

| | |
|---|---|
| Wirkstoff aus den Tabellen 1 bis 10 | 40% |
| Ethylenglykol | 10% |
| Nonylphenolpolyethylenglykolether (15 Mol Ethylenoxid) | 6% |
| N-Lingninsulfonat | 10% |
| Carboxymethylcellulose | 1% |
| 37%ige wässrige Formaldehyd-Lösung | 0,2% |
| Silikonöl in Form einer 75%igen | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

## Biologische Beispiele

### Beispiel 1

### Wirkung gegen Puccinia graminis auf Weizen

a) Residual-preotektive Wirkung

Weizenpflanzen wurden 6 Tage nach der Aussat mit einer aus Spritzpulver des Wirkstoffes hergestellten Spritzbrühe (0,06% Aktivsubstanz) besprüht. Nach 24 Stunden wurden die behandelten Pflanzen mit einer Uredosporensuspension des Pilzes infiziert. Nach einer Inkubation während 48 Stunden bei 95—100% relativer Luftfeuchtigkeit und ca. 20°C wurden die infizierten Pflanzen in einem Gewächshaus bei ca. 22°C aufgestellt. Die Beurteilung der Rostpustelnentwicklung erfolgte 12 Tage nach der Infektion.

b) Systemische Wirkung

Zu Weizenpflanzen wurde 5 Tage nach der Aussaat eine aus Spritzpulver des Wirkstoffes hergestellte Spritzbrühe gegossen (0,006% Aktivsubstanz bezogen auf das Bodenvolumen). Nach 48 Stunden wurden die behandelten Pflanzen mit einer Uredosporensuspension des Pilzes infiziert. Nach einer Inkubation während 48 Stunden bei 95—100% relativer Luftfeuchtigkeit und ca. 20°C wurden die infizierten Pflanzen in einem Gewächshaus bei ca. 22°C aufgestellt. Die Beurteilung der Rostpustelentwicklung erfolgte 12 Tage nach der Infektion.

Verbindungen aus den Tabellen 1 bis 10 zeigten gegen Puccinia-Pilze eine gute Wirkung. Unbehandelte aber inifizierte Kontrollpflanzen zeigten einen Puccinia-Befall von 100%. Unter anderem hemmten die Verbindungen Nr. 1.17, 1.24, 2.17, 3.1, 3.6, 3.7, 4.50, 4.77, 4.81, 4.90, 4.91, 4.93, 4.94, 5.13, 5.14, 5.36, 5.70 und 6.8 bei residual-protektiver Behandlung den Pilzbefall auf 0 bis 5%. Darüberhinaus zeigte Verbindung Nr. 6.8 volle systemische Wirkung (0% Befall) auch noch bei einer Verdünnung auf 0,006%.

### Beispiel 2

### Wirkung gegen Cercospora arachidicola auf Erdnusspflanzen

10—15 cm hohe Erdnusspflanzen wurden mit einer aus Spritzpulver der Wirksubstanz hergestellten Spritzbrühe (0,02% Aktivsubstanz) besprüht und 48 Stunden später mit einer Konidiensuspension des Pilzes infiziert. Die infizierten Pflanzen wurden während 72 Stunden bei ca. 21°C und hoher Luftfeuchtigkeit inkubiert und anschliessend bis zum Auftreten der typischen Blattflecken in einem Gewächshaus aufgestellt. Die Beurteilung der fungiziden Wirkung erfolgte 12 Tage nach der Infektion basierend auf Anzahl und Grösse der auftretenden Flecken.

Im Vergleich zu unbehandelten, aber inifizierten Kontrollpflanzen (Anzahl und Grösse der Flecken = 100%), zeigten Erdnusspflanzen, die mit Wirkstoffen aus den Tabellen 1 bis 10 behandelte wurden, einen stark reduzierten Cercospora-Befall. So verhinderten die Verbindungen Nr. 1.9, 1.17, 1.24, 2.17, 3.6, 3.7, 3.11, 4.50, 4.89, 4.90, 4.91, 4.92, 4.92, 4.94, 5.13, 5.14, 5.36, 5.40, 5.70, 5.78, 6.1 und 6.8 in obigem Versuch das Auftreten von Flecken fast vollständig (0 bis 5%).

Beispiel 3
Wirkung gegen Erysiphae graminis auf Gerste

a) Residual-protektive Wirkung

Ca. 8 cm hohe Gerstenpflanzen wurden mit einer aus Spritzpulver des Wirkstoffes hergestellten Spritzbrühe (0,02% Aktivsubstanz) besprüht. Nach 3—4 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

b) Systemische Wirkung

Zu ca. 8 cm hohen Gerstenpflanzen wurde eine aus Spritzpulver des Wirkstoffes hergestellte Spritzbrühe gegossen (0,006% Aktivsubstanz bezogen auf das Erdvolumen). Es wurde dabei darauf geachtet, dass die Spritzbrühe nicht mit dem oberirdischen Pflanzenteilen in Berührung kam. Nach 48 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

Verbindung der Formel I zeigten eine gute residual-protektive Wirkung gegen Erysiphe-Pilze. Unbehandelte, aber infizierte Kontrollpflanzen zeigten einen Erysiphe-Befall von 100%. Unter anderen Verbindungen aus den Tabellen 1 bis 10 hemmten verbindungen Nr. 1.9, 1.17, 1.24, 1.25, 2.17, 3.1, 3.2, 3.6, 3.7, 3.8, 3.11, 3.12, 3.26, 4.50, 4.78, 4.89, 4.90, 4.91, 4.92, 4.93, 4.94, 5.13, 5.14, 5.36, 5.40, 5.70, 5.78, 6.1 und 6.8 den Pilzbefall auf Gerste auf weniger als 5%. Verbindung Nr. 4.50 zeigte diesen Effekt auch bei der Bodenbehandlung (systemische Wirkung) und bei einer Verdünnung auf 0,006%.

Beispiel 4
Residual-protektive Wirkung gegen Venturia inaequalis auf Apfeltrieben

Apfelstecklinge mit 10—20 cm langen Frischtrieben wurden mit einer aus Spritzpulver des Wirkstoffes hergestellten Spritzbrühe (0,06% Aktivsubstanz) besprüht. Nach 24 Stunden wurden die behandelten Pflanzen mit einer Konidiensuspension des Pilzes infiziert. Die Pflanzen wurden dann während 5 Tagen bei 90—100% relativer Luftfeuchtigkeit inkubiert und während 10 weiteren Tagen in einem Gewächshaus bei 20—24°C aufgestellt. Der Schorbefall wurde 15 Tage nach der Infektion beurteilt. Verbindungen Nr. 1.9, 1.17, 1.24, 2.17, 3.6, 3.7, 3.11, 4.50, 4.81, 4.89, 4.90, 4.91, 4.92, 5.13, 5.14, 5.36, 5.40, 5.70, 6.1 und 6.8 und andere hemmten den Krankheitsbefall auf weniger als 10%. Unbehandelte aber infizierte Triebe zeigten einen 100%igen Venturia-Befall.

Beispiel 5
Wirkung gegen Botrytis cinerea auf Bohnen

Residual protektive Wirkung

Ca. 10 cm hohe Bohnen-Pflanzen wurden mit einer aus Spritzpulver des Wirkstoffes hergestellten Spritzbrühe (0,02% Aktivsubstanz) besprüht. Nach 48 Stunden wurden die behandelten Pflanzen mit einer Konidiensuspension des Pilzes infiziert. Nach einer Inkubation der infizierten Pflanzen während 3 Tagen bei 95—100% relativer Luftfeuchtigkeit und 21°C erfolgte die Beurteilung des Pilzbefalls. Die Verbindungen aus den Tabellen 1 bis 10 hemmten in vielen Fällen die Pilzinfektion sehr stark. Bei einer Konzentration von 0,02% erwisen sich z.B. die Verbindungen Nr. 1.9, 3.6, 3.7, 4.50, 4.78, 5.13, 5.36, 5.40, 5.70, 5.77, 6.1 und 6.8als vollwirksam (Krankheitsbefall 0 bis 5%). Der Botrytis-Befall unbehandelter aber infizierter Bohnenpflanzen betrug 100%.

**Patentansprüche für die Vertragsstaaten: BE CH DE FR GB IT LI LU NL SE**

1. Verbindungen der Formel I

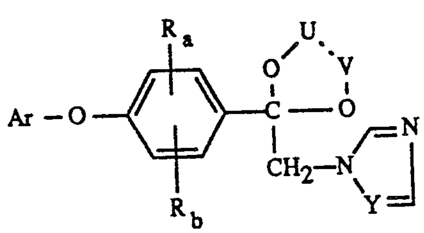

(I)

worin

Y für —CH= oder —N= steht; $R_a$ und $R_b$ unabhängig voneinander für Wasserstoff, Halogen, $C_1$—$C_3$-Alkyl, $C_1$—$C_3$-Alkoxy oder Nitro stehen; Ar für die Gruppe

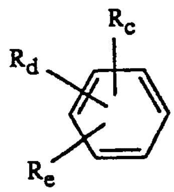

oder

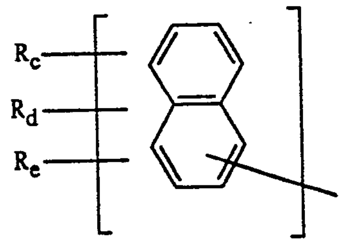

steht; wobei

$R_c$, $R_d$ und $R_e$ unabhängig voneinander für Wasserstoff, Halogen, $C_1$—$C_3$-Alkyl, $C_1$—$C_3$-Alkoxy, Nitro oder $CF_3$ stehen;

U und V unabhängig voneinander für $C_1$—$C_{12}$-Alkyl stehen oder zusammen eine der folgenden Alkylenbrücken

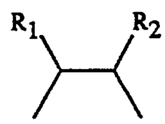

,

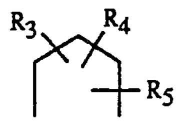

oder

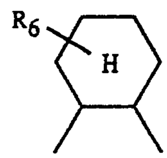

bilden, wobei

$R_1$ und $R_2$ unabhängig voneinander für Wasserstoff, $C_1$—$C_{12}$-Alkyl, ein- oder mehrfach durch Halogen substituiertes $C_1$—$C_{12}$-Alkyl, Phenyl, ein- oder mehrfach durch Halogen und/oder $C_1$—$C_3$-Alkyl substituiertes Phenyl oder für die Gruppe —$CH_2$—Z—$R_7$ stehen, wobei

Z Sauerstoff oder Schwefel bedeutet und

$R_7$ für Wasserstoff, $C_1$—$C_8$-Alkyl, ein durch $C_1$—$C_2$-Alkoxy substituiertes $C_1$—$C_8$-Alkyl, $C_3$—$C_4$-Alkenyl, 2-Propionyl, 3-Halogen-2-propinyl, Phenyl, ein durch Halogen, $C_1$—$C_3$-Alkyl, $C_1$—$C_3$-Alkoxy, Nitro und/oder $CF_3$ ein- oder mehrfach substituiertes Phenyl, Benzyl oder ein durch Halogen, $C_1$—$C_3$-Alkyl und/oder $C_1$—$C_3$-Alkoxy ein- oder mehrfach substituiertes Benzyl steht;

$R_3$, $R_4$ und $R_5$ unabhängig voneinander Wasserstoff oder $C_1$—$C_4$-Alkyl bedeuten, wobei die Gesamtzahl der Kohlenstoffatome in $R_3$, $R_4$ und $R_5$ die Zahl 6 nicht übersteigt und

$R_6$ für Wasserstoff oder $C_1$—$C_3$-Alkyl steht;

unter Einschluss ihrer Säureadditionssalze und Metallkomplexe.

2. Verbindungen der Formel I gemäss Anspruch 1, worin Y für —CH= oder —N= steht; Ar die unter Formel I angegebenen Bedeutungen hat; $R_a$, $R_b$, $R_c$, $R_d$ und $R_e$ unabhängig voneinander für Wasserstoff, Chlor, Brom, Fluor, Methyl, Methoxy oder Nitro stehen; U und V unabhängig voneinander für $C_1$—$C_3$-Alkyl stehen oder zusammen eine der unter Formel I genannten Alkylengruppen bilden, wobei $R_1$, $R_2$, $R_3$, $R_4$, $R_5$ und $R_6$ unabhängig voneinander für Wasserstoff oder $C_1$—$C_3$-Alkyl stehen oder $R_1$ für —$CH_2$—O—$R_7$ steht, wobei $R_7$ $C_1$—$C_3$-Alkyl, durch $C_1$—$C_3$-Alkoxy substituiertes $C_2$—$C_4$-Alkyl, $C_3$—$C_4$-Alkenyl oder Phenyl bedeutet.

3. 2-[p-(Phenoxy)-phenyl]-2-[1-(1H-1,2,4-triazolyl)-methyl]-4-methyl-1,3-dioxan oder ein Salz davon.

4. 2-[p-(Phenoxy)-phenyl]-2-[1-(1H-1,2,4-triazolyl)-methyl]-4-ethyl-1,3-dioxan oder ein Salz davon.

5. 2-[p-(Phenoxy)-phenyl]-2-[1-(1H-1,2,4-triazolyl)-methyl]-4-methyl-1,3-dioxolan oder ein Salz davon.

6. 2-[p-(Phenoxy)-phenyl]-2-[1-(1H-1,2,4-triazolyl)-methyl]-4-ethyl-1,3-dioxolan oder ein Salz davon.

7. 2-[p-(Phenoxy)-phenyl]-2-[1-(1H-1,2,4-triazolyl)-methyl]-4-methyl-5-methyl-1,3-dioxolan.

8. 2-[p-(Phenoxy)-phenyl]-2-[1-(1H-1,2,4-triazolyl)-methyl]-1,3-dioxan oder ein Salz davon.

9. 2-[p-(Phenoxy)-phenyl]-2-(1-imidazolylmethyl)-4-ethyl-1,3-dioxolan oder ein Salz davon.

10. 2-[p-(3-Chlorphenoxy)-phenyl]-2-(1-imidazolylmethyl)-4-ethyl-1,3-dioxolan oder ein Salz davon.

11. 2-[p-(4-Chlorphenoxy)-phenyl]-2-(1-imidazolylmethyl)-4-ethyl-1,3-dioxolan oder ein Salz davon.

12. 2-[p-(Phenoxy)-phenyl]-2-[1-(1H-1,2,4-triazolyl)-methyl]-4-hydroxymethyl-1,3-dioxolan.

13. 2-[p-(2,5-Dichlorphenoxy)-phenyl]-2-(1-imidazolylmethyl)-4-ethyl-1,3-dioxolan oder ein Salz davon.

14. Verbindungen der Formel I

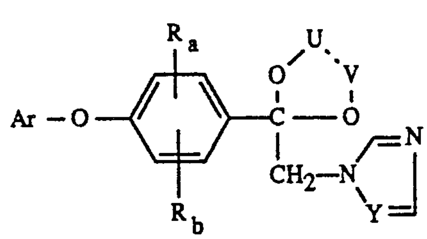

(I)

worin

Y für —CH= oder —N= steht;

$R_a$ und $R_b$ unabhängig voneinander für Wasserstoff, Halogen, $C_1$—$C_3$-Alkyl, $C_1$—$C_3$-Alkoxy oder Nitro stehen;

Ar unsubstituiertes oder durch Halogen, $C_1$—$C_7$-Alkyl, $C_1$—$C_7$-Alkoxy, Nitro und/oder $CF_3$ ein- oder mehrfach substituiertes Phenyl oder Naphthyl bedeutet;

U and V unabhängig voneinander für gegebenenfalls durch Halogen oder $C_1$—$C_6$-Alkoxy substituiertes $C_1$—$C_{12}$-Alkyl stehen oder zusammen eine der folgenden Alkylenbrücken

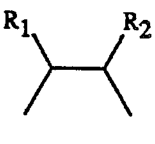 , 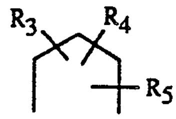 oder 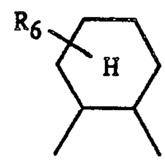

bilden, wobei

$R_1$ und $R_2$ unabhängig voneinander für Wasserstoff, $C_1$—$C_{12}$-Akyl, ein- oder mehrfach durch Halogen substituiertes $C_1$—$C_{12}$-Alkyl, Phenyl, ein- oder mehrfach durch Halogen und/oder $C_1$—$C_3$-Alkyl substituiertes Phenyl oder für die Gruppe —$CH_2$—Z—$R_7$ stehen, wobei

Z Sauerstoff oder Schwefel bedeutet und

$R_7$ für Wasserstoff, $C_1$—$C_8$-Alkyl, ein durch $C_1$—$C_2$-Alkoxy substituiertes $C_1$—$C_8$-Alkyl, $C_3$—$C_4$-Alkenyl, 2-Propinyl, 3-Halogen-2-propinyl, Phenyl, ein durch Halogen, $C_1$—$C_3$-Alkyl, $C_1$—$C_3$-Alkoxy, Nitro und/oder $CF_3$ ein- oder mehrfach substituiertes Phenyl, Benzyl oder ein durch Halogen, $C_1$—$C_3$-Alkyl und/oder $C_1$—$C_3$-Alkoxy ein- oder mehrfach substituiertes Benzyl steht;

$R_3$, $R_4$ und $R_5$ unabhängig voneinander Wasserstoff oder $C_1$—$C_4$-Alkyl bedeuten, wobei die Gesamtzahl der Kohlenstoffatome in $R_3$, $R_4$ und $R_5$ die Zahl 6 nicht übersteigt und

$R_6$ für Wasserstoff oder $C_1$—$C_3$-Alkyl steht;

unter Einschluss ihrer Säureadditionssalze und Metallkomplexe, mit der Massgabe, dass der Gegenstand des Anspruchs 1 vom Schutzumfang ausgenommen ist.

15. 2-[p-(3,4-Dichlorphenoxy)-phenyl]-2-(1-imidazolylmethyl)-4-methoxymethyl-1,3-dioxolan.

16. 2-[p-(4-Chlor-2-methyl-phenoxy)-phenyl]-2-(1-imidazolylmethyl)-4-methoxymethyl-1,3-dioxolan.

17. 2-[p-(4-Fluorphenoxy)-phenyl]-2-(1-imidazolylmethyl)-4-hydroxymethyl-1,3-dioxolan.

18. 2-[p-(4-Fluorphenoxy)-phenyl]-2-[1-(1H-1,2,4-triazolyl-methyl)]-1,3-dioxan.

19. Verfahren zur Herstellung von Verbindungen der Formel I gemäss Anspruch 1, dadurch gekennzeichnet, das man

a) eine Verbindung der Formel II

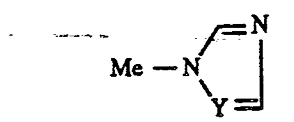 (II)

worin Me Wasserstoff oder ein Metallkation darstellt, mit einer Verbindung der Formel III

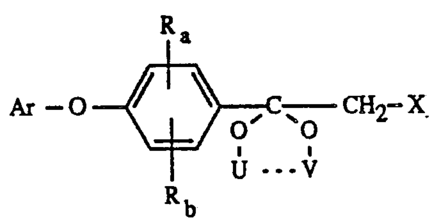 (III)

worin X eine Abgangsgruppe bedeutet, kondensiert oder

b) in einer Verbindung der Formel IV

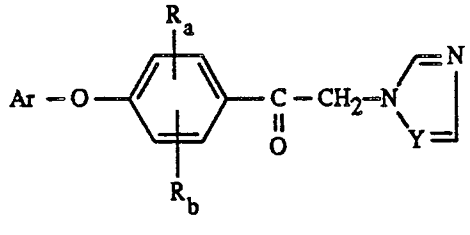 (IV)

die Carbonylgruppe durch Umsetzung mit einem Diol der Formel HO—U- - - - V—OH (Vb) in eine Gruppe der Formel V

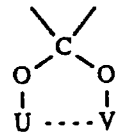

(V)

überführt, oder

C) Verbindungen der Formeln VIII und IX

Ar-$X_3$ (VIII) und 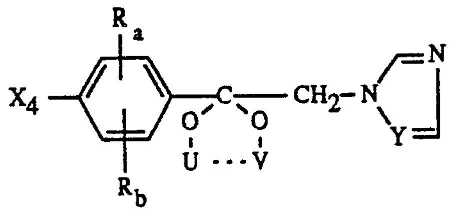 (IX)

worin $X_3$ eine Gruppe —O—Me, in der Me Wasserstoff oder vorzugsweise ein Metallkation ist und auch $X_4$ Fluor bedeutet, miteinander kondensiert und, wenn erwünscht eine verfahrensgemäss erhältliche freie Verbindung in ein Säureadditionssalz, ein verfahrensgemäss erhältliches Säureadditionssalz in die freie Verbindung oder in ein anderes Säureadditionssalz, oder eine verfahrensgemäss erhältliche freie Verbindung bzw. ein verfahrensgemäss erhältliches Säureadditionssalz in einen Metallkomplex überführt, wobei die Substituenten in den Formeln II bis IX die unter Formel I angegebenen Bedeutungen haben und Me Wasserstoff oder ein Metallkation bedeutet.

20. Verfahren zur Herstellung von Verbindungen der Formel I gemäss Anspruch 1, worin U and V gemeinsam eine Gruppe der Formel —$CH_2$—$CH(CH_2ZR_7')$- darstellen und $R_7'$ einen von Wasserstoff verschiedenen Rest $R_7$ bedeutet, dadurch gekennzeichnet, dass man eine Verbindung der Formel VI

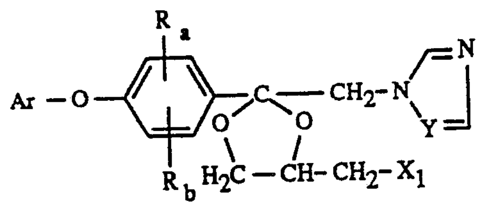

(VI)

mit einer Verbindung der Formel VIII zur Reaktion bringt

$$X_2—R_7 \qquad (VII)$$

worin einer der Reste $X_1$ und $X_2$ Hydroxy oder Mercapto und der andere eine nukleophile Abgangsgruppe X bedeutet oder sowohl $X_1$ als auch $X_2$ Hydroxygruppen darstellen, und worin die übrigen Substituenten Ar, $R_a$, $R_b$, Y, Z, $R_7$ gemäss Anspruch 1 definiert sind, und,

wenn erwünscht, eine verfahrensgemäss erhältliche freie Verbindung in ein Säureadditionssalz, ein verfahrensgemäss erhältliches Säureadditionssalz in die freie Verbindung oder in ein anderes Säureadditionssalz, oder eine verfahrensgemäss erhälitliche freie Verbindung bzw. ein verfahrensgemäss erhältliches Säureadditionssalz in einen Metallkomplex überführt.

21. Verfahren gemäss Anspruch 19 zur Herstellung von Verbindungen der Formel I gemäß Anspruch 14 mit Ausnahme der Herstellung jener Verbindung der Formel I, die den Gegenstand des Anspruchs 1 dartellen.

22. Agrochemisches Schädlingsbekämpfungsmittel zur Bekämpfung oder Verhütung eines Befalls durch Mikroorganismen, dadurch gekennzeichnet, dass es als mindestens eine aktive Komponente eine Verbindung der Formel I gemäss Anspruch 1 enthält.

23. Mittel gemäss Anspruch 22, dadurch gekennzeichnet, dass es als mindestens eine aktive Komponente eine Verbindung der Formel I gemäss einem der Ansprüche 2 bis 13 enthält.

24. Agrochemisches Schädlingsbekämpfungsmittel zur Bekämpfung oder Verhütung eines Befalls durch Mikroorganismen, dadurch gekennzeichnet, dass es als mindestens eine aktive Komponente eine Verbindung der Formel I gemäss Anspruch 14 enthält.

25. Mittel gemäss Anspruch 24, dadurch gekennzeichnet, das es als mindestens eine aktive Komponente eine Verbindung der Formel I gemäss einem der Ansprüche 15 bis 18 enthält.

26. Verwendung von Verbindungen der Formel I gemäss Anspruch 1 zur Bekämpfung und/oder zur präventiven Verhütung eines Befalls von Mikroorganismen an Pflanzen.

27. Verwendung nach Anspruch 26 von Verbindungen der Formel I gemäss einem der Ansprüche 2 bis 13.

28. Verwendung von Verbindungen der Formel I gemäss Anspruch 14 zur Bekämpfung und/oder zur präventiven Verhütung eines Befalls von Mikroorganismen an Pflanzen.

29. Verwendung nach Anspruch 28 von Verbindungen der Formel I gemäss einem der Ansprüche 15 bis 18.

30. Verwendung gemäss einem der Ansprüche 26 bis 29, dadurch gekennzeichnet, dass es sich bei den Mikroorganismen um phytopathogene Pilze handelt.

**Patentansprüche für den Vertragsstaat: AT**

1. Agrochemisches Schädlingsbekämpfungsmittel zur Bekämpfung oder Verhütung eines Befalls durch Mikroorganismen, dadurch gekennzeichnet, dass es als mindestens eine aktive Komponente eine Verbindung der Formel I

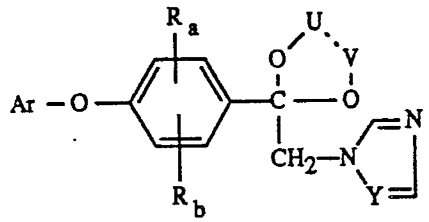
(I)

enthalt,
worin
Y für —CH= oder —N= steht; $R_a$ und $R_b$ unabhängig voneinander für Wasserstoff, Halogen, $C_1$—$C_3$-Alkyl, $C_1$—$C_3$-Alkoxy oder Nitro stehen; Ar für die Gruppe

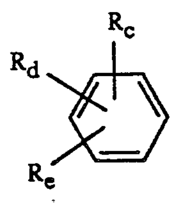
oder
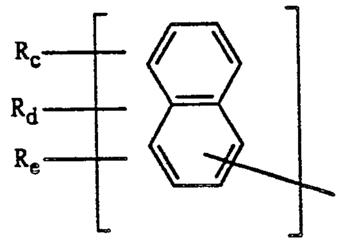

steht; wobei
$R_c$, $R_d$ und $R_e$ unabhängig voneinander für Wasserstoff, Halogen, $C_1$—$C_3$-Alkyl, $C_1$—$C_3$-Alkoxy, Nitro oder $CF_3$ stehen;
U und V unabhängig voneinander für $C_1$—$C_{12}$-Alkyl stehen oder zusammen eine der folgenden Alkylenbrücken

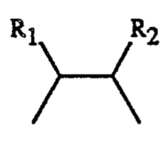
,
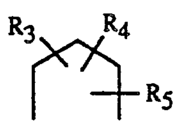
oder
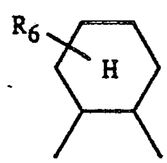

bilden, wobei
$R_1$ und $R_2$ unabhängig voneinander für Wasserstoff, $C_1$—$C_{12}$-Alkyl, ein- oder mehrfach durch Halogen substituiertes $C_1$—$C_{12}$-Alkyl, Phenyl, ein- oder mehrfach durch Halogen und/oder $C_1$—$C_3$-Alkyl substituiertes Phenyl oder für die Gruppe —$CH_2$—Z—$R_7$ stehen, wobei
Z Sauerstoff oder Schwefel bedeutet und
$R_7$ für Wasserstoff, $C_1$—$C_8$-Alkyl, ein durch $C_1$—$C_2$-Alkoxy substituiertes $C_1$—$C_8$-Alkyl, $C_3$—$C_4$-Alkenyl, 2-Propionyl, 3-Halogen-2-propinyl, Phenyl, ein durch Halogen, $C_1$—$C_3$-Alkyl, $C_1$—$C_3$-Alkoxy, Nitro und/oder $CF_3$ ein- oder mehrfach substituiertes Phenyl, Benzyl oder ein durch Halogen, $C_1$—$C_3$-Alkyl und/oder $C_1$—$C_3$-Alkoxy ein- oder mehrfach substituiertes Benzyl steht;
$R_3$, $R_4$ und $R_5$ unabhängig voneinander Wasserstoff oder $C_1$—$C_4$-Alkyl bedeuten, wobei die Gesamtzahl der Kohlenstoffatome in $R_3$, $R_4$ und $R_5$ die Zahl 6 nicht übersteigt und
$R_6$ für Wasserstoff oder $C_1$—$C_3$-Alkyl steht;
unter Einschluss ihrer Säureadditionssalze und Metallkomplexe.

2. Mittel nach Anspruch 1, dadurch gekennzeichnet, dass es als aktive Komponente eine Verbindung der Formel I enthält, worin Y für —CH= oder —N= steht; Ar die unter Formel I angegebenen Bedeutungen hat; $R_a$, $R_b$, $R_c$, $R_d$ und $R_e$ unabhängig voneinander für Wasserstoff, Chlor, Brom, Fluor, Methyl, Methoxy oder Nitro stehen; U und V unabhängig voneinander für $C_1$—$C_3$-Alkyl stehen oder zusammen eine der unter Formel I genannten Alkylengruppen bilden, wobei $R_1$, $R_2$, $R_3$, $R_4$, $R_5$ und $R_6$ unabhängig voneinander für

Wasserstoff oder $C_1$—$C_3$-Alkyl stehen oder $R_1$ für —$CH_2$—O—$R_7$ steht, wobei $R_7$ $C_1$—$C_3$-Alkyl, durch $C_1$—$C_3$-Alkoxy substituiertes $C_2$—$C_4$-Alkyl, $C_3$—$C_4$-Alkenyl oder Phenyl bedeutet.

3. Mittel nach Anspruch 1, dadurch gekennzeichnet, dass es als aktive Komponente 2 - [p - (Phenoxy) - phenyl] - 2 - [1 - (1H - 1,2,4 - triazolyl) - methyl] - 4 - methyl - 1,3 - dioxan oder ein Salz davon enthält.

4. Mittel nach Anspruch 1, dadurch gekennzeichnet, dass es als aktive Komponente 2 - [p - (Phenoxy) - phenyl] - 2 - [1 - (1H - 1,2,4 - triazolyl) - methyl] - 4 - ethyl - 1,3 - dioxan oder ein Salz davon enthält.

5. Mittel nach Anspruch 1, dadurch gekennzeichnet, dass es als aktive Komponente 2 - [p - (Phenoxy) - phenyl] - 2 - [1 - (1H - 1,2,4 - triazolyl) - methyl] - 4 - methyl - 1,3 - dioxolan oder ein Salz davon enthält.

6. Mittel nach Anspruch 1, dadurch gekennzeichnet, dass es als aktive Komponente eine der Verbindungen

2-[p-(Phenoxy)-phenyl]-2-[1-(1H-1,2,4-triazolyl)-methyl]-4-ethyl-1,3-dioxolan,
2-[p-(Phenoxy)-phenyl]-2-[1-(1H-1,2,4-triazolyl)-methyl]-4-methyl-5-methyl-1,3-dioxolan,
2-[p-(Phenoxy)-phenyl]-2-[1-(1H-1,2,4-triazolyl)-methyl]-1,3-dioxan,
2-[p-(Phenoxy)-phenyl]-2-(1-imidazolylmethyl)-4-ethyl-1,3-dioxolan,
2-[p-(3-Chlorphenoxy)-phenyl]-2-(1-imidazolylmethyl)-4-ethyl-1,3-dioxolan,
2-[p-(4-Chlorphenoxy)-phenyl]-2-(imidazolylmethyl)-4-ethyl-1,3-dioxolan,
2-[p-(Phenoxy)-phenyl-2-[1-(1H-1,2,4-triazolyl)-methyl]-4-hydroxymethyl-1,3-dioxolan,
2-[p-(2,5-Dichlorphenoxy)-phenyl]-2-(1-imidazolylmethyl)-4-ethyl-1,3-dioxolan, oder je ein Salz davon,

enthält.

7. Agrochemisches Schädlingsbekämpfungsmittel zur Bekämpfung oder Verhütung eines Befalls durch Mikroorganismen, dadurch gekennzeichnet, dass es als mindestens eine aktive Komponente eine Verbindung der Formel I

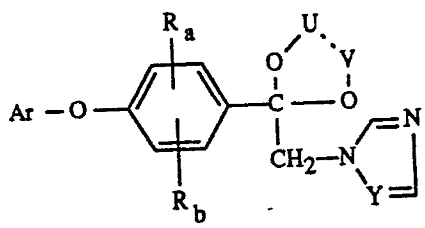
, (I)

enthalt

worin

Y für —CH= oder —N= steht;

$R_a$ und $R_b$ unabhängig voneinander für Wasserstoff, Halogen, $C_1$—$C_3$-Alkyl, $C_1$—$C_3$-Alkoxy oder Nitro stehen;

Ar unsubstituiertes oder durch Halogen, $C_1$—$C_7$-Alkyl, $C_1$—$C_7$-Alkoxy, Nitro und/oder $CF_3$ ein- oder mehrfach substituiertes Phenyl oder Naphthyl bedeutet;

U and V unabhängig voneinander für gegebenenfalls durch Halogen oder $C_1$—$C_6$-Alkoxy substituiertes $C_1$—$C_{12}$-Alkyl stehen oder zusammen eine der folgenden Alkylenbrücken

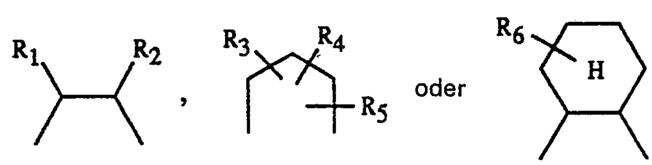

bilden, wobei

$R_1$ und $R_2$ unabhängig voneinander für Wasserstoff, $C_1$—$C_{12}$-Akyl, ein- oder mehrfach durch Halogen substituiertes $C_1$—$C_{12}$-Alkyl, Phenyl, ein- oder mehrfach durch Halogen und/oder $C_1$—$C_3$-Alkyl substituiertes Phenyl oder für die Gruppe —$CH_2$—Z—$R_7$ stehen, wobei

Z Sauerstoff oder Schwefel bedeutet und

$R_7$ für Wasserstoff, $C_1$—$C_8$-Alkyl, ein durch $C_1$—$C_2$-Alkoxy substituiertes $C_1$—$C_8$-Alkyl, $C_3$—$C_4$-Alkenyl, 2-Propinyl, 3-Halogen-2-propinyl, Phenyl, ein durch Halogen, $C_1$—$C_3$-Alkyl, $C_1$—$C_3$-Alkoxy, Nitro und/oder $CF_3$ ein- oder mehrfach substituiertes Phenyl, Benzyl oder ein durch Halogen, $C_1$—$C_3$-Alkyl und/oder $C_1$—$C_3$-Alkoxy ein- oder mehrfach substituiertes Benzyl steht;

$R_3$, $R_4$ und $R_5$ unabhängig voneinander Waserstoff oder $C_1$—$C_4$-Alkyl bedeuten, wobei die Gesamtzahl der Kohlenstoffatome in $R_3$, $R_4$ und $R_5$ die Zahl 6 nicht übersteigt und

$R_6$ für Wasserstoff oder $C_1$—$C_3$-Alkyl steht;

unter Einschluss ihrer Säureadditionssalze und Metallkomplexe, mit Ausnahme der im Anspruch 1 genannten Verbindungen der Formel I und ihrer Säureadditionssalze und Metallkomplex.

8. Mittel nach Anspurch 7, dadurch gekenzeichnet, dass es als aktive Komponente eine der Verbindungen

2-[p-(3,4-Dichlorphenoxy)-phenyl]-2-(1-imidazolylmethyl)-4-methoxymethyl-1,3-dioxolan,

2-[p-(4-Chlor-2-methyl-phenoxy)-phenyl]-2-(1-imidazolylmethyl)-4-methoxymethyl-1,3-dioxolan,

2-[p-(4-Fluorphenoxy)-phenyl]-2-(1-imidazolylmethyl)-4-hydroxymethyl-1,3-dioxolan,

2-[p-(4-Fluorphenoxy)-phenyl]-2-[1-(1H-1,2,4-triazolyl-methyl)]-1,3-dioxan, oder je ein Salz davon, enthält.

9. Verfahren zur Herstellung von Verbindungen der Formel I gemäss Anspruch 1, worin Y für —CH= oder —N= steht; $R_a$ und $R_b$ unabhängig voneinander für Wasserstoff, Halogen, $C_1$—$C_3$-Alkyl, $C_1$—$C_3$-Alkoxy oder Nitro stehen; Ar für die Gruppe

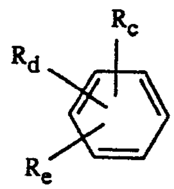

oder

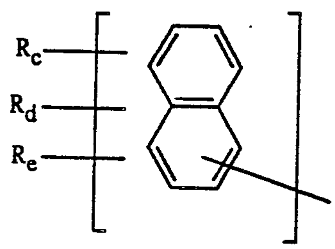

steht; wobei

$R_c$, $R_d$ und $R_e$ unabhängig voneinander für Wasserstoff, Halogen, $C_1$—$C_3$-Alkyl, $C_1$—$C_3$-Alkoxy, Nitro oder $CF_3$ stehen;

U und V unabhängig voneinander für $C_1$—$C_{12}$-Alkyl stehen oder zusammen eine der folgenden Alkylenbrücken

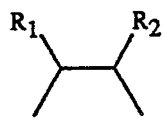

,

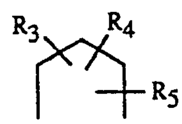

oder

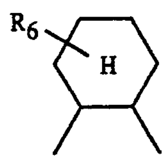

bilden, wobei

$R_1$ und $R_2$ unabhängig voneinander für Wasserstoff, $C_1$—$C_{12}$-Alkyl, ein- oder mehrfach durch Halogen substituiertes $C_1$—$C_{12}$-Alkyl, Phenyl, ein- oder mehrfach durch Halogen und/oder $C_1$—$C_3$-Alkyl substituiertes Phenyl oder für die Gruppe —$CH_2$—Z—$R_7$ stehen, wobei

Z Sauerstoff oder Schwefel bedeutet und

$R_7$ für Wasserstoff, $C_1$—$C_8$-Alkyl, ein durch $C_1$—$C_2$-Alkoxy substituiertes $C_1$—$C_8$-Alkyl, $C_3$—$C_4$-Alkenyl, 2-Propinyl, 3-Halogen-2-propinyl, Phenyl, ein durch Halogen, $C_1$—$C_3$-Alkyl, $C_1$—$C_3$-Alkoxy, Nitro und/oder $CF_3$ ein- oder mehrfach substituiertes Phenyl, Benzyl oder ein durch Halogen, $C_1$—$C_3$-Alkyl und/oder $C_1$—$C_3$-Alkoxy ein- oder mehrfach substituiertes Benzyl steht;

$R_3$, $R_4$ und $R_5$ unabhängig voneinander Wasserstoff oder $C_1$—$C_4$-Alkyl bedeuten, wobei die Gesamtzahl der Kohlenstoffatome in $R_3$, $R_4$ und $R_5$ die Zahl 6 nicht übersteigt und

$R_6$ für Wasserstoff oder $C_1$—$C_3$-Alkyl steht;

unter Einschluss ihrer Säureadditionssalze und Metallkomplexe, dadurch gekennzeichnet, dass man

a) eine Verbindung der Formel II

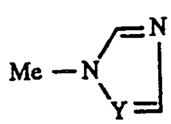

(II)

worin Me Wasserstoff oder ein Metallkation darstellt, mit einer Verbindung der Formel III

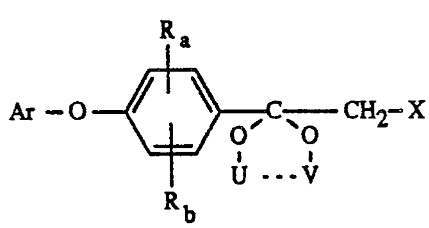

(III)

worin X eine Abgangsgruppe bedeutet, kondensiert oder

b) in einer Verbindung der Formel IV

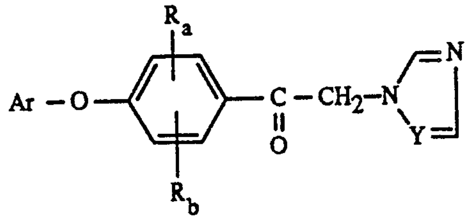

(IV)

die Carbonylgruppe durch Umsetzung mit einem Diol der Formel HO—U- - - - -V—OH (Vb) in eine Gruppe der Formel V

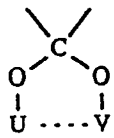

(V)

überführt, oder

c) Verbindungen der Formeln VIII und IX

Ar-$X_3$ (VIII) and

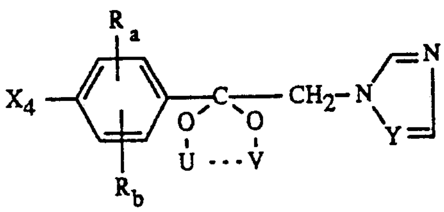

(IX)

worin $X_3$ eine Gruppe —O—Me, in der Me Wasserstoff oder vorzugsweise ein Metallkation ist und auch $X_4$ Fluor bedeutet, miteinander kondensiert und, wenn erwünscht eine verfahrensgemäss erhältliche freie Verbindung in ein Säureadditionssalz, ein verfahrensgemäss erhältliches Säureadditionssalz in die freie Verbindung oder in ein anderes Säureadditionssalz, oder eine verfahrensgemäss erhältliche freie Verbindung bzw. ein verfahrensgemäss erhältliches Säureadditionssalz in einen Metallkomplex überführt, wobei die Substituenten in den Formeln II bis IX die unter Formel I angegebenen Bedeutungen haben und Me Wasserstoff oder ein Metallkation bedeutet.

10. Verfahren zur Herstellung von Verbindungen der Formel I gemäss Anspruch 1, worin U und V gemeinsam eine Gruppe der Formel $-CH_2-CH(CH_2ZR_7')-$ darstellen und $R_7'$ einen von Waserstoff verschiedenen Rest $R_7$ bedeutet, dadurch gekennzeichnet, dass man eine Verbindung der Formel VI

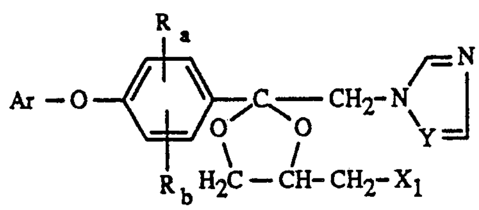

(VI)

mit einer Verbindung der Formel VII zur Reaktion bringt

$$X_2-R_7 \qquad (VII)$$

worin einer der Reste $X_1$ und $X_2$ Hydroxy oder Mercapto und der andere eine nukleophile Abgangsgruppe X bedeutet oder sowohl $X_1$ als auch $X_2$ Hydroxygruppen darstellen, und worin die übrigen Substituenten Ar, $R_a$, $R_b$, Y, Z, $R_7$ gemäss Anspruch 1 definiert sind, und,

wenn erwünscht, eine verfahrensgemäss erhältliche freie Verbindung in ein Säureadditionssalz, ein verfahrensgemäss erhältliches Säureadditionssalz in die freie Verbindung oder in ein anderes Säureadditionssalz, oder eine verfahrensgemäss erhältliche freie Verbindung bzw. ein verfahrensgemäss erhältliches Säureadditionssalz in einen Metallkomplex überführt.

11. Verfahren gemäss Anspruch 9 zur Herstellung von Verbindungen der Formel I gemäss Anspruch 7, mit Ausnahme von Verbindungen der Formel I gemäss Anspruch 1.

12. Verwendung von Verbindungen der Formel I definiert in Anspruch 1 zur Bekämpfung und/oder zur präventiven Verhütung eines Befalls von Mikroorganismen an Pflanzen.

13. Verwendung nach Anspruch 12 von Verbindungen der Formel I definiert in einem der Ansprüche 2 bis 6.

14. Verwendung von Verbindungen der Formel I definiert in Anspruch 7 zur Bekämpfung und/oder zur präventiven Verhütung eines Befalls von Mikroorganismen an Pflanzen.

15. Verwendung nach Anspruch 14 von Verbindungen der Formel I definiert in Anspruch 8.

16. Verwendung gemäss einem der Ansprüche 12 bis 15, dadurch gekennzeichnet, dass es sich bei den Mikroorganismen um phytopathogene Pilze handelt.

**Revendications pour les Etats contractants: BE CH DE FR GB IT LI LU NL SE**

1. Composés de formule I

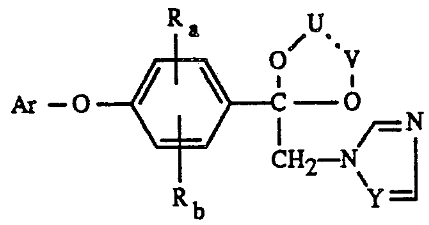

(I)

dans laquelle Y représente —CH= ou —N=; $R_a$ et $R_b$ représentent chacun, indépendamment l'un de l'autre, l'hydrogène, un halogène, un groupe alkyle en $C_1$—$C_3$, alcoxy en $C_1$—$C_3$ ou nitro; Ar représente un groupe

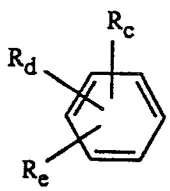

ou

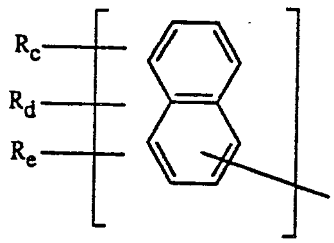

dans lequel

$R_c$, $R_d$ et $R_e$ représentent chacun, indépendamment les uns des autres, l'hydrogène, un halogène, un groupe alkyle en $C_1$—$C_3$, alcoxy en $C_1$—$C_3$, nitro ou $CF_3$;

U et V représentent chacun, indépendamment l'un de l'autre, un groupe alkyle en $C_1$—$C_{12}$ ou forment ensemble l'un des ponts alkylène suivants:

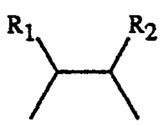

,

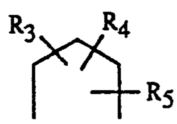

ou

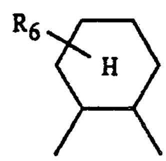

dans lesquels

$R_1$ et $R_2$ représentent chacun, indépendamment l'un de l'autre, l'hydrogène, un groupe alkyle en $C_1$—$C_{12}$, un groupe alkyle en $C_1$—$C_{12}$ mono- ou poly-substitué par des halogènes, un groupe phényle, un groupe phényle mono- ou poly-substitué par des halogènes et/ou des groupes alkyle en $C_1$—$C_3$, ou bien le groupe —$CH_2$—Z—$R_7$ dans lequel

Z représente l'oxygène ou le soufre et

$R_7$ représente l'hydrogène, un groupe alkyle en $C_1$—$C_8$, un groupe alkyle en $C_1$—$C_8$ substitué par un groupe alcoxy en $C_1$—$C_2$, un groupe alcényle en $C_3$—$C_4$, 2-propynyle, 3-halogéno-2-propynyle, phényle, un groupe phényle mono- ou poly-substitué par des halogènes, des groupes alkyle en $C_1$—$C_3$, alcoxy en $C_1$—$C_3$, nitro et/ou $CF_3$, un groupe benzyle ou un groupe benzyle mono- ou poly-substitué par des halogènes, des groupes alkyle en $C_1$—$C_3$ et/ou alcoxy en $C_1$—$C_3$;

$R_3$, $R_4$ et $R_5$ représentent chacun, indépendamment les uns des autres, l'hydrogène ou un groupe alkyle en $C_1$—$C_4$, le nombre total des atomes de carbone de $R_3$, $R_4$ et $R_5$ ne devant pas dépasser 6, et

$R_6$ représente l'hydrogène ou un groupe alkyle en $C_1$—$C_3$;

y compris leurs sels formés par addition avec des acides et complexes métalliques.

2. Composés de formule I selon la revendication 1, dans lesquels Y représente —CH= ou —N=; Ar a les significations indiquées en référence à la formule I;

$R_a$, $R_b$, $R_c$, $R_d$ et $R_e$ représentent chacun, indépendamment les uns des autres, l'hydrogène, le chlore, le brome, le fluor, un groupe méthyle, méthoxy ou nitro; U et V représentent chacun, indépendamment l'un de l'autre, un groupe alkyle en $C_1$—$C_3$ ou forment ensemble l'un des groupes alkylène mentionnés en référence à la formule I, dans lesquels $R_1$, $R_2$, $R_3$, $R_4$, $R_5$ et $R_6$ représentent chacun, indépendamment les uns des autres, l'hydrogène ou un groupe alkyle en $C_1$—$C_3$ ou bien $R_1$ représente un groupe —$CH_2$—O—$R_7$ dans lequel $R_7$ représente un groupe alkyle en $C_1$—$C_3$, un groupe alkyle en $C_2$—$C_4$ substitué par un groupe alcoxy en $C_1$—$C_3$, un groupe alcényle en $C_3$—$C_4$ ou phényle.

3. Le 2-[p-(phénoxy)-phényl]-2-[1-(1H-1,2,4-triazolyl)-méthyl]-4-méthyl-1,3-dioxanne ou un sel de ce composé.

4. Le 2-[p-(phénoxy)-phényl]-2-[1-(1H-1,2,4-triazolyl)-méthyl]-4-éthyl-1,3-dioxanne ou un sel de ce composé.

5. Le 2-[p-(phénoxy)-phényl]-2-[1-(1H-1,2,4-triazolyl)-méthyl]-4-méthyl-1,3-dioxolanne ou un sel de ce composé.

6. Le 2-[p-(phénoxy)-phényl]-2-[1-(1H-1,2,4-triazolyl)-méthyl]-4-éthyl-1,3-dioxolanne ou un sel de ce composé.

7. Le 2-[p-(phénoxy)-phényl]-2-[1-(1H-1,2,4-triazolyl)-méthyl]-4-méthyl-5-méthyl-1,3-dioxolanne.

8. Le 2-[p-(phénoxy)-phényl]-2-[1-(1H-1,2,4-triazolyl)-méthyl]-1,3-dioxanne ou un sel de ce composé.

9. Le 2-[p-(phénoxy)-phényl]-2(1-imidazolylméthyl)-4-éthyl-1,3-dioxolanne ou un sel de ce composé.

10. Le 2-[p-(3-chlorophénoxy)-phényl]-2-(1-imidazolylméthyl)-4-éthyl-1,3-dioxolanne ou un sel de ce composé.

11. Le 2-[p-(4-chlorophénoxy)-phényl]-2-(1-imidazolylméthyl)-4-éthyl-1,3-dioxolanne ou un sel de ce composé.

12. Le 2-[p-(phénoxy)-phényl]-2-[1-(1H-1,2,4-triazolyl)-méthyl]-4-hydroxyméthyl-1,3-dioxolanne.

13. Le 2-[p-(2,5-dichlorophénoxy)-phényl]-2-(1-imidazolylméthyl)-4-éthyl-1,3-dioxolanne ou un sel de ce composé.

14. Composés de formule I

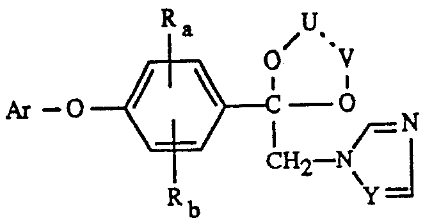

(I)

dans laquelle Y représente —CH= ou —N=;

$R_a$ et $R_b$ représentent chacun, indépendamment l'un de l'autre, l'hydrogène, un halogène; un groupe alkyle en $C_1$—$C_3$, alcoxy en $C_1$—$C_3$ ou nitro;

Ar représente un groupe phényle ou naphtyle non substitué ou mono- ou poly-substitué par des halogènes, des groupes alkyle en $C_1$—$C_7$, alcoxy en $C_1$—$C_7$, nitro et/ou $CF_3$;

U et V représentent chacun, indépendamment l'un de l'autre, un groupe alkyle en $C_1$—$C_{12}$ éventuellement substitué par des halogènes ou des groupes alcoxy en $C_1$—$C_6$ ou forment ensemble l'un des ponts alkylène suivants:

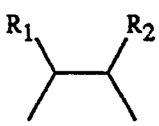 , 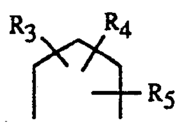 ou 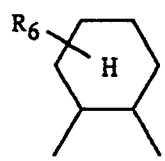

dans lesquels

$R_1$ et $R_2$ représentent chacun, indépendamment l'un de l'autre, l'hydrogène, un groupe alkyle en $C_1$—$C_{12}$, un groupe alkyle en $C_1$—$C_{12}$ mono- ou poly-substitué par des halogènes, un groupe phényle, un groupe phényle mono- ou poly-substitué par des halogènes et/ou des groupes alkyle en $C_1$—$C_3$, ou le groupe —$CH_2$—Z—$R_7$ dans lequel

Z représente l'oxygène ou le soufre, et

$R_7$ représente l'hydrogène, un groupe alkyle en $C_1$—$C_8$, un groupe alkyle en $C_1$—$C_8$ substitué par un groupe alcoxy en $C_1$—$C_2$, un groupe alcényle en $C_3$—$C_4$, 2-propynyle, 3-halogèno-2-propynyle, phényle, un groupe phényle mono- ou poly-substitué par des halogènes, des groupes alkyle en $C_1$—$C_3$, alcoxy en $C_1$—$C_3$, nitro et/ou $CF_3$, un groupe benzyle ou un groupe benzyle mono- ou poly-substitué par des halogènes, des groupes alkyle en $C_1$—$C_3$ et/ou alcoxy en $C_1$—$C_3$;

$R_3$, $R_4$ et $R_5$ représentent chacun, indépendamment les uns des autres, l'hydrogène ou un groupe alkyle en $C_1$—$C_4$, le nombre total des atomes de carbone de $R_3$, $R_4$ et $R_5$ ne devant pas dépasser 6, et

$R_6$ représente l'hydrogène ou un groupe alkyle en $C_1$—$C_3$;

y compris leurs sels formés par addition avec des acides et complexes métalliques,

étant spécifié que l'objet de la revendication 1 est exclu du cadre de la protection.

15. Le 2-[p-(3,4-dichlorophénoxy)-phényl]-2-(1-imidazolylméthyl)-4-méthoxyméthyl-1,3-dioxolanne.

16. Le 2-[p-(4-chloro-2-méthyl-phénoxy)-phényl]-2-(1-imidazolylméthyl)-4-méthoxyméthyl-1,3-dioxolanne.

17. Le 2-[p-(4-fluorophénoxy)-phényl]-2-(1-imidazolylméthyl)-4-hydroxyméthyl-1,3-dioxolanne.

18. Le 2-[p-(4-fluorophénoxy)-phényl]-2-[1-(1H-1,2,4-triazolylméthyl)]-1,3-dioxanne.

19. Procédé de préparation des composés de formule I selon la revendication 1, caractérisé en ce que

a) on condense un composé de formule II

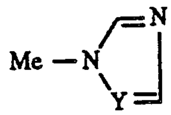 (II)

dans laquelle Me représente l'hydrogène ou un cation métallique, avec un composé de formule III

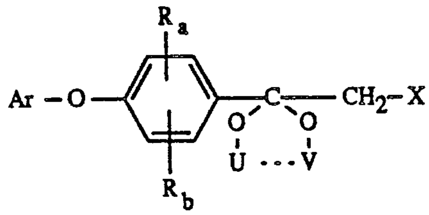 (III)

dans laquelle X représente un groupe éliminable, ou bien

b) dans un composé de formule IV

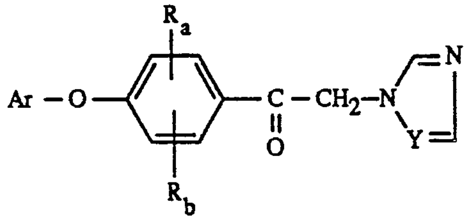 (IV)

on convertit le groupe carbonyle, par réaction avec un diol de formule HO—U——V—OH (Vb) en un groupe de formule V

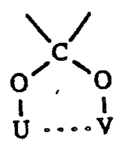 (V)

ou bien

c) on condense entre eux les composés de formules VIII et IX

Ar-$X_3$ (VIII) et 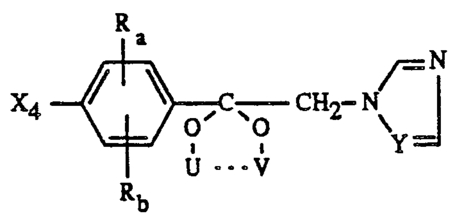 (IX)

dans lesquelles $X_3$ représente un groupe O—Me dans lequel Me représente l'hydrogène ou, de préférence, un cation métallique et $X_4$ peut également représenter le fluor et, si on le désire, on convertit un composé ainsi obtenu à l'état libre en un sel formé par addition avec un acide, un sel d'acide ainsi obtenu en le composé libre ou en un autre sel formé par addition avec un acide, ou bien un composé ainsi obtenu à l'état libre ou à l'état de sel d'acide en un complexe métallique, les symboles des formules II à IX ayant les significations indiquées en référence à la formule I et Me représentant l'hydrogène ou un cation métallique.

20. Procédé de préparation des composés de formule I selon la revendication 1 dans lesquels U et V forment ensemble un groupe de formule —$CH_2$—$CH(CH_2ZR'_7)$— et $R'_7$ est un substituant $R_7$ autre que l'hydrogène, caractérisé en ce que l'on fait réagir un composé de formule VI

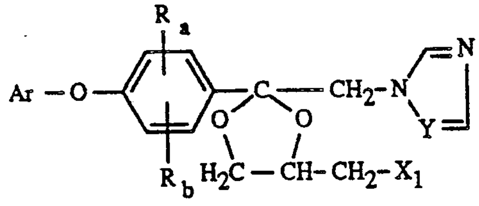 (VI)

avec un composé de formule VII

$X_2$—$R_7$ (VII)

l'un des symboles $X_1$, $X_2$ représentant un groupe hydroxy ou mercapto et l'autre un groupe éliminable nucléophile X ou bien $X_1$ et $X_2$ représentant tous deux des groupes hydroxy, les autres symboles Ar, $R_a$, $R_b$, Y, Z, $R_7$ ayant les significations indiquées dans la revendication 1, et, si on le désire, on convertit un composé ainsi obtenu à l'état libre en un sel par addition avec un acide, un sel d'acide ainsi obtenu en le composé libre ou en un autre sel d'acide, ou bien un composé ainsi obtenu à l'état libre ou à l'état de sel en un complexe métallique.

21. Procédé selon la revendication 19 pour préparer les composés de formule I de la revendication 14, à l'exclusion de la préparation des composés de formule I qui constituent l'objet de la revendication 1.

22. Produit agrochimique pesticide pour prévenir ou combattre une attaque par des microorganismes, caractérisé en ce qu'il contient au moins un composant actif consistant en un composé de formule I selon la revendication 1.

23. Produit selon la revendication 22, caractérisé en ce qu'il contient au moins un composant actif consistant en un composé de formule I selon l'une des revendications 2 à 13.

24. Produit agrochimique pesticide pour combattre ou prévenir une infestation par des microorganismes, caractérisé en ce qu'il contient au moins un composant actif consistant en un composé de formule I selon la revendication 14.

25. Produit selon la revendication 24, caractérisé en ce qu'il contient au moins un composant actif consistant en un composé de formule I selon l'une des revendications 15 à 18.

26. Utilisation des composés de formule I selon la revendication 1 pour combattre et/ou prévenir une attaque de végétaux par des microorganismes.

27. Utilisation selon la revendication 26 des composés de formule I selon l'une des revendications 2 à 13.

28. Utilisation des composés de formule I selon la revendication 14, pour combattre et/ou prévenir une attaque de végétaux par des microorganismes.

29. Utilisation selon la revendication 28 de composés de formule I selon l'une des revendications 15 à 18.

30. Utilisation selon l'une des revendications 26 à 29, caractérisé en ce que les microorganismes sont des mycètes phytopathogènes.

**Revendications pour l'Etat contractant: AT**

1. Produit agrochimique pesticide prévu pour combattre ou prévenir une attaque par des microorganismes, caractérisé en ce qu'il contient au moins un composant actif consistant en un composé de formule I

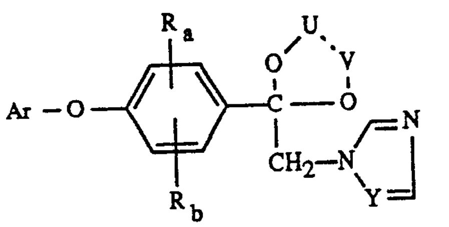

(I)

dans laquelle Y représente —CH= ou —N=; $R_a$ et $R_b$ représentent chacun, indépendamment l'un de l'autre, l'hydrogène, un halogène, un groupe alkyle en $C_1$—$C_3$, alcoxy en $C_1$—$C_3$ ou nitro; Ar représente un groupe

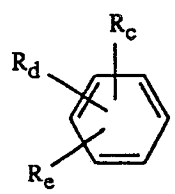

or

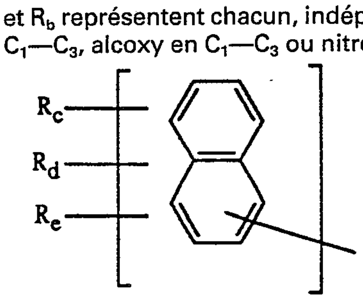

dans lequel

$R_c$, $R_d$ et $R_e$ représentent chacun, indépendamment les uns des autres, l'hydrogène, un halogène, un groupe alkyle en $C_1$—$C_3$, alcoxy en $C_1$—$C_3$, nitro ou $CF_3$;

U et V représentent chacun, indépendamment l'un de l'autre, un groupe alkyle en $C_1$—$C_{12}$ ou forment ensemble l'un des ponts alkylène suivants:

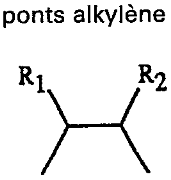

,

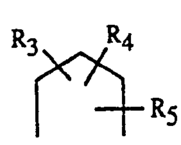

ou

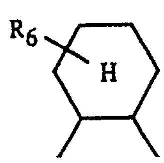

dans lesquels

$R_1$ et $R_2$ représentent chacun, indépendamment l'un de l'autre, l'hydrogène, un groupe alkyle en $C_1$—$C_{12}$, un groupe alkyle en $C_1$—$C_{12}$ mono- ou poly-substitué par des halogènes, un groupe phenyle, un groupe phényle mono- ou poly-substitué par des halogènes et/ou des groupes alkyle en $C_1$—$C_3$, ou le groupe —$CH_2$—Z—$R_7$ dans lequel

Z représente l'oxygène ou le soufre et

$R_7$ représente l'hydrogène, un groupe alkyle en $C_1$—$C_8$, un groupe alkyle en $C_1$—$C_8$ substitué par un groupe alcoxy en $C_1$—$C_2$, un groupe alcényle en $C_3$—$C_4$, un groupe 2-propynyle, 3-halogéno-2-propynyle, phényle, un groupe phényle mono- ou poly-substitué par des halogènes, des groupes alkyle en $C_1$—$C_3$, alcoxy en $C_1$—$C_3$, nitro et/ou $CF_3$, un groupe benzyle ou un groupe benzyle mono- ou poly-substitué par des halogènes, des groupes alkyle en $C_1$—$C_3$ et/ou alcoxy en $C_1$—$C_3$;

$R_3$, $R_4$ et $R_5$ représentent chacun, indépendamment les uns des autres, l'hydrogène ou un groupe alkyle en $C_1$—$C_4$, le nombre total des atomes de carbone de $R_3$, $R_4$ et $R_5$ ne devant pas dépasser 6, et

$R_6$ représente l'hydrogène ou un groupe alkyle en $C_1$—$C_3$;

y compris leurs sels formés par addition avec des acides et complexes métalliques.

2. Produit selon la revendication 1, caractérisé en ce qu'il contient en tant que composant actif un composé de formule I dans laquelle Y représente —CH= ou —N=; Ar a les significations indiquées en référence à la formule I; $R_a$, $R_b$, $R_c$, $R_d$ et $R_e$ représentent chacun, indépendamment les uns des autres, l'hydrogène, le chlore, le brome, le fluor, un groupe méthyle, méthoxy ou nitro; U et V représentent chacun, indépendamment l'un de l'autre, un groupe alkyle en $C_1$—$C_3$ ou forment ensemble l'un des groupes alkylène mentionnés en référence à la formule I, dans lesquels $R_1$, $R_2$, $R_3$, $R_4$, $R_5$ et $R_6$ représentent chacun, indépendamment les uns des autres, l'hydrogène ou un groupe alkyle en $C_1$—$C_3$ ou bien $R_1$ représente un groupe —$CH_2$—O—$R_7$ dans lequel $R_7$ représente un groupe alkyle en $C_1$—$C_3$, un groupe alkyle en $C_2$—$C_4$ substiutué par un groupe alcoxy en $C_1$—$C_3$, un groupe alcényle en $C_3$—$C_4$ ou phényle.

3. Produit selon la revendication 1, caractérisé en ce qu'il contient en tant que composant actif le 2-[p-(phénoxy)-phényl]-2-[1-(1H-1,2,4-triazolyl)-méthyl]-4-méthyl-1,3-dioxanne ou un sel de ce composé.

4. Produit selon la revendication 1, caractérisé en ce qu'il contient en tant que composant actif le 2-[p-(phénoxy)-phényl]-2-[1-(1H-1,2,4-triazolyl)-méthyl]-4-éthyl-1,3-dioxanne ou un sel de ce composé.

5. Produit selon la revendication 1, caractérisé en ce qu'il contient en tant que composant actif le 2-[p-(phénoxy)-phényl]-2-[1-(1H-1,2,4-triazolyl)-méthyl]-4-méthyl-1,3-dioxolanne ou un sel de ce composé.

6. Produit selon la revendication 1, caractérisé en ce qu'il contient en tant que composant actif l'un des composés suivants:

le 2-[p-(phénoxy)-phényl]-2-[1-(1H-1,2,4-triazolyl)-méthyl]-4-éthyl-1,3-dioxolanne,
le 2-[p-(phénoxy)-phényl]-2-[1-(1H-1,2,4-triazolyl)-méthyl]-4-méthyl-5-méthyl-1,3-dioxolanne,
le 2-[p-(phénoxy)-phényl]-2-[1-(1H-1,2,4-triazolyl)-méthyl]-1,3-dioxanne,
le 2-[p-(phénoxy)-phényl]-2-(1-imidazolylméthyl)-4-éthyl-1,3-dioxolanne,
le 2-[p-(3-chlorophénoxy)-phényl]-2-(1-imidazolylméthyl)-4-éthyl-1,3-dioxolanne,
le 2-[p-(4-chlorophénoxy)-phényl]-2-(1-imidazolylméthyl)-4-éthyl-1,3-dioxolanne,
le 2-[p-(phénoxy)-phényl]-2-[1-(1H-1,2,4-triazolyl)-méthyl]-4-hydroxyméthyl-1,3-dioxolanne,
le 2-[p-(2,5-dichlorophénoxy)-phényl]-2-(1-imidazolylméthyl)-4-éthyl-1,3-dioxolanne, ou un sel de l'un de ces composés.

7. Produit agrochimique pesticide pour combattre ou prèvenir une attaque par des microorganismes, caractérisé en ce qu'il contient au moins un composé actif consistant en un composé de formule I

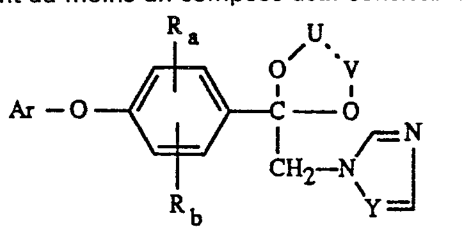

(I)

dans laquelle

Y représente —CH= ou —N=;

$R_a$ et $R_b$ représentent chacun, indépendamment l'un de l'autre, l'hydrogène, un halogène; un groupe alkyle en $C_1$—$C_3$, alcoxy en $C_1$—$C_3$ ou nitro;

Ar représente un groupe phényle ou naphtyle non substitué ou mono- ou poly-substitué par des halogènes, des groupes alkyle en $C_1$—$C_7$, alcoxy en $C_1$—$C_7$, nitro et/ou $CF_3$;

U et V représentent chacun, indépendamment l'un de l'autre, un groupe alkyle en $C_1$—$C_{12}$ éventuellement substitué par des halogènes ou des groupes alcoxy en $C_1$—$C_6$ ou forment ensemble l'un des ponts alkylène suivants:

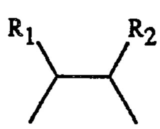 , 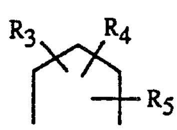 ou 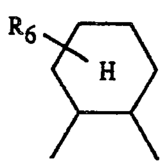

dans lesquels

$R_1$ et $R_2$ représentent chacun, indépendamment l'un de l'autre, l'hydrogène, un groupe alkyle en $C_1$—$C_{12}$, un groupe alkyle en $C_1$—$C_{12}$ mono- ou poly-substitué par des halogènes, un groupe phényle, un groupe phényle mono- ou poly-substitué par des halogènes et/ou des groupes alkyle en $C_1$—$C_3$, ou bien le groupe —$CH_2$—Z—$R_7$ dans lequel

Z représente l'oxygène ou le soufre, et

$R_7$ représente l'hydrogène, un groupe alkyle en $C_1$—$C_8$, un groupe alkyle en $C_1$—$C_8$ substitué par des groupes alcoxy en $C_1$—$C_2$, un groupe alcényle en $C_3$—$C_4$, 2-propynyle, 3-halogèno-2-propynyle, phényle, un groupe phényle mono- ou poly-substitué par des halogènes, des groupes alkyle en $C_1$—$C_3$, alcoxy en $C_1$—$C_3$, nitro et/ou $CF_3$, un groupe benzyle ou un groupe benzyle mono- ou poly-substitué par des halogènes, des groupes alkyle en $C_1$—$C_3$ et/ou alcoxy en $C_1$—$C_3$;

$R_3$, $R_4$ et $R_5$ représentent chacun, indépendamment les uns des autres, l'hydrogène ou un groupe alkyle en $C_1$—$C_4$, le nombre total des atomes de carbone ne devant pas dépasser 6, et

$R_6$ représente l'hydrogène ou un groupe alkyle en $C_1$—$C_3$;

y compris leurs sels formés par addition et complexes métalliques, a l'exception des composés de formule I mentionnés dans la revendication 1 et de leurs sels formés par addition avec des acides et complexes métalliques.

8. Produit selon la revendication 7, caractérisé en ce qu'il contient en tant que composant actif l'un des composés suivants:

2-[p-(3,4-dichlorophénoxy)-phéhyl]-2-(1-imidazolylméthyl)-4-méthoxyméthyl-1,3-dioxolanne,

2-[p-(4-chloro-2-méthyl-phénoxy)-phéhyl]-2-(1-imidazolylméthyl)-4-méthoxyméthyl-1,3-dioxolanne,

2-[p-(4-fluorophénoxy)-phényl]-2-(1-imidazolylméthyl)-4-hydroxyméthyl-1,3-dioxolanne,

2-[p-(4-fluorophénoxy)-phényl]-2-[1-(1H-1,2,4-triazolylméthyl)]-1,3-dioxanne, ou un sel de l'un de ces composés.

9. Procédé de préparation des composés de formule I selon la revendication 1, dans lesquels Y représente —CH= ou —N=; $R_a$ et $R_b$ représentent chacun, indépendamment l'un de l'autre, l'hydrogène, un halogène, un groupe alkyle en $C_1$—$C_3$, alcoxy en $C_1$—$C_3$ ou nitro;

Ar représente un groupe

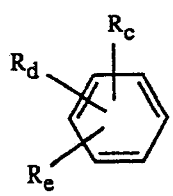

ou

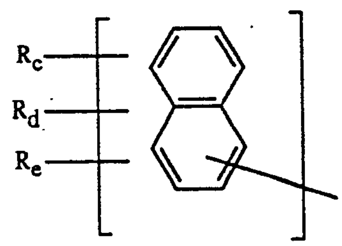

,

dans lequel

$R_c$, $R_d$ et $R_e$ représentent chacun, indépendamment les uns des autres, l'hydrogène, un halogène, un groupe alkyle en $C_1$—$C_3$, alcoxy en $C_1$—$C_3$, nitro ou $CF_3$;

U et V représentent chacun, indépendamment l'un de l'autre, un groupe alkyle en $C_1$—$C_{12}$ ou forment ensemble l'un des ponts alkylène suivants:

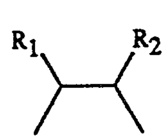

,

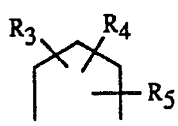

ou

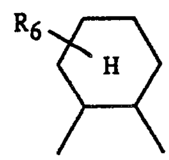

dans lesquels

$R_1$ et $R_2$ représentent chacun, indépendamment l'un de l'autre, l'hydrogène, un groupe alkyle en $C_1$—$C_{12}$, un groupe alkyle en $C_1$—$C_{12}$ mono- ou poly-substitué par des halogènes, un groupe phenyle, un groupe phényle mono- ou poly-substitué par des halogènes et/ou des groupes alkyle en $C_1$—$C_3$, ou le groupe —$CH_2$—Z—$R_7$ dans lequel

Z représente l'oxygène ou le soufre et

$R_7$ représente l'hydrogène, un groupe alkyle en $C_1$—$C_8$, un groupe alkyle en $C_1$—$C_8$ substitué par un groupe alcoxy en $C_1$—$C_2$, un groupe alcényle en $C_3$—$C_4$, un groupe 2-propynyle, 3-halogéno-2-propynyle, phényle, un groupe phényle mono- ou poly-substitué par des halogènes, des groupes alkyle en $C_1$—$C_3$, alcoxy en $C_1$—$C_3$, nitro et/ou $CF_3$, un groupe benzyle ou un groupe benzyle mono- ou poly-substitué par des halogènes, des groupes alkyle en $C_1$—$C_3$ et/ou alcoxy en $C_1$—$C_3$;

$R_3$, $R_4$ et $R_5$ représentent chacun, indépendamment les uns des autres, l'hydrogène ou un groupe alkyle en $C_1$—$C_4$, le nombre total des atomes de carbone de $R_3$, $R_4$ et $R_5$ ne devant pas dépasser 6, et

$R_6$ représente l'hydrogène ou un groupe alkyle en $C_1$—$C_3$;

y compris leurs sels formés par addition avec des acides et complexes métalliques, caractérisé en ce que:

a) on condense un composé de formule II

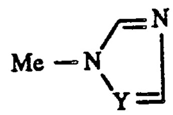

(II)

dans laquelle Me représente l'hydrogène ou un cation métallique, avec un composé de formule III

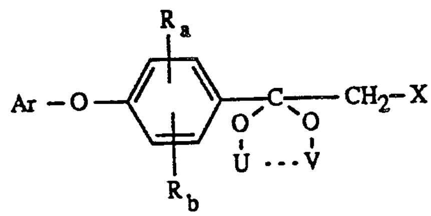

(III)

dans laquelle X représente un groupe éliminable, ou bien

b) dans un composé de formule IV

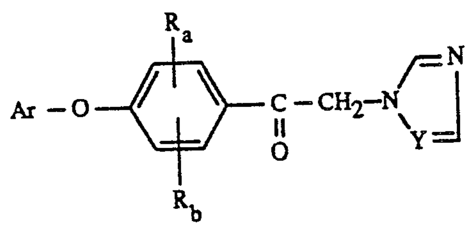

(IV)

on convertit le groupe carbonyle, par réaction avec un diol de formule HO—U——V—OH (Vb) en un groupe de formule V

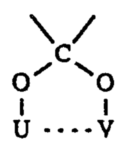

(V)

ou bien

c) on condense entre eux les composés de formules VIII et IX

$Ar\text{-}X_3$ (VIII) et

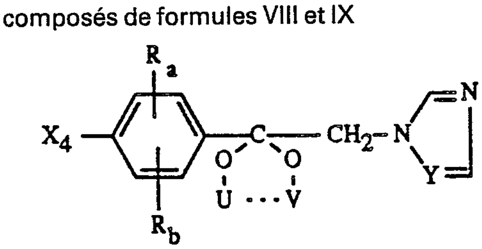

(IX)

dans lesquelles $X_3$ représente un groupe O—Me dans lequel Me représente l'hydrogène ou de préférence un cation métallique et $X_4$ peut également représenter le fluor et, si on le désire, on convertit un composé ainsi obtenu à l'état libre en un sel par addition avec un acide, un sel d'acide ainsi obtenu en le composé libre ou en un autre sel d'acide, ou bien un composé ainsi obtenu à l'état libre ou à l'état de sel en un complexe métallique, les symboles des formules II à IX ayant les significations indiquées en référence à la formule I et Me représentant l'hydrogène ou un cation métallique.

10. Procédé de préparation des composés de formule I selon la revendication 1 dans lesquels U et V forment ensemble un groupe de formule $—CH_2—CH(CH_2ZR'_7)—$ et $R'_7$ a les mêmes significations que $R_7$ à l'exception l'hydrogène, caractérisé en ce que l'on fait réagir un composé de formule VI

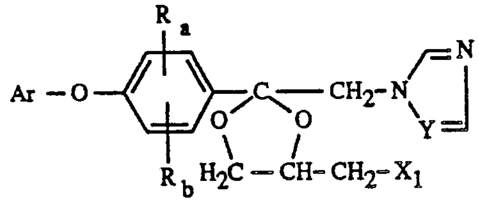

(VI)

avec un composé de formule VII

$$X_2—R_7 \qquad (VII)$$

dans lesquels l'un des symboles $X_1$ et $X_2$ représente un groupe hydroxy ou mercapto et l'autre un groupe éliminable nucléophile X, ou bien $X_1$ et $X_2$ représentent tous deux des groupes hydroxy, les autres symboles Ar, $R_a$, $R_b$, Y, Z, $R_7$ ayant les significations indiquées dans la revendication 1, et si on le désire, on convertit un composé ainsi obtenu à l'état libre en un sel par addition avec un acide, un sel d'acide ainsi obtenu en le composé libre ou en un autre sel d'acide, ou un composé ainsi obtenu à l'état libre ou à l'état de sel en un complexe métallique.

11. Procédé selon la revendication 9, pour la préparation des composés de formule I de la revendication 7, à l'exception des composés de formule I de la revendication 1.

12. Utilisation des composés de formule I définie dans la revendication 1, pour combattre et/ou prévenir une attaque de végétaux par des microorganismes.

13. Utilisation selon la revendication 12, de composés de formule I définis dans l'une des revendications 2 à 6.

14. Utilisation des composés de formule I définis dans la revendication 7, pour combattre et/ou prévenir une attaque de végétaux par des microorganismes.

15. Utilisation selon la revendication 14, des composés de formule I définis dans la revendication 8.

16. Utilisation selon l'une des revendications 12 à 15, caractérisé en ce que les microorganismes sont des mycètes phytopathogènes.

**Claims for the Contracting States: BE CH DE FR IT LI LU NL SE**

1. A compound of formula I

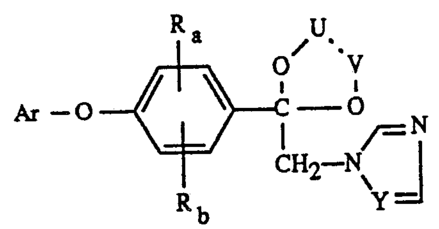

(I)

wherein Y is —CH= or —N=; $R_a$ and $R_b$ are each independently of the other hydrogen, halogen, $C_1$—$C_3$alkyl, $C_1$—$C_3$alkoxy or nitro; Ar is the group

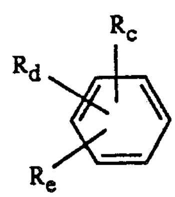

or

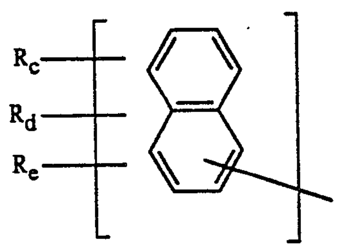

wherein

$R_c$, $R_d$ and $R_e$ are each independently of the other hydrogen, halogen, $C_1$—$C_3$alkyl, $C_1$—$C_3$alkoxy, nitro or $CF_3$;

U and V are each independently of the other $C_1$—$C_{12}$alkyl or, when taken together, are an alkylene bridge selected from

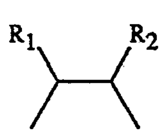

,

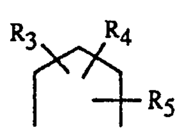

and

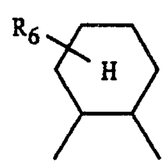

wherein

$R^1$ and $R^2$ are each independently of the other hydrogen, $C_1$—$C_{12}$alkyl or $C_1$—$C_{12}$alkyl which is substituted by one or more halogen atoms; phenyl or phenyl which is substituted by one or more halogen atoms and/or $C_1$—$C_3$alkyl groups; or are the group $—CH_2—Z—R_7$, wherein

Z is oxygen or sulfur, and

$R_7$ is hydrogen, $C_1$—$C_8$alkyl or $C_1$—$C_8$alkyl which is substituted $C_1$—$C_2$alkoxy, $C_3$—$C_4$alkenyl, 2-

propynyl, 3-halo-2-propynyl, phenyl or phenyl which is substituted by one or more of halogen, $C_1$—$C_3$alkyl, $C_1$—$C_3$alkoxy, nitro and/or $CF_3$; benzyl or benzyl which is substituted by one or more of halogen, $C_1$—$C_3$alkyl and/or $C_1$—$C_3$alkoxy;

$R_3$, $R_4$ and $R_5$ are each independently of the other hydrogen or $C_1$—$C_4$alkyl, the total number of carbon atoms in $R_3$, $R_4$ and $R_5$ not exceeding 6, and

$R_6$ is hydrogen or $C_1$—$C_3$alkyl,

or an acid addition salt or metal complex thereof.

2. A compound of formula I according to claim 1, wherein Y is —CH= or —N=; Ar is as defined for formula I; $R_a$, $R_b$, $R_c$, $R_d$ and $R_e$ are each independently of the other hydrogen, chloro, bromo, fluoro, methyl, methoxy or nitro; U and V are each independently of the other $C_1$—$C_3$alkyl or, when taken together, are an alkylene group as defined for formula I, and $R_1$, $R_2$, $R_3$, $R_4$, $R_5$ and $R_6$ are each independently of the other hydrogen or $C_1$—$C_3$alkyl, or $R_1$ is —$CH_2$—O—$R_7$, where $R_7$ is $C_1$—$C_3$alkyl, $C_2$—$C_4$alkyl which is substituted by $C_1$—$C_3$alkoxy, or is $C_3$—$C_4$alkenyl or phenyl.

3. 2-[p-(Phenoxy)phenyl]-2-[1-(1H-1,2,4-triazolyl)methyl]-4-methyl-1,3-dioxane or a salt thereof.

4. 2-[p-(Phenoxy)phenyl]-2-[1-(1H-1,2,4-triazolyl)methyl]-4-ethyl-1,3-dioxane or a salt thereof.

5. 2-[p-(Phenoxy)phenyl]-2-[1-(1H-1,2,4-triazolyl)methyl]-4-methyl-dioxolane or a salt thereof.

6. 2-[p-(Phenoxy)phenyl]-2-[1-(1H-1,2,4-triazolyl)methyl]-4-ethyl-dioxolane or a salt thereof.

7. 2-[p-(Phenoxy)phenyl]-2-[1-(1H-1,2,4-triazolyl)methyl]-4-methyl-5-methyl-1,3-dioxolane.

8. 2-[p-(Phenoxy)phenyl]-2-[1-(1H-1,2,4-triazolyl)methyl]-1,3-dioxolane or a salt thereof.

9. 2-[p-(Phenoxy)phenyl]-2-(1-imidazoyl)methyl]-4-ethyl]-1,3-dioxolane or a salt thereof.

10. 2-[p-(3-Chlorophenoxy)phenyl]-2-(1-imidazolylmethyl)-4-ethyl-dioxolane or a salt thereof.

11. 2-[p-(4-Chlorophenoxy)phenyl]-2-(1-imidazolylmethyl)-4-ethyl-dioxolane or a salt thereof.

12. 2-[p-(Phenoxy)phenyl]-2-[1-(1H-1,2,4-triazolyl)methyl]-4-hydroxymethyl-1,3-dioxolane.

13. 2-[p-(2,5-Dichlorophenoxy)phenyl]-2-(1-imidazolylmethyl)-4-ethyl-dioxolane or a salt thereof.

14. A compound of formula I

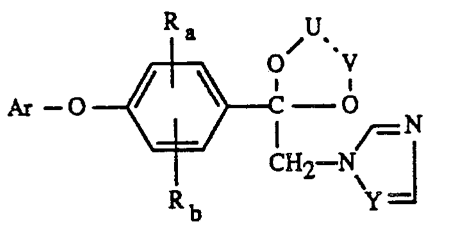

(I)

wherein Y is —CH= or —N=;

$R_a$ and $R_b$ are each independently of the other hydrogen, halogen, $C_1$—$C_3$alkyl, $C_1$—$C_3$alkoxy or nitro;

Ar is phenyl or naphthyl or phenyl or naphthyl each substituted by one or more of halogen, $C_1$—$C_7$alkyl, $C_1$—$C_7$alkoxy, nitro and/or $CF_3$,

U and V are each independently of the other $C_1$—$C_{12}$alkyl or $C_1$—$C_{12}$alkyl which is substituted by halogen or $C_1$—$C_6$alkoxy or, when taken together, are an alkylene bridge selected from

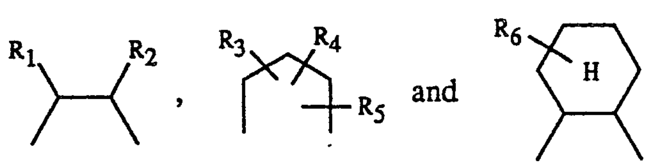

, and

wherein

$R_1$ and $R_2$ are each independently of the other hydrogen, $C_1$—$C_{12}$alkyl or $C_1$—$C_{12}$alkyl which is substituted by one or more halogen atoms; phenyl or phenyl which is substituted by one or more halogen atoms and/or $C_1$—$C_3$alkyl groups; or are the group —$CH_2$—Z—$R_7$, wherein

Z is oxygen or sulfur, and

$R_7$ is hydrogen, $C_1$—$C_8$alkyl or $C_1$—$C_8$alkyl which is substituted by $C_1$—$C_2$alkoxy, or is $C_3$—$C_4$alkenyl, 2-propynyl, 3-halo-2-propynyl, phenyl or phenyl which is substituted by one or more of halogen, $C_1$—$C_3$alkyl, $C_1$—$C_3$alkoxy, nitro and/or $CF_3$; benzyl or benzyl which is substituted by one or more of halogen, $C_1$—$C_3$alkyl and/or $C_1$—$C_3$alkoxy;

$R_3$, $R_4$ and $R_5$ are each independently of the other hydrogen or $C_1$—$C_4$alkyl, the total number of carbon atoms in $R_3$, $R_4$ and $R_5$ not exceeding 6, and

$R_6$ is hydrogen or $C_1$—$C_3$alkyl;

or an acid addition salt or metal complex thereof, with the proviso that the subject matter of claim 1 is excluded from the scope of the protection.

15. 2-[p-(3,4-Dichlorophenoxy)phenyl]-2-(1-imidazolylmethyl)-4-methoxymethyl-1,3-dioxolane.

16. 2-[p-(4-Chloro-2-methylphenoxy)phenyl]-2-(1-imidazolylmethyl)-4-methoxymethyl-1,3-dioxolane.

17. 2-[p-(4-Fluorophenoxy)phenyl]-2-(1-imidazolylmethyl)-4-hydroxymethyl-1,3-dioxolane.

18. 2-[p-(4-Fluorophenoxy)phenyl]-2-[1-(1H-1,2,4,triazolylmethyl)]-1,3-dioxolane.

19. A process for the preparation of a compound of formula I according to claim 1, which comprises

a) condensing a compound of formula II

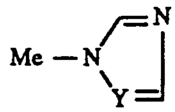 (II)

wherein Me is hydrogen or a metal cation, with a compound of formula III

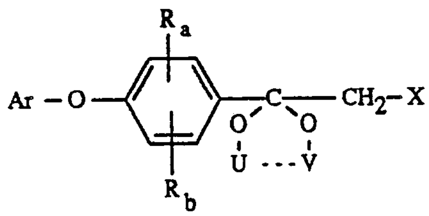 (III)

wherein X is a leaving group, or

b) converting the carbonyl group in a compound of formula IV

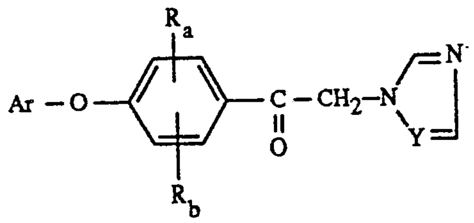 (IV)

by reaction with a diol of formula HO—U——V—OH (Vb) into a group of formula V

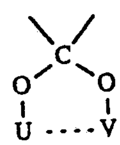 (V)

or

c) condensing compounds of formulae VIII and IX

Ar-$X_3$ (VIII) and 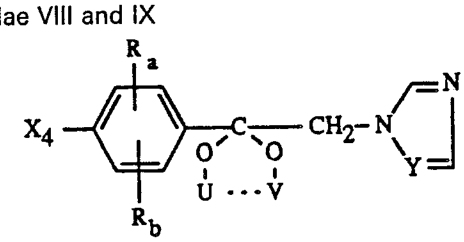 (IX)

wherein $X_3$ is a group —O—Me, wherein Me is hydrogen or preferably a metal cation, and also $X_4$ is fluoro, with each other and, if desired, converting a resultant free compound into an acid addition salt, a resultant acid addition salt into the free compound or into another acid addition salt, or a resultant free compound or acid addition salt into a metal complex, the substituents in formulae II to IX being as defined for formula I and Me is hydrogen or a metal cation.

20. A process for the preparation of a compound of formula I according to claim 1, wherein U and V, when taken together, are a group of formula $—CH_2—CH(CH_2ZR'_7)$, which comprises reacting a compound of formula VI

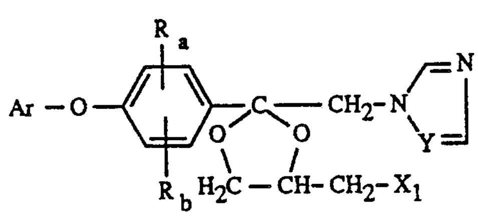 (VI)

with a compound of formula VII

$$X_2—R_7 \qquad \text{(VII)}$$

wherein one of $X_1$ and $X_2$ is hydroxy or mercapto and the other is a nucleophilic leaving group X, or both $X_1$ and $X_2$ together are hydroxyl groups, and wherein the remaining substituents Ar, $R_a$, $R_b$, Y, Z and $R_7$ are as defined in claim 1, and, if desired, converting a resultant free compound into an acid addition salt, a resultant acid addition salt into the free compound or into another acid addition salt, or a resultant free compound or acid addition salt into a metal complex.

21. A process according to claim 19 for the preparation of a compound of formula I as claimed in claim 14, excluding the preparation of a compound of formula I which forms the subject matter of claim 1.

22. An agrochemical pesticidal composition for controlling or preventing infestation by microorganisms, which contains a compound of formula I according to claim 1 as at least one active component.

23. A composition according to claim 22, which contains a compound of formula I as claimed in any one of 2 to 13 as at least one active component.

24. An agrochemical pesticidal composition for controlling or preventing infestation by microorganisms, which contains a compound of formula I according to claim 14 as at least one active component.

25. A composition according to claim 22, which contains a compound of formula I as claimed in any one of claims 15 to 18 as at least one active component.

26. Use of a compound of formula I according to claim 1 for controlling microorganisms and/or for preventing infestation of plants by said microorganisms.

27. Use according to claim 26 of a compound of formula I as claimed in any one of claims 2 to 13.

28. Use of a compound of formula I according to claim 14 for controlling microorganisms and/or for preventing infestation of plants by said microorganisms.

29. Use according to claim 28 of a compound of formula I as claimed in any one of claims 15 to 18.

30. Use according to any one of claims 26 to 29, wherein the microorganisms are phytopathogenic fungi.

**Claims for the Contracting State: AT**

1. An agrochemical pesticidal composition for controlling or preventing infestation by microorganisms, which contains at least one active component a compound of formula I

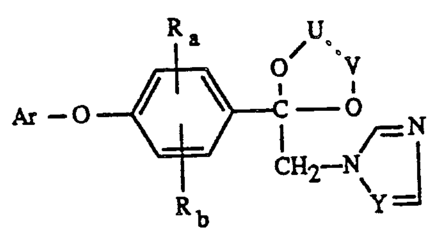

(I)

wherein Y is —CH= or —N=; $R_a$ and $R_b$ are each independently of the other hydrogen, halogen, $C_1$—$C_3$alkyl, $C_1$—$C_3$alkoxy or nitro; Ar is the group

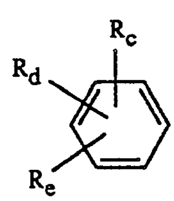

or

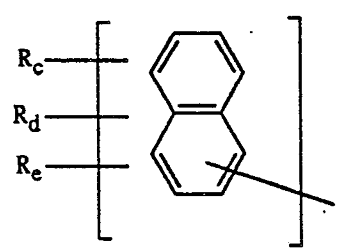

wherein

$R_c$, $R_d$ and $R_e$ are each independently of the other hydrogen, halogen, $CH_2$alkyl, $C_1$—$C_3$alkoxy, nitro or $CF_3$;

U and V are each independently of the other $C_1$—$C_{12}$alkyl or together are an alkylene bridge selected from

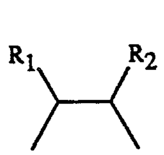

,

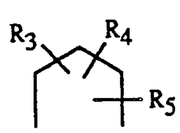

and

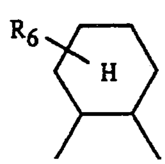

wherein

$R^1$ and $R^2$ are each independently of the other hydrogen, $C_1$—$C_{12}$alkyl or $C_1$—$C_{12}$alkyl which is

substituted by one or more halogen atoms; phenyl or phenyl which is substituted by one or more halogen atoms and/or $C_1$—$C_3$alkyl groups; or are the group —$CH_2$—Z—$R_7$, wherein

Z is oxygen or sulfur, and

$R_7$ is hydrogen, $C_1$—$C_8$alkyl or $C_1$—$C_8$alkyl which is substituted by $C_1$—$C_2$alkoxy, $C_3$—$C_4$alkenyl, 2-propynyl, 3-halo-2-propynyl, phenyl or phenyl which is substituted by one or more of halogen, $C_1$—$C_3$alkyl, $C_1$—$C_3$alkoxy, nitro and/or $CF_3$; benzyl or benzyl which is substituted by halogen, $C_1$—$C_3$alkyl and/or $C_1$—$C_3$alkoxy;

$R_3$, $R_4$ and $R_5$ are each independently of the other hydrogen or $C_1$—$C_4$alkyl, the total number of carbon atoms in $R_3$, $R_4$ and $R_5$ not exceeding 6, and

$R_6$ is hydrogen or $C_1$—$C_3$alkyl,

or an acid addition salt or metal complex thereof.

2. A composition according to claim 1, which contains as active component a compound of formula I, wherein Y is —CH= or —N=; Ar is as defined for formula I; $R_a$, $R_b$, $R_c$, $R_d$ and $R_e$ are each independently of the other hydrogen, chloro, bromo, fluoro, methyl, methoxy or nitro; U and V are each independently of the other $C_1$—$C_3$alkyl or, when taken together, are an alkylene group as defined for formula I, and $R_1$, $R_2$, $R_3$, $R_4$, $R_5$ and $R_6$ are each independently of the other hydrogen or $C_1$—$C_3$alkyl, or $R_1$ is —$CH_2$—O—$R_7$, where $R_7$ is $C_1$—$C_3$alkyl, $C_2$—$C_4$alkyl which is substituted by $C_1$—$C_3$alkoxy, $C_3$—$C_4$alkenyl or phenyl.

3. A composition according to claim 1 which contains as active component 2-[p-(phenoxy)phenyl]-2-[1-(1H-1,2,4-triazolyl)methyl]-4-methyl-1,3-dioxane or a salt thereof.

4. A composition according to claim 1 which contains as active component 2-[p-(phenoxy)phenyl]-2-[1-(1H-1,2,4-triazolyl)methyl]-4-ethyl-1,3-dioxane or a salt thereof.

5. A composition according to claim 1 which contains as active component 2-[p-(phenoxy)phenyl]-2-[1-(1H-1,2,4-triazolyl)methyl]-4-methyl-dioxolane or a salt thereof.

6. A composition according to claim 1 which contains as active component one of the compounds

2-[p-(phenoxy)phenyl]-2-[1-(1H-1,2,4-triazolyl)methyl]-4-ethyl-dioxolane,
2-[p-(phenoxy)phenyl]-2-[1-(1H-1,2,4-triazolyl)methyl]-4-methyl-5-methyl-1,3-dioxolane,
2-[p-(phenoxy)phenyl]-2-[1-(1H-1,2,4-triazolyl)methyl]-1,3-dioxane,
2-[p-(phenoxy)phenyl]-2-(1-imidazolylmethyl]-4-ethyl-1,3-dioxolane,
2-[p-(3-chlorophenoxy)phenyl]-2-(1-imidazolylmethyl)-4-ethyl-1,3-dioxolane,
2-[p-(4-chlorophenoxy)phenyl]-2-(1-imidazolylmethyl)-4-ethyl-1,3-dioxolane,
2-[p-(phenoxy)phenyl]-2-[1-(1H-1,2,4-triazolyl)methyl]-4-hydroxymethyl-1,3-dioxolane,
2-[p-(2,5-dichlorophenoxy)phenyl]-2-(1-imidazolylmethyl)-4-ethyl-1,3-dioxolane, or respectively a salt thereof.

7. An agrochemical pesticidal composition for controlling or preventing infestation by microorganisms, which contains as at least one active component a compound of formula I

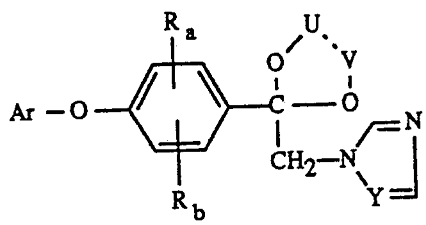

(I)

wherein Y is —CH= or —N=;

$R_a$ and $R_b$ are each independently of the other hydrogen, halogen, $C_1$—$C_3$alkyl, $C_1$—$C_3$alkoxy or nitro;

Ar is phenyl or naphthyl or phenyl or naphthyl each substituted by one or more of halogen, $C_1$—$C_7$alkyl, $C_1$—$C_7$alkoxy, nitro and/or $CF_3$,

U and V are each independently of the other $C_1$—$C_{12}$alkyl or $C_1$—$C_{12}$alkyl which is substituted by halogen or $C_1$—$C_6$alkoxy or, when taken together, are an alkylene bridge selected from

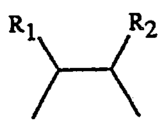

,

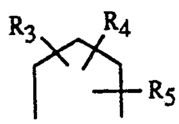

and

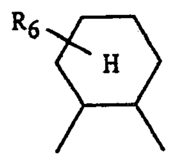

wherein

$R_1$ and $R_2$ are each independently of the other hydrogen, $C_1$—$C_{12}$alkyl or $C_1$—$C_{12}$alkyl which is substituted by one or more halogen atoms; phenyl or phenyl which is substituted by one or more halogen atoms and/or $C_1$—$C_3$alkyl groups; or are the group —$CH_2$—Z—$R_7$, wherein

Z is oxygen or sulfur, and

$R_7$ is hydrogen, $C_1$—$C_8$alkyl or $C_1$—$C_8$alkyl which is substituted by $C_1$—$C_2$alkoxy, $C_3$—$C_4$alkenyl, 2-propynyl, 3-halo-2-propynyl, phenyl or phenyl which is substituted by one or more of halogen, $C_1$—$C_3$alkyl,

$C_1$—$C_3$alkoxy, nitro and/or $CF_3$; benzyl or benzyl which is substituted by one or more of halogen, $C_1$—$C_3$alkyl and/or $C_1$—$C_3$alkoxy;

$R_3$, $R_4$ and $R_5$ are each independently of the other hydrogen or $C_1$—$C_4$alkyl, the total number of carbon atoms in $R_3$, $R_4$ and $R_5$ not exceeding 6, and

$R_6$ is hydrogen or $C_1$—$C_3$alkyl;

or an acid addition salt or metal complex thereof, excluding a compound of formula I as indicated in claim 1 or an acid addition salt or metal complex thereof.

8. A composition according to claim 7 which contains as active component one of the compounds

2-[p-(3,4-dichlorophenoxy)phenyl]-2-(1-imidazolylmethyl)-4-methoxymethyl-1,3-dioxolane,

2-[p-(4-chloro-2-methylphenoxy)phenyl]-2-(1-imidazolylmethyl)-4-methoxymethyl-1,3-dioxolane,

2-[p-(4-fluorophenoxy)phenyl]-2-(1-imidazolylmethyl)-4-hydroxymethyl-1,3-dioxolane,

2-[p-(4-fluorophenoxy)phenyl]-2-[1-(1H-1,2,4,triazolylmethyl)]-1,3-dioxane,

or respectively a salt thereof.

9. A process for the preparation of a compound of formula I according to claim 1, wherein

Y is —CH= or —N=; $R_a$ and $R_b$ are each independently of the other hydrogen, halogen, $C_1$—$C_3$alkyl, $C_1$—$C_3$alkoxy or nitro; Ar is the group

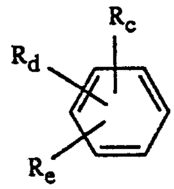

or

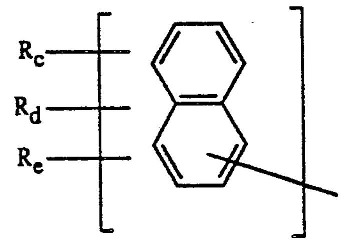

wherein

$R_c$, $R_d$ and $R_e$ are each independently of the other hydrogen, halogen, $C_1$—$C_3$alkyl, $C_1$—$C_3$alkoxy, nitro or $CF_3$;

U and V are each independently of the other $C_1$—$C_{12}$alkyl or together are an alkylene bridge selected from

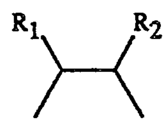

,

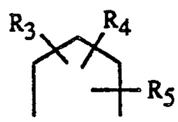

and

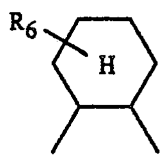

wherein

$R^1$ and $R^2$ are each independently of the other hydrogen, $C_1$—$C_{12}$alkyl or $C_1$—$C_{12}$alkyl which is substituted by one or more halogen atoms; phenyl, phenyl which is substituted by one or more halogen atoms and/or $C_1$—$C_3$alkyl groups; or are the group —$CH_2$—Z—$R_7$, and

Z is oxygen or sulfur, and

$R_7$ is hydrogen, $C_1$—$C_8$alkyl or $C_1$—$C_8$alkyl which is substituted by $C_1$—$C_2$alkoxy, or is $C_3$—$C_4$alkenyl, 2-propynyl, 3-halo-2-propynyl, phenyl or phenyl which is substituted by one or more of halogen, $C_1$—$C_3$alkyl, $C_1$—$C_3$alkoxy, nitro and/or $CF_3$; benzyl or benzyl which is substituted by one or more of halogen, $C_1$—$C_3$alkyl and/or $C_1$—$C_3$alkoxy;

$R_3$, $R_4$ and $R_5$ are each independently of the other hydrogen or $C_1$—$C_4$alkyl, the total number of carbon atoms in $R_3$, $R_4$ and $R_5$ not exceeding 6, and

$R_6$ is hydrogen or $C_1$—$C_3$alkyl,

or an acid addition salt or metal complex thereof,

which process comprises

a) condensing a compound of formula II

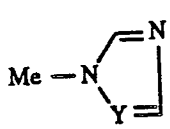

(II)

wherein Me is hydrogen or a metal cation, with a compound of formula III

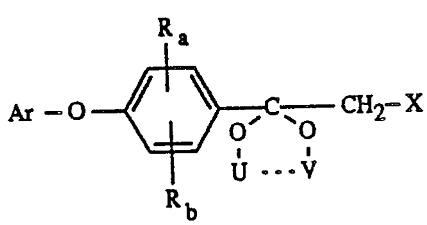

(III)

wherein X is a leaving group, or

b) converting the carbonyl group in a compound of formula IV

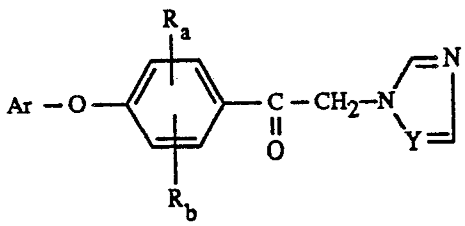

(IV)

by reaction with a diol of formula HO—U——V—OH (Vb) into a group of formula V

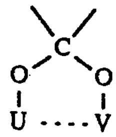

(V)

or

c) condensing compounds of formulae VIII and IX

$Ar\text{-}X_3$ (VIII) and 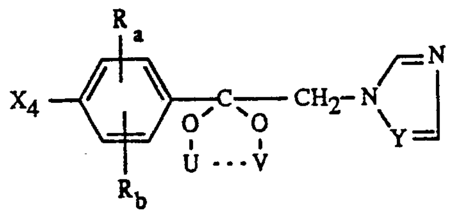 (IX)

wherein $X_3$ is a group —O—Me, wherein Me is hydrogen or preferably a metal cation, and also $X_4$ is fluoro, with each other and, if desired, converting a resultant free compound into an acid addition salt, a resultant acid addition salt into the free compound or into another acid addition salt, or a resultant free compound or acid addition salt into a metal complex, the substituents in formulae II to IX being as defined for formula I and Me is hydrogen or a metal cation.

10. A process for the preparation of a compound of formula I according to claim 1, wherein U and V, when taken together, are a group of formula $—CH_2—CH(CH_2ZR'_7)$, and $R'_7$ is a radical $R_7$ which differs from hydrogen, which comprises reacting a compound of formula VI

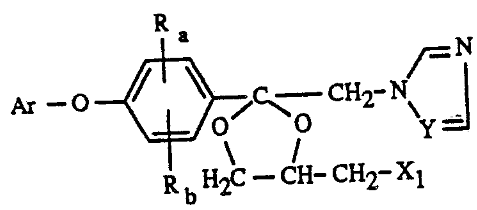

(VI)

with a compound of formula VII

$$X_2—R_7 \qquad \text{(VII)}$$

wherein one of $X_1$ and $X_2$ is hydroxy or mercapto and the other is a nucleophilic leaving group X, or both $X_1$ and $X_2$ together are hydroxyl groups, and wherein the remaining substituents Ar, $R_a$, $R_b$, Y, Z and $R_7$ are as defined in claim 1, and, if desired, converting a resultant free compound into an acid addition salt, a resultant acid addition salt into the free compound or into another acid addition salt, or a resultant free compound or acid addition salt into a metal complex.

11. A process according to claim 9 for the preparation of a compound of formula I as claimed in claim 7, excluding a compound of formula I according to claim 1.

12. Use of a compound of formula I as defined in claim 1 for controlling microorganisms and/or preventing infestation of plants by microorganisms.

13. Use according to claim 12 of a compound of formula I as defined in any one of claims 2 to 6.

14. Use of a compound of formula I as defined in claim 7 for controlling microorganisms and/or preventing infestation of plants by said microorganisms.

15. Use according to claim 14 of a compound of formula I as defined in claim 8.

16. Use according to any one of claims 12 to 15, wherein the microorganisms are phytopathogenic fungi.